# EUROPEAN PATENT SPECIFICATION

(11) **EP 4 003 345 B1**
(45) Date of publication and mention of the grant of the patent: **04.12.2024**
(21) Application number: 20848360.2
(22) Date of filing: 30.07.2020
(51) Int. Cl.: C07D 471/10, C07D 471/20, A61K 31/444, A61K 31/4545, A61K 31/4745, C07D 471/04, A61P 35/00

(54) **DUAL ATM AND DNA-PK INHIBITORS FOR USE IN ANTI-TUMOR THERAPY**
DUALE ATM- UND DNA-PK-INHIBITOREN ZUR VERWENDUNG IN DER ANTITUMORTHERAPIE
INHIBITEURS DOUBLES DE L'ATM ET DE L'ADN-PK DESTINÉS À UNE UTILISATION EN THÉRAPIE ANTITUMORALE

(30) Priority: 30.07.2019 CN 201910695148; 06.08.2019 US 201962883325 P
(43) Date of publication of application: 01.06.2022
(73) Proprietor: XRad Therapeutics, Inc., Orlando, FL 32810 (US)
(72) Inventor: FU, Jianmin, Beijing, 100176 (CN); WANG, Yaode, Beijing, 101102 (CN); SUN, Yue, Beijing, 101102 (CN); WU, Guosheng, Beijing, 100176 (CN); LU, Aijun, Beijing, 100078 (CN); ZHANG, Shuang, Beijing, 100176 (CN); GOODNOW, Robert, A., Concord, MA 01742 (US); GILMER, Tona, Rougemont, NC 27572 (US); KASTAN, Michael, Chapel Hill, NC 27514 (US); KIRSCH, David, Durham, NC 27705 (US)
(74) Representative: Carpmaels & Ransford LLP
(86) International application number: PCT/US2020/044322
(87) International publication number: WO 2021/022078

(56) References cited:
- WO-A1-2019/201283
- CN-A- 110 386 932
- US-A1- 2017 129 888
- US-A1- 2018 072 715
- US-A1- 2018 280 377
- No further relevant documents disclosed

## Description

### FIELD OF THE INVENTION

The invention relates to compounds and pharmaceutically acceptable salts thereof and to compounds for use for the treatment of cancer as a monotherapy or in combination with radiotherapy, chemotherapy, and/or immunotherapy.

### BACKGROUND OF THE INVENTION

Several members of the PIKK (PI-3K-like Kinase) family of serine-threonine kinases are known mediators of DNA damage signaling.

Radiation therapy (RT) is used to treat >50% of all cancer patients at some point during their illness. Despite significant effort, previous approaches to develop clinical radiosensitizers have not been highly effective, primarily as a result of targeting non-specific pathways which are not direct regulators of the cellular response to radiation.

There is a need for new therapies for oncological diseases.

### SUMMARY OF THE INVENTION

In general, the invention provides a compound 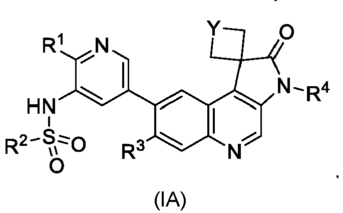 or a pharmaceutically acceptable salt thereof.
Y is CHR5;
R1 is -O-L-N(R7)2;
R2 is C1-3 alkyl;
each R3 is independently halogen or C1-3 alkyl;
R4 is alkyl;
R5 is hydrogen or benzyloxy;
each R7 is independently H or C1-3 alkyl; and
L is ethylene.

In particular embodiments, R³ is halogen (e.g., fluorine).

In some embodiments, one R⁷ is H, and the remaining R⁷ is optionally C₁₋₃ alkyl. In certain embodiments, at least one R⁷ is isopropyl. In particular embodiments, R² is methyl, ethyl, or isopropyl. In further embodiments, R² is methyl. In yet further embodiments, R⁴ is methyl. In still further embodiments, R⁵ is hydrogen. In other embodiments, R⁵ is optionally substituted C₁₋₃ alkyl. In yet other embodiments, R⁵ is benzyloxy.

In particular embodiments, the compound is selected from the group consisting of: 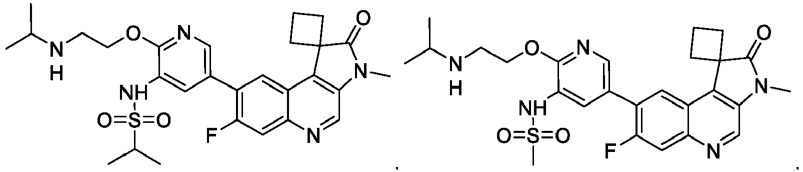 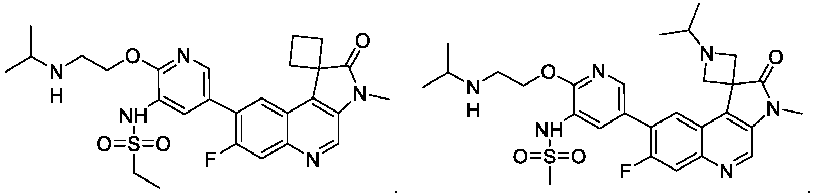 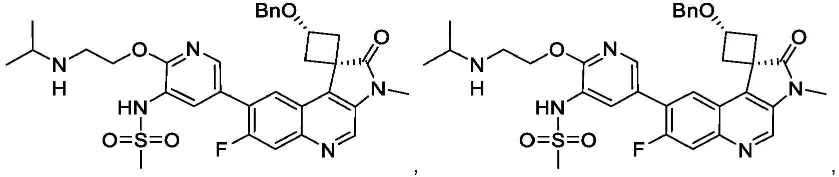 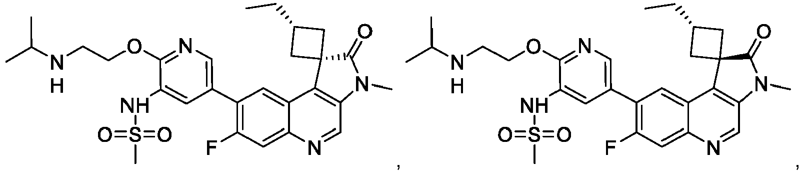 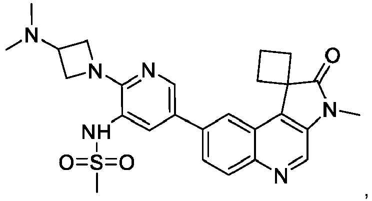 and pharmaceutically acceptable salts thereof.

In further embodiments, the compound is of the following structure: 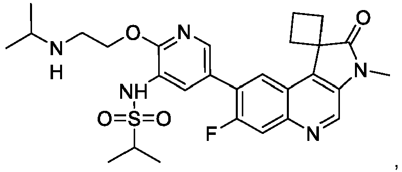 or a pharmaceutically acceptable salt thereof.

In yet further embodiments, the compound is of the following structure: 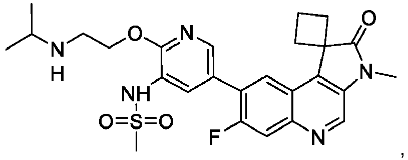 or a pharmaceutically acceptable salt thereof.

In still further embodiments, the compound is of the following structure: 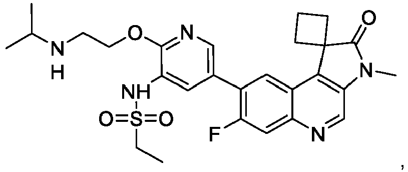 or a pharmaceutically acceptable salt thereof.

In another aspect, the invention provides a pharmaceutical composition including the compound of the invention or a pharmaceutically acceptable salt thereof and a pharmaceutically acceptable excipient.

The references to methods of treatment in the subsequent paragraphs of this description are to be interpreted as references to the compounds, pharmaceutical compositions and medicaments of the present invention for use in a method for treatment of the human (or animal) body by therapy (or for diagnosis).

In yet another aspect, the invention provides a method of treating an oncological disease (e.g., cancer, e.g., those cancers described herein) by administering a therapeutically effective amount of the compound of the invention, or a pharmaceutically acceptable salt thereof, or the pharmaceutical composition of the invention to a patient in need thereof. In still another aspect, the invention provides pharmaceutical compositions for use in the treatment of an oncological disease (e.g., cancer, e.g., those cancers described herein). The pharmaceutical compositions include the compound of the invention. In a further aspect, the invention provides use of the compound of the invention in the manufacture of a medicament for the treatment of an oncological disease (e.g., cancer, e.g., those cancers described herein). In some embodiments, the oncological disease is a brain cancer, bladder cancer, breast cancer, central nervous system cancer, cervical cancer, colon cancer, endometrial cancer, esophageal cancer, gastrointestinal stromal tumor, gastric cancer, head and neck cancer, buccal cancer, cancer of the mouth, hepatocellular cancer, lung cancer, melanoma, Merkel cell carcinoma, mesothelioma, nasopharyngeal cancer, neuroblastoma, osteosarcoma, ovarian cancer, pancreatic cancer, prostate cancer, renal cancer, salivary gland cancer, sarcomas, testicular cancer, urothelial cancer, vulvar cancer, or Wilm's tumor. In further embodiments, the oncological disease is a breast cancer, lung cancer, head and neck cancer, pancreatic cancer, rectal cancer, glioblastoma, hepatocellular carcinoma, cholangiocarcinoma, metastic liver lesions, melanoma, bone sarcoma, soft tissue sarcoma, endometrial cancer, cervical cancer, prostate cancer, or Merkel cell carcinoma.

In some embodiments, the patient is receiving radiotherapy. In certain embodiments, the compound or the pharmaceutical composition is administered to the patient concomitantly with the radiotherapy. In particular embodiments, the compound or the pharmaceutical composition is administered to the patient before radiotherapy. In further embodiments, the compound or the pharmaceutical composition is administered to the patient after radiotherapy. In yet further embodiments, the radiotherapy comprises external, internal, brachytherapy, or systemic exposure, e.g., with a radionuclide (e.g., a β-emitting radionuclide (e.g., ³²Phosphorus, ⁶⁷Copper, ⁷⁷Bromine, ⁸⁹Strontium, ⁹⁰Yttrium, ¹⁰⁵Rhodium, ¹³¹Iodine, ¹³⁷Cesium, ¹⁴⁹Prometheum, ¹⁵³Samarium, ¹⁶⁶Holmium, ¹⁷⁷Lutetium, ¹⁸⁶Rhenium, ¹⁸⁸Rhenium, or ¹⁹⁹Gold), α-emitting radionuclide (e.g., ²¹¹Astatine, ²¹³Bismuth, ²²³Radium, ²²⁵Actinium, or ²²⁷Thorium), γ-ray emitting radionuclide (e.g., ¹⁹²Iridium), or electron capturing radionuclides (e.g., ⁶⁷Gallium, ¹⁰³Palladium, or ¹²⁵Iodine)), antibody radionuclide conjugate (e.g., ⁹⁰Y-ibritumomab tiuxetane, ¹³¹I-tositumomab, ²²⁵Ac-lintuzumab satetraxetan, ²²⁷Th-anetumab corixetan, ⁹⁰Y-epitumomab cituxetan, ⁹⁰Y-clivatuzumab tetraxetan, ¹⁷⁷Lu-lilotomab satetraxetan, ⁹⁰Y-rosopatamab tetraxetan, ⁹⁰Y-tabituximab barzuxetan, or ⁹⁰Y-tacatuzumab tetraxetan), or another targeted radionuclide conjugate (e.g., ¹³¹I-PSMA, ⁹⁰Y-PSMA, ¹⁷⁷Lu-PSMA, or ¹⁷⁷Lu-satoreotide tetraxetan). Preferably, the radiotherapy comprises administering an antibody radionuclide conjugate. In still further embodiments, the patient is receiving an anti-tumor agent. In other embodiments, the anti-tumor agent is one or more of cisplatin, oxaliplatin, carboplatin, valrubicin, idarubicin, calicheamicin, or a PARP inhibitor. In yet other embodiments, the anti-tumor agent is an anti-tumor biological agent and/or anti-tumor immunotherapeutic agent. In still other embodiments, the compound or the pharmaceutical composition is administered to the patient concomitantly with the anti-tumor agent. In some embodiments, the compound or the pharmaceutical composition is administered to the patient before the anti-tumor agent. In certain embodiments, the compound or the pharmaceutical composition is administered to the patient after the anti-tumor agent.

### DETAILED DESCRIPTION

### DEFINITIONS

It is to be understood that the terminology employed herein is for the purpose of describing particular embodiments, and is not intended to be limiting. Further, although any methods, devices and materials similar or equivalent to those described herein can be used in the practice or testing of the invention, the preferred methods, devices and materials are now described. In addition to the foregoing, as used in the specification and appended claims, unless specified to the contrary, the following terms have the meaning indicated:
"Amino" refers to the -NH₂ radical.
"Cyano" refers to the -CN radical.
"Hydroxyl" refers to the -OH radical.
"Imino" refers to the = NH substituent.
"Nitro" refers to the -NOz radical.
"Oxo" refers to the = O substituent.
"Thioxo" refers to the = S substituent.
"Trifluoromethyl" refers to the -CF₃ radical.

"Alkyl" refers to a linear, saturated, acyclic, monovalent hydrocarbon radical or branched, saturated, acyclic, monovalent hydrocarbon radical, having from one to twelve carbon atoms, preferably one to eight carbon atoms or one to six carbon atoms, and which is attached to the rest of the molecule by a single bond, e.g., methyl, ethyl, n-propyl, 1-methylethyl (iso-propyl), n-butyl, n-pentyl, 1,1-dimethylethyl (t-butyl), 3-methylhexyl, 2-methylhexyl and the like. An optionally substituted alkyl radical is an alkyl radical that is optionally substituted, valence permitting, by one, two, three, four, or five substituents independently selected from the group consisting of halo, cyano, nitro, aryl, cycloalkyl, heterocyclyl, heteroaryl, oxo, trimethylsilanyl, -OR¹⁴, -OC(O)-R¹⁴, -N(R¹⁴)₂, -C(O)R¹⁵, -C(O)OR¹⁴, -C(O)N(R¹⁴)₂,
-N(R¹⁴)C(O)OR¹⁶, -N(R¹⁴)C(O)R¹⁶, -N(R¹⁴)S(O)ₜR¹⁶ (where t is 1 or 2), -S(O)ₜOR¹⁶ (where t is 1 or 2), - S(O)ₚR¹⁶ (where p is 0, 1, or 2) and -S(O)ₜN(R¹⁴)₂ (where t is 1 or 2), where each R¹⁴ is independently hydrogen, alkyl, haloalkyl, cycloalkyl, cycloalkylalkyl, aryl, aralkyl, heterocyclyl, or heteroaryl; each R¹⁵ is independently hydrogen, cycloalkyl, aryl, heterocyclyl, or heteroaryl; and each R¹⁶ is independently alkyl, haloalkyl, cycloalkyl, aryl, heterocyclyl, or heteroaryl.

"Alkenyl" refers to a linear, acyclic, monovalent hydrocarbon radical or branched, acyclic, monovalent hydrocarbon radical, containing one, two, or three carbon-carbon double bonds, having from two to twelve carbon atoms, preferably two to eight carbon atoms and which is attached to the rest of the molecule by a single bond, *e.g.,* ethenyl, prop-1-enyl, but-1-enyl, pent-1-enyl, penta-1,4-dienyl and the like. An optionally substituted alkenyl radical is an alkenyl radical that is optionally substituted, valence permitting, by one, two, three, four, or five substituents independently selected from the group consisting of: halo, cyano, nitro, aryl, cycloalkyl, heterocyclyl, heteroaryl, oxo, trimethylsilanyl, -OR¹⁴, -OC(O)-R¹⁴, - N(R¹⁴)₂, -C(O)R¹⁵, -C(O)OR¹⁴, -C(O)N(R¹⁴)₂, -N(R¹⁴)C(O)OR¹⁶, -N(R¹⁴)C(O)R¹⁶, -N(R¹⁴)S(O)ₜR¹⁶ (where t is 1 or 2), -S(O)ₜOR¹⁶ (where t is 1 or 2), -S(O)ₚR¹⁶ (where p is 0, 1, or 2) and -S(O)ₜN(R¹⁴)₂ (where t is 1 or 2), where each R¹⁴ is independently hydrogen, alkyl, haloalkyl, cycloalkyl, aryl, heterocyclyl, or heteroaryl; each R¹⁵ is independently hydrogen, cycloalkyl, aryl, heterocyclyl, or heteroaryl; and each R¹⁶ is independently alkyl, haloalkyl, cycloalkyl, cycloalkylalkyl, aryl, heterocyclyl, or heteroaryl.

"Alkynyl" refers to a linear, acyclic, monovalent hydrocarbon radical or branched, acyclic, monovalent hydrocarbon radical, containing one or two carbon-carbon triple bonds and, optionally, one, two, or three carbon-carbon double bonds, and having from two to twelve carbon atoms, preferably two to eight carbon atoms and which is attached to the rest of the molecule by a single bond, *e.g.,* ethynyl, prop-1-ynyl, but-1-ynyl, pent-1-ynyl, penta-1-en-4-ynyl and the like. An optionally substituted alkynyl radical is an alkynyl radical that is optionally substituted by one, two, three, four, or five substituents independently selected from the group consisting of: halo, cyano, nitro, aryl, cycloalkyl, heterocyclyl, heteroaryl, oxo, trimethylsilanyl, -OR¹⁴, -OC(O)-R¹⁴, -N(R¹⁴)₂, -C(O)R¹⁵, -C(O)OR¹⁴, -C(O)N(R¹⁴)₂, -N(R¹⁴)C(O)OR¹⁶, -N(R¹⁴)C(O)R¹⁶, -N(R¹⁴)S(O)ₜR¹⁶ (where t is 1 or 2), -S(O)ₜOR¹⁶ (where t is 1 or 2), -S(O)ₚR¹⁶ (where p is 0, 1, or 2) and -S(O)ₜN(R¹⁴)₂ (where t is 1 or 2) where each R¹⁴ is independently hydrogen, alkyl, haloalkyl, cycloalkyl, aryl, heterocyclyl, or heteroaryl; each R¹⁵ is independently hydrogen, cycloalkyl, aryl, heterocyclyl, or heteroaryl; and each R¹⁶ is independently alkyl, haloalkyl, cycloalkyl, aryl, heterocyclyl, or heteroaryl.

"Alkylene" or "alkylene chain" refers to a linear, acyclic, saturated, divalent hydrocarbon chain or branched, acyclic, saturated, divalent hydrocarbon chain, having from one to twelve carbon atoms, e.g., methylene, ethylene, propylene, n-butylene, and the like. The alkylene chain is attached through single bonds. The points of attachment of the alkylene chain may be on the same carbon atom or on different carbon atoms within the alkylene chain. An optionally substituted alkylene chain is an alkylene chain that is optionally substituted, valence permitting, by one, two, three, four, or five substituents independently selected from the group consisting of: halo, cyano, nitro, aryl, cycloalkyl, heterocyclyl, heteroaryl, oxo, trimethylsilanyl, -OR¹⁴, -OC(O)-R¹⁴, -N(R¹⁴)₂, -C(O)R¹⁵, -C(O)OR¹⁴, -C(O)N(R¹⁴)₂, -N(R¹⁴)C(O)OR¹⁶, -N(R¹⁴)C(O)R¹⁶, -N(R¹⁴)S(O)ₜR¹⁶ (where t is 1 or 2), -S(O)ₜOR¹⁶ (where t is 1 or 2), -S(O)ₚR¹⁶ (where p is 0, 1, or 2) and -S(O)ₜN(R¹⁴)₂ (where t is 1 or 2) where each R¹⁴ is independently hydrogen, alkyl, haloalkyl, cycloalkyl, aryl, heterocyclyl, or heteroaryl; each R¹⁵ is independently hydrogen, cycloalkyl, aryl, heterocyclyl, or heteroaryl; and each R¹⁶ is independently alkyl, haloalkyl, cycloalkyl, aryl, heterocyclyl, or heteroaryl. In some embodiments, alkylene is ethylene.

"Alkenylene" or "alkenylene chain" refers to a linear, acyclic, divalent hydrocarbon chain or branched, acyclic, divalent hydrocarbon chain, containing one, two, or three carbon-carbon double bonds and having from two to twelve carbon atoms, *e.g.,* ethenylene, propenylene, n-butenylene and the like. The alkenylene chain is attached through single bonds. The points of attachment of the alkenylene chain may be on the same carbon atom or on different carbon atoms within the alkenylene chain. An optionally substituted alkenylene chain is an alkenylene chain that is optionally substituted, valence permitting, by one, two, three, four, or five substituents independently selected from the group consisting of: halo, cyano, nitro, aryl, cycloalkyl, heterocyclyl, heteroaryl, oxo, trimethylsilanyl, -OR¹⁴, -OC(O)-R¹⁴, -N(R¹⁴)₂, -C(O)R¹⁵, -C(O)OR¹⁴, -C(O)N(R¹⁴)₂, -N(R¹⁴)C(O)OR¹⁶, -N(R¹⁴)C(O)R¹⁶, -N(R¹⁴)S(O)ₜR¹⁶ (where t is 1 or 2), -S(O)ₜOR¹⁶ (where t is 1 or 2), -S(O)ₚR¹⁶ (where p is 0, 1, or 2) and -S(O)ₜN(R¹⁴)₂ (where t is 1 or 2) where each R¹⁴ is independently hydrogen, alkyl, haloalkyl, cycloalkyl, aryl, heterocyclyl, or heteroaryl; each R¹⁵ is independently hydrogen, cycloalkyl, aryl, heterocyclyl, or heteroaryl; and each R¹⁶ is independently alkyl, haloalkyl, cycloalkyl, aryl, heterocyclyl, or heteroaryl.

"Alkynylene" or "alkynylene chain" refers to a linear, acyclic, divalent, hydrocarbon chain or branched, acyclic, divalent hydrocarbon chain, containing one or two carbon-carbon triple bonds and, optionally, one, two, or three carbon-carbon double bonds, and having from two to twelve carbon atoms, *e.g.,* propynylene, *n*-butynylene and the like. The alkynylene chain is attached through single bonds. The points of attachment of the alkynylene may be on the same carbon atom or on different carbon atoms within the alkynylene chain. An optionally substituted alkynylene chain is an alkynelene chain that is optionally substituted by one, two, three, four, or five substituents independently selected from the group consisting of: halo, cyano, nitro, aryl, cycloalkyl, heterocyclyl, heteroaryl, oxo, trimethylsilanyl, -OR¹⁴, -OC(O)-R¹⁴, -N(R¹⁴)₂, -C(O)R¹⁵, -C(O)OR¹⁴, -C(O)N(R¹⁴)₂, -N(R¹⁴)C(O)OR¹⁶, -N(R¹⁴)C(O)R¹⁶, -N(R¹⁴)S(O)ₜR¹⁶ (where t is 1 or 2), -S(O)ₜOR¹⁶ (where t is 1 or 2), -S(O)ₚR¹⁶ (where p is 0, 1, or 2) and -S(O)ₜN(R¹⁴)₂ (where t is 1 to 2) where each R¹⁴ is independently hydrogen, alkyl, haloalkyl, cycloalkyl, aryl, heterocyclyl, or heteroaryl; each R¹⁵ is independently hydrogen, cycloalkyl, aryl, heterocyclyl, or heteroaryl; and each R¹⁶ is independently alkyl, haloalkyl, cycloalkyl, aryl, heterocyclyl, or heteroaryl.

"Alkoxy" refers to a radical of the formula -ORₐ where Rₐ is an alkyl radical as defined above containing one to twelve carbon atoms. The alkyl part of the optionally substituted alkoxy radical is optionally substituted as defined above for an alkyl radical.

"Alkoxyalkyl" refers to a radical of the formula -Rₐ-O-R_{b} where Rₐ is alkylene and R_{b} is alkyl as defined above. Alkyl and alkylene parts of the optionally substituted alkoxyalkyl radical are optionally substituted as defined above for an alkyl radical and alkylene chain, respectively.

"Aralkyl" refers to a radical of the formula -Rₐ-R_{b}, where Rₐ is alkylene and R_{b} is aryl as described herein. Alkylene and aryl portions of optionally substituted aralkyl are optionally substituted as described herein for alkylene and aryl, respectively.

"Aryl" refers to an aromatic monocyclic or multicyclic hydrocarbon ring system radical containing from 6 to 18 carbon atoms, where the multicyclic aryl ring system is a bicyclic, tricyclic, or tetracyclic ring system. Aryl radicals include, but are not limited to, groups such as fluorenyl, phenyl and naphthyl. An optionally substituted aryl is an aryl radical that is optionally substituted by one, two, three, four, or five substituents independently selected from the group consisting of alkyl, akenyl, halo, haloalkyl, haloalkenyl, cyano, nitro, aryl, heteroaryl, heteroarylalkyl, -R¹⁵-OR¹⁴, -R¹⁵-OC(O)-R¹⁴, -R¹⁵-N(R¹⁴)₂, -R¹⁵-C(O)R¹⁴, -R¹⁵-C(O)OR¹⁴, -R¹⁵-C(O)N(R¹⁴)₂, -R¹⁵-N(R¹⁴)C(O)OR¹⁶, -R¹⁵-N(R¹⁴)C(O)R¹⁶, -R¹⁵-N(R¹⁴)S(O)ₜR¹⁶ (where t is 1 or 2), -R¹⁵-S(O)ₜOR¹⁶ (where t is 1 or 2), -R¹⁵-S(O)ₚR¹⁶ (where p is 0, 1, or 2), and -R¹⁵-S(O)ₜN(R¹⁴)₂ (where t is 1 or 2), where each R¹⁴ is independently hydrogen, alkyl, haloalkyl, cycloalkyl, aryl, heterocyclyl, or heteroaryl; each R¹⁵ is independently a direct bond or a linear or branched alkylene or alkenylene chain; and each R¹⁶ is independently alkyl, haloalkyl, cycloalkyl, cycloalkylalkyl, aryl, heterocyclyl, or heteroaryl.

"Arylalkoxy" refers to a group of formula -O-R, where R is aralkyl. An optionally substituted arylalkoxy is an arylalkoxy that is optionally substituted as described herein for aralkyl. In some embodiments, arylalkoxy is benzyloxy.

"Cycloalkyl" refers to a stable non-aromatic monocyclic or polycyclic hydrocarbon radical having from three to fifteen carbon atoms, preferably having from three to ten carbon atoms, and which is saturated or unsaturated, and which attaches to the rest of the molecule by a single bond. A polycyclic hydrocarbon radical is bicyclic, tricyclic, or tetracyclic ring system. An unsaturated cycloalkyl contains one, two, or three carbon-carbon double bonds and/or one carbon-carbon triple bond. Monocyclic cycloalkyl radicals include, for example, cyclopropyl, cyclobutyl, cyclopentyl, cyclohexyl, cycloheptyl, and cyclooctyl. Polycyclic cycloalkyl radicals include, for example, adamantyl, norbornyl, decalinyl, and the like. An optionally substituted cycloalkyl is a cycloalkyl radical that is optionally substituted by one, two, three, four, or five substituents independently selected from the group consisting of alkyl, alkenyl, halo, haloalkyl, haloalkenyl, cyano, nitro, oxo, aryl, aralkyl, cycloalkyl, heterocyclyl, heteroaryl, -R¹⁵-OR¹⁴, -R¹⁵-OC(O)-R¹⁴, -R¹⁵-N(R¹⁴)₂, -R¹⁵-C(O)R¹⁴, -R¹⁵-C(O)OR¹⁴, -R¹⁵-C(O)N(R¹⁴)₂, -R¹⁵-N(R¹⁴)C(O)OR¹⁶, -R¹⁵-N(R¹⁴)C(O)R¹⁶, -R¹⁵-N(R¹⁴)S(O)ₜR¹⁶ (where t is 1 or 2), -R¹⁵-S(O)ₜOR¹⁶ (where t is 1 or 2), -R¹⁵-S(O)ₚR¹⁶ (where p is 0, 1, or 2) and -R¹⁵-S(O)ₜN(R¹⁴)₂ (where t is 1 or 2) where each R¹⁴ is independently hydrogen, alkyl, haloalkyl, cycloalkyl, aryl, heterocyclyl, or heteroaryl; each R¹⁵ is independently a direct bond or a linear or branched alkylene or alkenylene chain; and each R¹⁶ is independently alkyl, haloalkyl, cycloalkyl, cycloalkylalkyl, aryl, aralkyl, heterocyclyl, or heteroaryl.

"Fused" refers to any ring system described herein which is fused to an existing ring structure in the compounds of the invention. When the fused ring system is a heterocyclyl or a heteroaryl, any carbon atom on the existing ring structure which becomes part of the fused ring system may be replaced with a nitrogen atom.

"Halo" refers to the halogen substituents: bromo, chloro, fluoro, and iodo.

"Haloalkyl" refers to an alkyl radical, as defined above, that is further substituted by one or more halogen substituents. The number of halo substituents included in haloalkyl is from one and up to the total number of the hydrogen atoms available for replacement with the halo substituents (e.g., perfluoroalkyl). Non-limiting examples of haloalkyl include trifluoromethyl, difluoromethyl, trichloromethyl, 2,2,2-trifluoroethyl, 1-fluoromethyl-2-fluoroethyl, 3-bromo-2-fluoropropyl, 1-bromomethyl-2-bromoethyl and the like. For an optionally substituted haloalkyl, the hydrogen atoms bonded to the carbon atoms of the alkyl part of the haloalkyl radical may be optionally replaced with substituents as defined above for an optionally substituted alkyl.

"Haloalkenyl" refers to an alkenyl radical, as defined above, that is further substituted by one or more halo substituents. The number of halo substituents included in haloalkenyl is from one and up to the total number of the hydrogen atoms available for replacement with the halo substituents (e.g., perfluoroalkenyl). Non-limiting examples of haloalkenyl include 2,2-difluoroethenyl, 3-chloroprop-1-enyl, and the like. For an optionally substituted haloalkenyl, the hydrogen atoms bonded to the carbon atoms of the alkenyl part of the haloalkenyl radical may be optionally replaced with substitutents as defined above for an optionally substituted alkenyl group.

"Haloalkynyl" refers to an alkynyl radical, as defined above, that is further substituted by one or more halo substituents. The number of halo substituents included in haloalkynyl is from one and up to the total number of the hydrogen atoms available for replacement with the halo substituents (e.g., perfluoroalkynyl). Non-limiting examples of haloalkynyl include 3-chloroprop-1-ynyl and the like. The alkynyl part of the haloalkynyl radical may be additionally optionally substituted as defined above for an alkynyl group.

"Heteroarylalkyl" refers to a radical of the formula -Rₐ-R_{b}, where Rₐ is alkylene and R_{b} is heteroaryl as described herein. Alkylene and heteroaryl portions of optionally substituted heteroarylalkyl are optionally substituted as described herein for alkylene and heteroaryl, respectively.

"Heterocyclyl" refers to a stable 3- to 18-membered non-aromatic ring system radical having the carbon count of two to twelve and containing a total of one to six heteroatoms independently selected from the group consisting of nitrogen, oxygen, phosphorus, and sulfur. A heterocyclyl radical is a monocyclic, bicyclic, tricyclic, or tetracyclic ring system. A bicyclic, tricyclic, or tetracyclic heterocyclyl is a fused, spiro, and/or bridged ring system. The heterocyclyl radical may be saturated or unsaturated. An unsaturated heterocyclyl contains one, two, or three carbon-carbon double bonds and/or one carbon-carbon triple bond. An optionally substituted heterocyclyl is a heterocyclyl radical that is optionally substituted by one, two, three, four, or five substituents independently selected from the group consisting of alkyl, alkenyl, halo, haloalkyl, haloalkenyl, cyano, oxo, thioxo, nitro, aryl, aralkyl, cycloalkyl, heterocyclyl, heteroaryl, - R¹⁵-OR¹⁴, -R¹⁵-OC(O)-R¹⁴, -R¹⁵-N(R¹⁴)₂, -R¹⁵-C(O)R¹⁴,
-R¹⁵-C(O)OR¹⁴, -R¹⁵-C(O)N(R¹⁴)₂, -R¹⁵-N(R¹⁴)C(O)OR¹⁶, -R¹⁵-N(R¹⁴)C(O)R¹⁶, -R¹⁵-N(R¹⁴)S(O)ₜR¹⁶ (where t is 1 or 2), -R¹⁵-S(O)ₜOR¹⁶ (where t is 1 or 2), -R¹⁵-S(O)ₚR¹⁶ (where p is 0, 1, or 2), and -R¹⁵-S(O)ₜN(R¹⁴)₂ (where t is 1 or 2), where each R¹⁴ is independently hydrogen, alkyl, alkenyl, haloalkyl, cycloalkyl, aryl, heterocyclyl, or heteroaryl; each R¹⁵ is independently a direct bond or a linear or branched alkylene or alkenylene chain; and each R¹⁶ is independently alkyl, alkenyl, haloalkyl, cycloalkyl, aryl, heterocyclyl, or heteroaryl. The nitrogen, carbon, or sulfur atoms in the heterocyclyl radical may be optionally oxidized (when the substituent is oxo and is present on the heteroatom); the nitrogen atom may be optionally quaternized (when the substituent is alkyl, alkenyl, aryl, aralkyl, cycloalkyl, heterocyclyl, heteroaryl, - R¹⁵-OR¹⁴, -R¹⁵-OC(O)-R¹⁴, -R¹⁵-N(R¹⁴)₂, -R¹⁵-C(O)R¹⁴, -R¹⁵-C(O)OR¹⁴, -R¹⁵-C(O)N(R¹⁴)₂, - R¹⁵-N(R¹⁴)C(O)OR¹⁶, -R¹⁵-N(R¹⁴)C(O)R¹⁶, -R¹⁵-N(R¹⁴)S(O)ₜR¹⁶ (where t is 1 or 2), -R¹⁵-S(O)ₜOR¹⁶ (where t is 1 or 2), -R¹⁵-S(O)ₚR¹⁶ (where p is 0, 1, or 2), and -R¹⁵-S(O)ₜN(R¹⁴)₂ (where t is 1 or 2), where R¹⁵ is a linear or branched alkylene or alkenylene chain, and R¹⁴ and R¹⁶ are as defined above). Examples of optionally substituted heterocyclyl radicals include, but are not limited to, azetidinyl, dioxolanyl, thienyl[1,3]dithianyl, decahydroisoquinolyl, imidazolinyl, imidazolidinyl, isothiazolidinyl, isoxazolidinyl, morpholinyl, octahydroindolyl, octahydroisoindolyl, 2-oxopiperazinyl, 2-oxopiperidinyl, 2-oxopyrrolidinyl, oxazolidinyl, piperidinyl, piperazinyl, 4-piperidonyl, pyrrolidinyl, pyrazolidinyl, thiazolidinyl, tetrahydrofuryl, trithianyl, tetrahydropyranyl, thiomorpholinyl, thiamorpholinyl, 1-oxo-thiomorpholinyl, and 1,1-dioxo-thiomorpholinyl.

"Heterocyclylene" refers to a heterocyclyl in which one hydrogen atom is replaced with a valency. An optionally substituted heterocyclylene is optionally substituted as described herein for heterocyclyl.

"Heteroaryl" refers to a 5- to 18-membered ring system radical containing at least one aromatic ring, having the carbon count of one to seventeen carbon atoms, and containing a total of one to ten heteroatoms independently selected from the group consisting of nitrogen, oxygen, and sulfur. The heteroaryl radical is a monocyclic, bicyclic, tricyclic, or tetracyclic ring system. The bicyclic, tricyclic, or tetracyclic heteroaryl radical is a fused and/or bridged ring system. An optionally substituted heteroaryl is a heteroaryl radical that is optionally substituted by one, two, three, four, or five substituents independently selected from the group consisting of alkyl, alkenyl, alkoxy, halo, haloalkyl, haloalkenyl, cyano, oxo, thioxo, nitro, oxo, aryl, aralkyl, cycloalkyl, heterocyclyl, heteroaryl, or heteroarylalkyl, - R¹⁵-OR¹⁴, -R¹⁵-OC(O)-R¹⁴, -R¹⁵-N(R¹⁴)₂, -R¹⁵-C(O)R¹⁴, -R¹⁵-C(O)OR¹⁴, -R¹⁵-C(O)N(R¹⁴)₂, - R¹⁵-N(R¹⁴)C(O)OR¹⁶, -R¹⁵-N(R¹⁴)C(O)R¹⁶, -R¹⁵-N(R¹⁴)S(O)ₜR¹⁶ (where t is 1 or 2), -R¹⁵-S(O)ₜOR¹⁶ (where t is 1 or 2), -R¹⁵-S(O)ₜR¹⁶ (where p is 0, 1, or 2), and -R¹⁵-S(O)ₜN(R¹⁴)₂ (where t is 1 or 2), where each R¹⁴ is independently hydrogen, alkyl, alkenyl, haloalkyl, cycloalkyl, aryl, heterocyclyl, or heteroaryl; each R¹⁵ is independently a direct bond or a linear or branched alkylene or alkenylene chain; and each R¹⁶ is alkyl, alkenyl, haloalkyl, cycloalkyl, aryl, heterocyclyl, or heteroaryl. The nitrogen, carbon, or sulfur atoms in the heterocyclyl radical may be optionally oxidized (when the substituent is oxo and is present on the heteroatom), provided that at least one ring in heteroaryl remains aromatic; the nitrogen atom may be optionally quaternized (when the substituent is alkyl, alkenyl, aryl, aralkyl, cycloalkyl, heterocyclyl, heteroaryl, -R¹⁵-OR¹⁴, -R¹⁵-OC(O)-R¹⁴, -R¹⁵-N(R¹⁴)₂, -R¹⁵-C(O)R¹⁴, -R¹⁵-C(O)OR¹⁴, -R¹⁵-C(O)N(R¹⁴)₂, -R¹⁵-N(R¹⁴)C(O)OR¹⁶, -R¹⁵-N(R¹⁴)C(O)R¹⁶, -R¹⁵-N(R¹⁴)S(O)ₜR¹⁶ (where t is 1 or 2), -R¹⁵-S(O)ₜOR¹⁶ (where t is 1 or 2), -R¹⁵-S(O)ₚR¹⁶ (where p is 0, 1, or 2), and -R¹⁵-S(O)ₜN(R¹⁴)₂ (where t is 1 or 2), where R¹⁵ is a linear or branched alkylene or alkenylene chain, and R¹⁴ and R¹⁶ are as defined above), provided that at least one ring in heteroaryl remains aromatic. Examples of optionally substituted heteroaryl radicals include, but are not limited to, azepinyl, acridinyl, benzimidazolyl, benzthiazolyl, benzindolyl, benzodioxolyl, benzofuranyl, benzooxazolyl, benzothiazolyl, benzothiadiazolyl, benzo[b][1,4]dioxepinyl, 1,4-benzodioxanyl, benzonaphthofuranyl, benzoxazolyl, benzodioxolyl, benzodioxinyl, benzopyranyl, benzopyranonyl, benzofuranyl, benzofuranonyl, benzothienyl (benzothiophenyl), benzotriazolyl, benzo[4,6]imidazo[1,2-a]pyridinyl, carbazolyl, cinnolinyl, dibenzofuranyl, dibenzothiophenyl, furanyl, furanonyl, isothiazolyl, imidazolyl, indazolyl, indolyl, indazolyl, isoindolyl, indolinyl, isoindolinyl, isoquinolyl, indolizinyl, isoxazolyl, naphthyl, naphthyridinyl, oxadiazolyl, 2-oxoazepinyl, oxazolyl, oxiranyl, 1-phenyl-1*H*-pyrrolyl, phenazinyl, phenothiazinyl, phenoxazinyl, phthalazinyl, pteridinyl, purinyl, pyrrolyl, pyrazolyl, pyridinyl, pyrazinyl, pyrimidinyl, pyridazinyl, pyrrolyl, quinazolinyl, quinoxalinyl, quinolinyl, quinuclidinyl, isoquinolinyl, tetrahydroquinolinyl, thiazolyl, thiadiazolyl, triazolyl, tetrazolyl, triazinyl and thiophenyl (i.e. thienyl).

The invention disclosed herein is also meant to encompass all pharmaceutically acceptable compounds of formula (I) being isotopically-labelled by having one or more atoms replaced by an atom having a different atomic mass or mass number. Examples of isotopes that can be incorporated into the disclosed compounds include isotopes of hydrogen, carbon, nitrogen, oxygen, phosphorous, fluorine, chlorine, and iodine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ³⁶Cl, ¹²³I, and ¹²⁵I, respectively. These radiolabelled compounds could be useful to help determine or measure the effectiveness of the compounds, by characterizing, for example, the site or mode of action on ATM and DNA-PK enzymes, or binding affinity to pharmacologically important site of action on ATM and DNA-PK enzymes. Certain isotopically-labelled compounds of formula (I), for example, those incorporating a radioactive isotope, are useful in drug and/or substrate tissue distribution studies. The radioactive isotopes tritium, *i.e.* ³H, and carbon-14, *i.e.* ¹⁴C, are particularly useful for this purpose in view of their ease of incorporation and ready means of detection.

Substitution with heavier isotopes such as deuterium, *i.e.* ²H, may afford certain therapeutic advantages resulting from greater metabolic stability, for example, increased *in vivo* half-life or reduced dosage requirements, and hence may be preferred in some circumstances.

Substitution with positron emitting isotopes, such as ¹¹C, ¹⁸F, ¹⁵O and ¹³N, can be useful in Positron Emission Topography (PET) studies for examining substrate receptor occupancy. Isotopically-labeled compounds of formula (I) can generally be prepared by conventional techniques known to those skilled in the art or by processes analogous to those described in the Examples and Preparations as set out below using an appropriate isotopically-labeled reagent in place of the non-labeled reagent previously employed.

The invention disclosed herein is also meant to encompass the *in vivo* metabolic products of the disclosed compounds. Such products may result from, for example, the oxidation, reduction, hydrolysis, amidation, esterification, and the like of the administered compound, primarily due to enzymatic processes. Accordingly, the invention includes compounds produced by a process comprising contacting a compound of this invention with a mammal for a period of time sufficient to yield a metabolic product thereof. Such products are typically identified by administering a radiolabelled compound of the invention in a detectable dose to an animal, such as rat, mouse, guinea pig, canine, monkey, or to human, allowing sufficient time for metabolism to occur, and isolating its conversion products from the urine, blood or other biological samples.

"Stable compound" and "stable structure" are meant to indicate a compound that is sufficiently robust to survive isolation to a useful degree of purity from a reaction mixture, and formulation into an efficacious therapeutic agent.

"Mammal" includes humans and both domestic animals such as laboratory animals and household pets, (e.g. cats, dogs, swine, cattle, sheep, goats, horses, rabbits), and non-domestic animals such as wildlife and the like.

"Optional" or "optionally" means that the subsequently described event of circumstances may or may not occur, and that the description includes instances where said event or circumstance occurs and instances in which it does not. For example, "optionally substituted aryl" means that the aryl radical may or may not be substituted and that the description includes both substituted aryl radicals and aryl radicals having no substitution.

"Patient" means a human or non-human animal (e.g., a mammal) that is suffering from a disease or condition, as determined by a qualified professional (e.g., a doctor, nurse practitioner, or veterinarian) with or without known in the art laboratory test(s) of sample(s) from the patient.

"Pharmaceutically acceptable carrier, diluent or excipient" includes without limitation any adjuvant, carrier, excipient, glidant, sweetening agent, diluent, preservative, dye/colorant, flavor enhancer, surfactant, wetting agent, dispersing agent, suspending agent, stabilizer, isotonic agent, solvent, or emulsifier which has been approved by the United States Food and Drug Administration as being acceptable for use in humans or domestic animals.

"Pharmaceutically acceptable salt," as used herein, represents those salts which are, within the scope of sound medical judgment, suitable for use in contact with the tissues of humans and animals without undue toxicity, irritation, allergic response and the like and are commensurate with a reasonable benefit/risk ratio. Pharmaceutically acceptable salts are well known in the art. For example, pharmaceutically acceptable salts are described in: Berge et al., J. Pharmaceutical Sciences 66:1-19, 1977 and in Pharmaceutical Salts: Properties, Selection, and Use, (Eds. P.H. Stahl and C.G. Wermuth), Wiley-VCH, 2008. Pharmaceutically acceptable salts include acid and base addition salts.

"Pharmaceutically acceptable acid addition salt" refers to those salts which retain the biological effectiveness and properties of the free bases, which are not biologically or otherwise undesirable, and which are formed with inorganic acids such as, but are not limited to, hydrochloric acid, hydrobromic acid, sulfuric acid, nitric acid, phosphoric acid and the like, and organic acids such as, but not limited to, acetic acid, 2,2-dichloroacetic acid, adipic acid, alginic acid, ascorbic acid, aspartic acid, benzenesulfonic acid, benzoic acid, 4-acetamidobenzoic acid, camphoric acid, camphor-10-sulfonic acid, capric acid, caproic acid, caprylic acid, carbonic acid, cinnamic acid, citric acid, cyclamic acid, dodecylsulfuric acid, ethane-1 ,2-disulfonic acid, ethanesulfonic acid, 2-hydroxyethanesulfonic acid, formic acid, fumaric acid, galactaric acid, gentisic acid, glucoheptonic acid, gluconic acid, glucuronic acid, glutamic acid, glutaric acid, 2-oxo-glutaric acid, glycerophosphoric acid, glycolic acid, hippuric acid, isobutyric acid, lactic acid, lactobionic acid, lauric acid, maleic acid, malic acid, malonic acid, mandelic acid, methanesulfonic acid, mucic acid, naphthalene-1 ,5-disulfonic acid, naphthalene-2-sulfonic acid, 1-hydroxy-2-naphthoic acid, nicotinic acid, oleic acid, orotic acid, oxalic acid, palmitic acid, pamoic acid, propionic acid, pyroglutamic acid, pyruvic acid, salicylic acid, 4-aminosalicylic acid, sebacic acid, stearic acid, succinic acid, tartaric acid, thiocyanic acid, *p*-toluenesulfonic acid, trifluoroacetic acid, undecylenic acid and the like.

"Pharmaceutically acceptable base addition salt" refers to those salts which retain the biological effectiveness and properties of the free acids, which are not biologically or otherwise undesirable. These salts are prepared from addition of an inorganic base or an organic base to the free acid. Salts derived from inorganic bases include, but are not limited to, the sodium, potassium, lithium, ammonium, calcium, magnesium, iron, zinc, copper, manganese, aluminum salts and the like. Preferred inorganic salts are the ammonium, sodium, potassium, calcium, and magnesium salts. Salts derived from organic bases include, but are not limited to, salts of primary, secondary, and tertiary amines, substituted amines including naturally occurring substituted amines, cyclic amines and basic ion exchange resins, such as ammonia, isopropylamine, trimethylamine, diethylamine, triethylamine, tripropylamine, diethanolamine, ethanolamine, deanol, 2-dimethylaminoethanol, 2-diethylaminoethanol, dicyclohexylamine, lysine, arginine, histidine, caffeine, procaine, hydrabamine, choline, betaine, benethamine, benzathine, ethylenediamine, glucosamine, methylglucamine, theobromine, triethanolamine, tromethamine, purines, piperazine, piperidine, *N*-ethylpiperidine, polyamine resins and the like. Particularly preferred organic bases are isopropylamine, diethylamine, ethanolamine, trimethylamine, dicyclohexylamine, choline and caffeine.

Often crystallizations produce a solvate of the compound of the invention. As used herein, the term "solvate" refers to an aggregate that comprises one or more molecules of a compound of the invention with one or more molecules of solvent. The solvent may be water, in which case the solvate may be a hydrate. Alternatively, the solvent may be an organic solvent. Thus, the compounds of the present invention may exist as a hydrate, including a monohydrate, dihydrate, hemihydrate, sesquihydrate, trihydrate, tetrahydrate and the like, as well as the corresponding solvated forms. The compound of the invention may be true solvates, while in other cases, the compound of the invention may merely retain adventitious water or be a mixture of water plus some adventitious solvent.

A "pharmaceutical composition" refers to a formulation of a compound of the invention and a medium generally accepted in the art for the delivery of the biologically active compound to mammals, e.g., humans. Such a medium includes all pharmaceutically acceptable carriers, diluents, or excipients therefor.

"Therapeutically effective amount" refers to that amount of a compound of the invention which, when administered to a mammal, preferably a human, is sufficient to effect treatment, as defined below, in the mammal, preferably a human or canine. The amount of a compound of the invention, or another pharmaceutical agent (e.g., an anti-tumor agent), which constitutes a "therapeutically effective amount" will vary depending on the compound, the condition and its severity, the manner of administration, and the age of the mammal to be treated, but can be determined routinely by one of ordinary skill in the art having regard to his own knowledge and to this disclosure.

"Treating" or "treatment" as used herein covers the treatment of the disease or condition of interest in a mammal, preferably a human, having the disease or condition of interest, and includes:
(i) preventing the disease or condition from occurring in a mammal, in particular, when such mammal is predisposed to the condition but has not yet been diagnosed as having it;
(ii) inhibiting the disease or condition, *i.e.,* arresting its development;
(iii) relieving the disease or condition, *i.e.,* causing regression of the disease or condition; or
(iv) relieving the symptoms resulting from the disease or condition, *i.e.,* relieving pain without addressing the underlying disease or condition. As used herein, the terms "disease" and "condition" may be used interchangeably or may be different in that the particular malady or condition may not have a known causative agent (so that etiology has not yet been worked out) and it is therefore not yet recognized as a disease but only as an undesirable condition or syndrome, wherein a more or less specific set of symptoms have been identified by clinicians.

The compounds of the invention, or their pharmaceutically acceptable salts may contain one or more asymmetric centres and may thus give rise to enantiomers, diastereomers, and other stereoisomeric forms that may be defined, in terms of absolute stereochemistry, as (*R*)- or (*S*)- or, as (D)- or (L)-for amino acids. The present invention is meant to include all such possible isomers, as well as their racemic and optically pure forms. Optically active (+) and (-), (*R*)- and (*S*)-, or (D)- and (L)-isomers may be prepared using chiral synthons or chiral reagents, or resolved using conventional techniques, for example, chromatography and fractional crystallisation. Conventional techniques for the preparation/isolation of individual enantiomers include chiral synthesis from a suitable optically pure precursor or resolution of the racemate (or the racemate of a salt or derivative) using, for example, chiral high-pressure liquid chromatography (HPLC). When the compounds described herein contain olefinic double bonds or other centres of geometric asymmetry, and unless specified otherwise, it is intended that the compounds include both E and Z geometric isomers. Likewise, all tautomeric forms are also intended to be included.

A "stereoisomer" refers to a compound made up of the same atoms bonded by the same bonds but having different three-dimensional structures, which are not interchangeable. The present invention contemplates various stereoisomers and mixtures thereof and includes "enantiomers", which refers to two stereoisomers whose molecules are nonsuperimposeable mirror images of one another.

A "tautomer" refers to a proton shift from one atom of a molecule to another atom of the same molecule. The present invention includes tautomers of any said compounds.

Also within the scope of the invention are intermediate compounds of formula (I) and all polymorphs of the aforementioned species and crystal habits thereof.

### BRIEF DESCRIPTION OF THE DRAWINGS

FIG. 1 is an image of an immunoblot from a representative experiment assessing the effect of compound 569 on MCF7 cells with or without radiation.
FIG. 2 is a graph showing the results of a clonogenic survival assay examining the MCF7 cell viability after exposure to vehicle (DMSO) or compound 569 with or without radiation.
FIG. 3 is an image of an immunoblot showing the induction of phosphorylation of TBK1 by compound 569, a selective ATM inhibitor (ATMi), a selective DNA-PK inhibitor (DNA-PKi), and a combination of the selective ATM inhibitor and the selective DNA-PK inhibitor (Ai+Di) in HCT116 cells expressing wild-type p53 or HCT116 cells that were negative for p53 expression. In FIG. 3, p.ATM, p.DNA-PK, p.TBK1, and p.STING indicate phosphorylated forms of ATM, DNA-PK, TBK1, and STING, respectively.
FIG. 4 is an immunoblot showing the inhibition of radiation-induced autophosphorylation of DNA-PK kinase and radiation-induced phosphorylation of KAP1, an ATM substrate, by compound 569 in FADU head and neck squamous cell carcinoma (HNSCC) human tumor xenografts. In FIG. 4, pDNA-PK and pKAP1 indicate phosphorylated forms of DNA-PK and KAP1, respectively.
FIG. 5 is an immunoblot showing the inhibition of radiation-induced autophosphorylation of DNA-PK kinase and radiation-induced phosphorylation of KAP1, an ATM substrate, by compound 569 in MDA-MB-231 breast carcinoma human tumor xenografts. In FIG. 5, pDNA-PK and pKAP1 indicate phosphorylated forms of DNA-PK and KAP1, respectively.
FIG. 6 illustrates the dosing of a FADU subcutaneous human xenograft mouse model with compound 569 and/or IR, qd × 3. The median relative tumor volume over time for each group in the study are shown. In FIG. 6, "569" means compound 569, "Veh." means vehicle, and "Rad." means radiation.
FIG. 7 represents the Kaplan-Meier quintupling-free survival for each group dosed in the FADU subcutaneous human xenograft mouse model with compound 569 and/or IR, qd × 3. In FIG. 7, "569" means compound 569, "Veh." means vehicle, and "Rad." means radiation.
FIG.8 illustrates the dosing of a MDA-MB-231 subcutaneous human xenograft mouse model with compound 569 and/or IR, qd × 3. The median relative tumor volume over time for each group in the study are shown. In FIG. 8, "569" means compound 569, "Veh." means vehicle, and "Rad." means radiation.
FIG. 9 represents the Kaplan-Meier quintupling-free survival data for each group dosed in the MDA-MB-231 subcutaneous human xenograft mouse model with compound 569 and/or IR, qd × 3. In FIG. 9, "569" means compound 569, "Veh." means vehicle, and "Rad." means radiation.

### COMPOUNDS, COMPOSITIONS, AND METHODS

The invention provides compounds and compositions that may be useful in the treatment of oncological diseases (e.g., cancer, e.g., those cancers described herein), e.g., alone or in combination with radiotherapy and/or anti-tumor therapy. A compound may be a compound of formula (I): 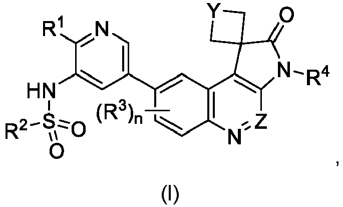 or a pharmaceutically acceptable salt thereof,
wherein
Z is CH, CR³, or N;
Y is CHR⁵ or NR⁶;
n is 0, 1, 2, or 3;
R¹ is -O-L-N(R⁷)₂ or optionally substituted, four-memberred, saturated *N*-heterocyclyl;
R² is C₁₋₃ alkyl;
each R³ is independently halogen or optionally substituted C₁₋₃ alkyl;
R⁴ is optionally substituted alkyl;
R⁵ is hydrogen, optionally substituted C₁₋₃ alkyl, or benzyloxy;
R⁶ is optionally substituted C₁₋₃ alkyl;
each R⁷ is independently H or optionally substituted C₁₋₃ alkyl; and
L is optionally substituted ethylene.

Advantageously, compounds of the invention (e.g., compound **568, 569,** or **570)** may exhibit superior inhibitory activity for ATM and DNA-PK. Advantageously, compounds of the invention (e.g., compound **568, 569,** or **570)** may exhibit superior selectivity as measured by reduced off-target activity (e.g., mTOR inhibition, PI3K α/δ inhibition, and/or hERG inhibition). For example, a compound of the invention (e.g., compound **568, 569,** or **570)** may have an mTOR IC₅₀ of at least 10 times (e.g., at least 20 times) greater than the ATM IC₅₀ or DNA-PK IC₅₀. A compound of the invention (e.g., compound **568, 569,** or **570)** may have an mTOR IC₅₀ of 10 nM or greater (e.g., > 100 nM). Additionally or alternatively, a compound of the invention (e.g., compound **568, 569,** or **570)** may have an hERG IC₅₀ of at least 100 times (e.g., at least 500 times, at least 1000 times, or at least 3000 times) greater than the ATM IC₅₀ or DNA-PK IC₅₀, when measured at the same compound concentration. A compound of the invention (e.g., compound **568, 569,** or **570)** may have an hERG IC₅₀ of 3 µM or greater (e.g., 10 µM or greater).

Advantageously, compounds of the invention (e.g., compound **568, 569,** or **570)** may exhibit superior pharmacokinetic properties (e.g., Cₘₐₓ, AUC, and/or t_{1/2}).

In some embodiments, the compound is selected from the group consisting of:

| |
|---|
| *trans*-*N*-(5-(3-Ethyl-7'-fluoro-3'-methyl-2'-oxo-2',3'-dihydrospiro[cyclobutane-1,1'-pyrrolo[2,3-*c*]quinolin]-8'-yl)-2-(2-(isopropylamino)ethoxy)pyridin-3-yl)methanesulfonamide |
| *trans-N*-(5-(3-(Benzyloxy)-7'-fluoro-3'-methyl-2'-oxo-2',3'-dihydrospiro[cyclobutane-1,1'-pyrrolo[2,3-c]quinolin]-8'-yl)-2-(2-(isopropylamino)ethoxy)pyridin-3-yl)methanesulfonamide |
| *N*-(5-(7'-Fluoro-3'-methyl-2'-oxo-2',3'-dihydrospiro[cyclobutane-1,1'-pyrrolo[2,3-*c*]quinolin]-8'-yl)-2-(2-(isopropylamino)ethoxy)pyridin-3-yl)propane-2-sulfonamide |
| *N*-(5-(7'-Fluoro-3'-methyl-2'-oxo-2',3'-dihydrospiro[cyclobutane-1,1'-pyrrolo[2,3-*c*]quinolin]-8'-yl)-2-(2-(isopropylamino)ethoxy)pyridin-3-yl)methanesulfonamide hydrochloride |
| *N*-(5-(7'-Fluoro-3'-methyl-2'-oxo-2',3'-dihydrospiro[cyclobutane-1,1'-pyrrolo[2,3-*c*]quinolin]-8'-yl)-2-(2-(isopropylamino)ethoxy)pyridin-3-yl)ethanesulfonamide hydrochloride |
| *N*-(5-(7'-Fluoro-1-isopropyl-3'-methyl-2'-oxo-2',3'-dihydrospiro[azetidine-3,1'-pyrrolo[2,3-*c*]quinolin]-8'-yl)-2-(2-(isopropylamino)ethoxy)pyridin-3-yl)methanesulfonamide |
| *cis*-*N*-(5-(3-(Benzyloxy)-7'-fluoro-3'-methyl-2'-oxo-2',3'-dihydrospiro[cyclobutane-1,1'-pyrrolo[2,3-*c*]quinolin]-8'-yl)-2-(2-(isopropylamino)ethoxy)pyridin-3-yl)methanesulfonamide |
| *cis*-*N*-(5-(3-Ethyl-7'-fluoro-3'-methyl-2'-oxo-2',3'-dihydrospiro[cyclobutane-1,1'-pyrrolo[2,3-*c*]quinolin]-8'-yl)-2-(2-(isopropylamino)ethoxy)pyridin-3-yl)methanesulfonamide |
| *N*-(2-(3-(Dimethylamino)azetidin-1-yl)-5-(3'-methyl-2'-oxo-2',3'-dihydrospiro[cyclobutane-1,1'-pyrrolo[2,3-c]quinolin]-8'-yl)pyridin-3-yl)methanesulfonamide hydrochloride |

The compounds of the invention are advantageous in that they can inhibit ATM (ataxia-telangiectasia, mutated) and DNA-PK kinases. The ATM (ataxia-telangiectasia, mutated) and DNA-PK kinases, in particular, are important modulators of cellular responses to DNA breakage and inhibition of either of these molecules markedly increases the sensitivity of cells to ionizing radiation. Thus, the compounds of the invention can be effective inhibitors of the actions of ATM and DNA-PK with or without radiation and with or without chemotherapy or immunotherapy to provide effective therapy for the treatment of oncological diseases (e.g., cancer, e.g., those cancers described herein). The treatment of a patient with a compound of the invention can delay or eliminate the repair of DNA damage by radiation therapy. As a result, patients receiving a compound of the invention may respond better to anti-tumor therapies. Advantageously, patients receiving a compound of the invention may derive therapeutic benefit by increasing tumor control from standard doses of radiation therapy or by achieving similar levels of tumor control from lower doses of ionizing radiation than routinely used in patients not receiving a compound of the invention. Advantageously, lower doses of ionizing radiation may be less damaging to non-cancerous tissues than the doses necessary for patients not receiving a compound of the invention.

Humans and mice having loss-of-function mutations in the ATM or PRKDC genes, which encode Ataxia Telangiectasia Mutated (ATM) kinase and DNA-dependent Protein Kinase (DNA-PK), respectively, are hypersensitive to ionizing radiation. Inhibition of ATM and DNA-PK kinases together can be effective in sensitizing tumor cells to radiation or DNA damaging agents (e.g., anti-tumor agents). The efficacy of dual inhibition of ATM and DNA-PK kinases may be superior to inhibition of either kinase by itself.

In addition, compounds of the invention may advantageously exhibit reduced inhibition of other kinases (ATR and mTOR) and thus may exhibit reduced toxicity.

Compounds of the invention may sensitize tumor cells to radiation and/or anti-tumor agents.

### Methods

In another aspect, the invention provides methods for the treatment of an oncological disease (e.g., cancer) in a mammal, preferably human or canine, wherein the methods comprise administering to the mammal in need thereof a therapeutically effective amount of a compound of the invention. In some embodiments, the compound is administered to the mammal receiving radiotherapy.

In another aspect, the invention provides methods for the treatment of an oncological disease (e.g., cancer) in a mammal, wherein the methods comprise administering to the mammal in need thereof a therapeutically effective amount of a compound of the invention. In some embodiments, the compound is administered to the mammal in combination with a DNA-damaging agent. Non-limiting examples of DNA-damaging agents include cisplatin, oxaliplatin, carboplatin, valrubicin, idarubicin, calicheamicin, PARP inhibitors.

In another aspect, the invention provides pharmaceutical compositions comprising the compounds of the invention and pharmaceutically acceptable excipients. In one embodiment, the pharmaceutical composition comprises a compound of the invention in a pharmaceutically acceptable carrier and in an amount effective to treat an oncoligcal disease in an animal, preferably a mammal.

A compound of the invention, when used in a combination therapy, may increase the potency of the other radiation or drug therapy if it allows the dose of the other treatment to be reduced, which may reduce the frequency and/or severity of adverse events associated with the other drug therapy. For example, side effects of radiation (e.g., oral or gastrointestinal mucositis, dermatitis, pneumonitis, or fatigue) may be reduced in patients receiving a combination therapy including a compound of the invention and reduced dose radiotherapy (e.g., incidence of the adverse events may be reduced by at least 1%, 5%, 10%, or 20%) relative to patients receiving standard full dose radiotherapy without a compound of the invention. Additionally, other adverse events that may be reduced in patients receiving a combination therapy including a compound of the invention and reduced dose radiotherapy (e.g., incidence of the adverse events may be reduced by at least 1%, 5%, 10%, or 20%) relative to patients receiving standard full dose radiotherapy without a compound of the invention may be late effects of radiation, e.g., radiation-induced lung fibrosis, cardiac injury, bowel obstruction, nerve injury, vascular injury, lymphedema, brain necrosis, or radiation-induced cancer. Similarly, when the compound is administered in a combination therapy with another anti-cancer drug (e.g., those described herein), the combined therapy may cause the same or even increased tumor cell death, even when the dose of the other anti-cancer drug is lowered. Reduced dosages of other anti-cancer drugs thus may reduce the severity of adverse events caused by the other anti-cancer drugs.

In another aspect, this invention is directed to the use of the compounds of the invention, as set forth above, as a stereoisomer, enantiomer, tautomer thereof or mixtures thereof, or a pharmaceutically acceptable salt or solvate thereof, or the use of a pharmaceutical composition comprising a pharmaceutically acceptable excipient and a compound of the invention, as set forth above, as a stereoisomer, enantiomer, tautomer thereof or mixtures thereof, or a pharmaceutically acceptable salt or solvate thereof, in the preparation of a medicament for use in the treatment of a disease. In some embodiments, the compound of the invention is administered in combination with radiotherapy. In other embodiments, the compound of the invention is administered in combination with a DNA damaging agent. In further embodiments, the compound of the invention is administered in combination with an anti-tumor immunotherapeutic agent (e.g., ipilimumab, ofatumumab, nivolumab, pembrolizumab, atezolizumab, avelumab, durvalumab, cemiplimab, obinutuzumab, ocaratuzumab, tremelimumab, spartalizumab, camrelizumab, sintilimab, tislelizumab, toripalimab, dostarlimab, veltuzumab, INCMGA00012, AMP-224, AMP-514, KN035, CK-301, AUNP12, CA-170, or BMS-986189). In other embodiments, the anti-tumor immunotherapeutic agent is ofatumumab, obinutuzumab, ocaratuzumab, or veltuzumab. In yet other embodiments, the anti-tumor immunotherapeutic agent is nivolumab, pembrolizumab, cemiplimab, spartalizumab, camrelizumab, sintilimab, tislelizumab, toripalimab, dostarlimab, INCMGA00012, AMP-224, or AMP-514. In still other embodiments, the anti-tumor immunotherapeutic agent is atezolizumab, avelumab, durvalumab, KN035, CK-301, AUNP12, CA-170, or BMS-986189. In certain preferred combination therapy embodiments, the compound of the invention is administered in combination with an anti-tumor immunotherapeutic agent.

Methods of the invention may be used in the treatment of an oncological disease as described herein. An oncological disease may be, e.g., a premalignant tumor or a malignant tumor (e.g., a solid tumor or a liquid tumor). Malignant tumors are typically referred to as cancers. In certain embodiments, the oncological disease is cancer.

In further embodiments, examples of cancer to be treated using methods and uses disclosed herein include but are not limited to hematologic cancers, e.g., leukemias and lymphomas. Non-limiting examples of cancers include acute myelogenous leukemia, acute lymphoblastic leukemia, acute megakaryocytic leukemia, promyelocytic leukemia, erythroleukemia, lymphoblastic T cell leukemia, chronic myelogenous leukemias, chronic lymphocytic leukemia, hairy-cell leukemia, chronic neutrophilic leukemia, plasmacytoma, immunoblastic large cell leukemia, mantle cell leukemia, multiple myelomas, malignant lymphoma, diffuse large B-cell lymphoma, Hodgkin's lymphoma, non-Hodgkin's lymphoma, lymphoblastic T cell lymphoma, Burkitt's lymphoma, and follicular lymphoma.

In yet further embodiments, examples of cancer to be treated using methods and uses disclosed herein include but are not limited to solid tumors. Non-limiting examples of solid tumors include brain cancers (e.g., astrocytoma, glioma, glioblastoma, medulloblastoma, or ependymoma), bladder cancer, breast cancer, central nervous system cancers, cervical cancer, colon cancer, endometrial cancer, esophageal cancer, gastrointestinal stromal tumor, gastric cancer, head and neck cancers, buccal cancer, cancer of the mouth, hepatocellular cancer, lung cancer, melanoma, Merkel cell carcinoma, mesothelioma, nasopharyngeal cancer, neuroblastoma, osteosarcoma, ovarian cancer, pancreatic cancer, prostate cancer, renal cancer, salivary gland cancer, sarcomas, testicular cancer, urothelial cancer, vulvar cancer, and Wilm's tumor. Preferably, the methods of the invention are used in the treatment of lung cancer, head and neck cancer, pancreatic cancer, rectal cancer, glioblastoma, hepatocellular carcinoma, cholangiocarcinoma, metastic liver lesions, melanoma, bone sarcoma, soft tissue sarcoma, endometrial cancer, cervical cancer, prostate cancer, or Merkel cell carcinoma.

In still further embodiments, examples of cancer to be treated using methods and uses disclosed herein but are not limited to metastases and metastatic cancer. For example, the methods and uses disclosed herein for treating cancer may involve treatment of both primary tumors and metastases.

In some embodiments, methods of the invention may reduce the tumor size in a subject, e.g., at least by 10%, 20%, 30%, 40%, 50%, 60%, 70%, 80%, or 90%, or may eliminate the tumor (e.g., relative to the tumor size at the time of the commencement of the therapy or relative to a reference subject that receives placebo instead of the compound of the invention). In some embodiments, methods of the invention may reduce the tumor burden in a subject, e.g., at least by 10%, 20%, 30%, 40%, 50%, 60%, 70%, 80%, or 90%, or may eliminate the tumor (e.g., relative to the tumor burden at the time of the commencement of the therapy or relative to a reference subject that receives placebo instead of the compound of the invention). In some embodiments, methods of the invention may increase mean survival time of the subject, e.g., by at least 10%, 20%, 30%, 40%, 50%, 60%, 70%, 80%, 90%, 100%, 150%, or 200% (e.g., relative to a reference subject that receives placebo instead of the compound of the invention). In some embodiments, methods of the invention may increase the ability of radiation therapy or drug therapy to palliate pain or other symtoms for a longer mean time for the subject, e.g., by at least 10%, 20%, 30%, 40%, 50%, 60%, 70%, 80%, 90%, 100%, 150%, or 200% (e.g., relative to a reference subject that receives placebo instead of the compound of the invention).

In some embodiments, the methods and uses disclosed herein comprise the pre-treatment of a patient with a dual an ATM and DNA-PK inhibitor prior to administration of radiation therapy or a DNA damaging agent. Pre-treatment of the patient with a dual ATM and DNA-PK inhibitor may delay or eliminate the repair of DNA damage following radiation therapy.

Radiation therapy includes, but is not limited to, external beam radiation therapy with X-rays (photons), gamma rays from ⁶⁰Cobalt or other radioactive isotopes, neutrons, electrons, protons, carbon ions, helium ions, and other charged particles. Radiation therapy also includes brachytherapy and radiopharmaceuticals that emits gamma rays, alpha particles, beta particles, Auger electrons, or other types of radioactive particles from isotopes including ³²Phosphorus, ⁶⁷Copper, ⁷⁷Bromine, ⁸⁹Strontium, ⁹⁰Yttrium, ¹⁰⁵Rhodium, ¹³¹Iodine, ¹³⁷Cesium, ¹⁴⁹Prometheum, ¹⁵³Samarium, ¹⁶⁶Holmium, ¹⁷⁷Lutetium, ¹⁸⁶Rhenium, ¹⁸⁸Rhenium, ¹⁹⁹Gold, ²¹¹Astatine, ²¹³Bismuth, ²²³Radium, ²²⁵Actinium, or ²²⁷Thorium, ¹⁹²Iridium, ⁶⁷Gallium, ¹⁰³Palladium, ¹²⁵Iodine, and other radioactive isotopes (e.g., ¹⁹²Iridium, ¹²⁵Iodine, ¹³⁷Cesium, ¹⁰³Palladium, ³²Phosphorus, ⁹⁰Yttrium, ⁶⁷Gallium, ²¹¹Astatine, or ²²³Radium). Radiation therapy also includes radioimmunotherapy (RIT) with antibodies or small molecules that are conjugated to radioactive isotopes including ¹³¹Iodine, ⁹⁰Yttrium, ²²⁵Actinium, ²¹¹Astatine, ⁶⁷Gallium, ¹⁷⁷Lutetium, ²²⁷Thorium, and other radioactive isotopes.

In some embodiments, the combination therapy comprises administration to a patient of an ATM and DNA-PK inhibitor and an anti-tumor agent, e.g., cisplatin, oxaliplatin, carboplatin, topoisomerase I inhibitors, topoisomerase II inhibitors, anthracyclines, valrubicin, idarubicin, calicheamicin, PARP inhibitors (e.g., olaparib, rucaparib, niraparib, veliparib, or talazoparib), as well as other anti-cancer agents known to those skilled in the art.

In certain embodiments, the combination therapy comprises administration to a patient of an ATM and DNA-PK inhibitor and an anti-tumor immunotherapeutic agents including by not limited to ipilimumab, ofatumumab, nivolumab, pembrolizumab, atezolizumab, avelumab, durvalumab, etc.

In the combination therapies described herein, an ATM and DNA-PK inhibitor may be administered to the patient simultaneously or sequentially (e.g., before or after) the other drug.

### PREPARATION OF THE COMPOUNDS OF THE INVENTION

The compounds of the present invention can be prepared using methods and techniques known in the art. Suitable processes for synthesizing these compounds are provided in the Examples. Generally, compounds of Formula (I) can be prepared according to the Schemes described below. The sources of the starting materials for these reactions are also described.

Protecting groups may be added or removed in the preparation of the compounds of the invention in accordance with standard techniques, which are known to one skilled in the art and as described herein. The use of protecting groups is described in detail in Greene, T.W. and P.G.M. Wuts, Greene's Protective Groups in Organic Synthesis (2006), 4th Ed., Wiley. The protecting group may also be a polymer resin such as a Wang resin or a 2-chlorotrityl-chloride resin.

It will also be appreciated by those skilled in the art, although such protected derivatives of compounds of this invention may not possess pharmacological activity as such, they may be administered to a mammal and thereafter metabolized in the body to form compounds of the invention which are pharmacologically active.

All of the compounds described below as being prepared which may exist in free base or acid form may be converted to their pharmaceutically acceptable salts by treatment with the appropriate inorganic or organic base or acid. Salts of the compounds prepared below may be converted to their free base or acid form by standard techniques. It is understood that all polymorphs, amorphous forms, anhydrates, hydrates, solvates and salts of the compounds of the invention are intended to be within the scope of the invention. Furthermore, all compounds of the invention which contain an acid or an ester group can be converted to the corresponding ester or acid, respectively, by methods known to one skilled in the art or by methods described herein.

A general representation of preparation of many of these compounds is shown below in **Scheme 1.** Compounds are prepared through the coupling of various components of the molecule: Suzuki coupling of halo substituted compound **3** (or **2'**) with a boronic acid or borate compound **2** (**3'**). Further reactions may or may not be needed to furnish the synthesis of the compounds of this invention. Preparations of specific compounds of this invention are shown in the following **Schemes.**

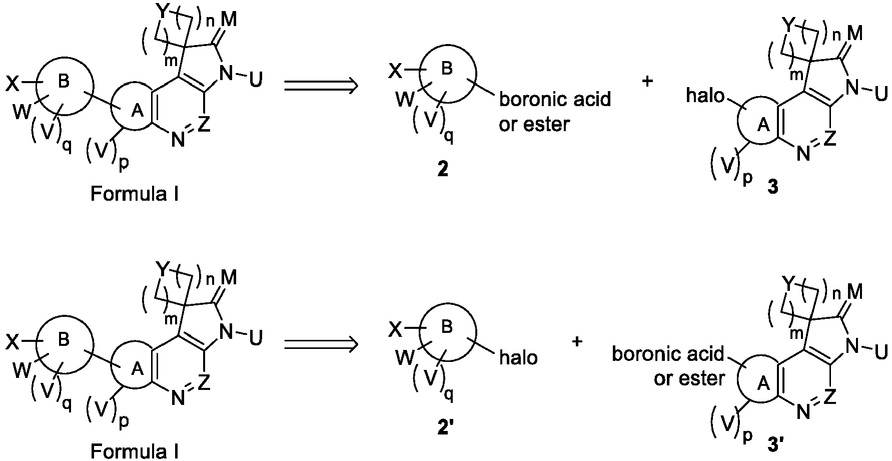

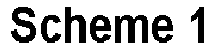

In aryl-aryl coupling reactions, halogen may be iodo, bromo, or chloro, preferable bromo or iodo. In this method, halogen substitutions may be transformed to aryl substitutions using Suzuki coupling reaction conditions. The conditions of this method are disclosed in many publications which have been reviewed by A. Suzuki in an article entitled "The Suzuki reaction with aryl boron compounds in arene chemistry" in *Modern Arene Chemistry* 2002, 53-106. In carrying out this reaction any of the suitable conditions conventional in a Suzuki reaction can be utilized. Generally, Suzuki coupling reactions are carried out in the presence of a transition metal catalyst such as a palladium catalyst utilizing any conventional organic solvent for this reaction and a weak inorganic or organic base. Among the preferred organic solvents are the polar aprotic solvents. Any conventional polar aprotic solvents can be utilized in preparing compounds of the invention. Suitable solvents are customary, especially higher-boiling solvents, e.g. dimethoxyethane. The weak inorganic base can be a carbonate or bicarbonate, such as potassium carbonate or cesium carbonate. The organic base can be an amine such as triethylamine.

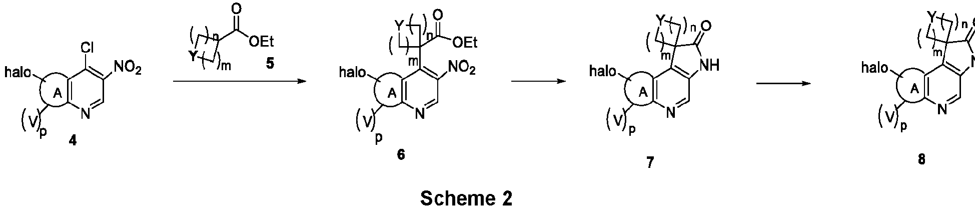

Specifically, the other spiro oxindole intermediate **7** is synthesized as shown in **Scheme 2.** The cyclyl or heterocyclyl substituted ester **5** is treated with a strong base such as, but not limited to, lithium diisopropylamide at low temperature in anhydrous solvent such as, but not limited to, tetrahydrofurn to react with starting material **4,** which is either commercially available or prepared by those skilled in the art following the literature described methods to provide intermediate **6.** Intermediate **6** is reduced by a reducing reagent such as, but not limited to, iron to give the corresponding amino intermediate which cyclizes to provide the oxindole compound **7** *in situ.* Thus, the compound **7** is then *N*-akylated with an alkylating reagent in the presence of a base such as, but not limited to, potassium carbonate or sodium hydride in a polar solvent such as, but not limited to, *N*,*N*-dimethylformamide or tetrahydrofuran thereby to generate the spiro oxindole intermediate **8.**

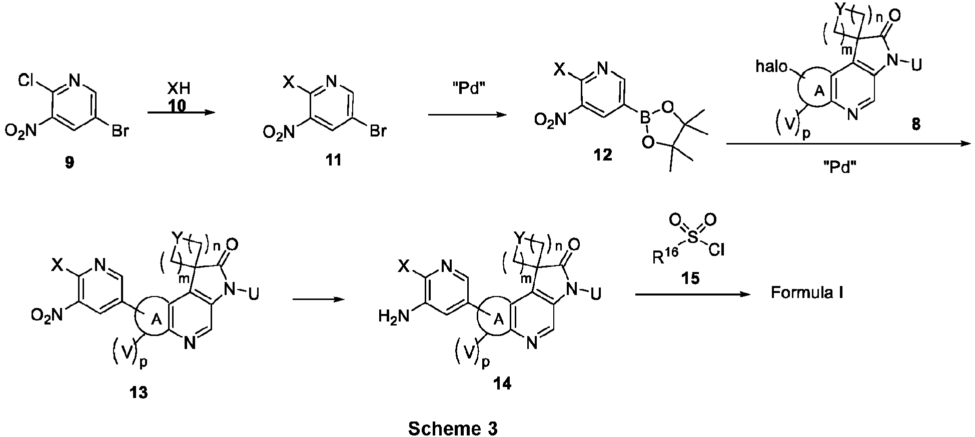

Specifically, the compounds of Formula (I) in this invention can be synthesized as shown in **Scheme 3.** Commercially available 5-bromo-2-chloro-3-nitro-pyridine **(9)** reacts with a nucleophile XH **(10)** in the presence of a strong base such as, but not limited to, sodium hydride to provide intermediate **11.** Under palladium catalyzed conditions, borate **12** can be prepared, which then reacts with the spiro intermediate **8** to provide the cross coupled product **13.** The nitro group in compound **13** is reduced to amino group using a reducing reagent such as, but not limited to, iron to provide intermediate **14.** Reaction of **14** with different sulphonyl chlorides **(15)** furnishes the synthesis of compounds of Formula (I).

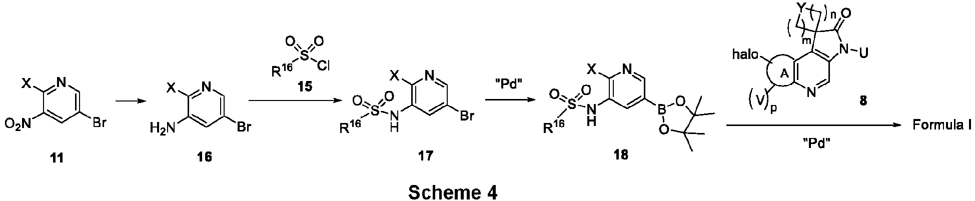

Specifically, the compounds of Formula (I) in this invention can also be synthesized as shown in **Scheme 4.** The nitro group in compound **11** is reduced to amino group using a reducing reagent such as, but not limited to, iron to provide intermediate **16.** Reaction of **16** with different sulphonyl chlorides **(15)** provides the sulphonamide intermediate **17,** which is converted to its corresponding borate **18** under palladium catalysis. Borate **18** can couple with the halo compound **8** under Suzuki reaction conditions to provide the compounds of Formula (I).

In **Scheme 3** and **Scheme 4,** the cross coupled compounds are also synthesizable using Suzuki coupling chemistry with components having reversed the halogen and boronate/boronic acid substitution patterns, for example, as shown in **Scheme 5**

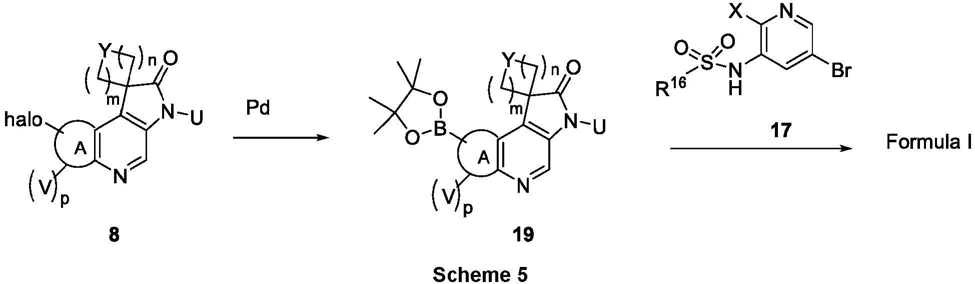

Specifically, the compounds of Formula (I) in this invention can also be synthesized as shown in **Scheme 5.** The halo compound **8** can be converted to its corresponding borate **19** under palladium catalysis. Borate **19** can couple with the halo compound **17** under Suzuki reaction conditions to provide the compounds of Formula (I).

In the practice of the method of the present invention, an effective amount of any one of the compounds of this invention or a combination of any of the compounds of this invention or a pharmaceutically acceptable salt thereof, is administered via any of the usual and acceptable methods known in the art, either singly or in combination. The compounds or compositions can thus be administered orally (e.g., buccal cavity), sublingually, parenterally (e.g., intramuscularly, intravenously, or subcutaneously), rectally (e.g., by suppositories or washings), transdermally (e.g., skin electroporation) or by inhalation (e.g., by aerosol), and in the form or solid, liquid or gaseous dosages, including tablets and suspensions. The administration can be conducted in a single unit dosage form with continuous therapy or in a single dose therapy ad lithium. The therapeutic composition can also be in the form of an oil emulsion or dispersion in conjunction with a lipophilic salt such as pamoic acid, or in the form of a biodegradable sustained-release composition for subcutaneous or intramuscular administration.

Useful pharmaceutical carriers for the preparation of the compositions thereof, can be solids, liquids or gases; thus, the compositions can take the form of tablets, pills, capsules, suppositories, powders, enterically coated or other protected formulations (e.g. binding on ion-exchange resins or packaging in lipid-protein vesicles), sustained release formulations, solutions, suspensions, elixirs, aerosols, and the like. The carrier can be selected from the various oils including those of petroleum, animal, vegetable or synthetic origin, e.g., peanut oil, soybean oil, mineral oil, sesame oil, and the like. Water, saline, aqueous dextrose, and glycols are preferred liquid carriers, particularly (when isotonic with the blood) for injectable solutions. For example, formulations for intravenous administration comprise sterile aqueous solutions of the active ingredient(s) which are prepared by dissolving solid active ingredient(s) in water to produce an aqueous solution, and rendering the solution sterile. Suitable pharmaceutical excipients include starch, cellulose, talc, glucose, lactose, gelatin, malt, rice, flour, chalk, silica, magnesium stearate, sodium stearate, glycerol monostearate, sodium chloride, dried skim milk, glycerol, propylene glycol, water, ethanol, and the like. The compositions may be subjected to conventional pharmaceutical additives such as preservatives, stabilizing agents, wetting or emulsifying agents, salts for adjusting osmotic pressure, buffers and the like. Suitable pharmaceutical carriers and their formulation are described in Remington's Pharmaceutical Sciences by E. W. Martin. Such compositions will, in any event, contain an effective amount of the active compound together with a suitable carrier so as to prepare the proper dosage form for proper administration to the recipient.

The dose of a compound of the present invention depends on a number of factors, such as, for example, the manner of administration, the age and the body weight of the patient, and the condition of the patient to be treated, and ultimately will be decided by the attending physician or veterinarian. Such an amount of the active compound as determined by the attending physician or veterinarian is referred to herein, and in the claims, as an "effective amount".

The invention will now be further described in the Examples below, which are intended as an illustration only and do not limit the scope of the invention.

### EXAMPLES

Reagents were purchased from Aldrich, Sigma, TCI (Shanghai) Development, Chembon Pharmaceutical Co., Ltd, Zhangjiagang Aimate Huaxue Youxiangongsi, Changzhou Qinuo BioTech Co. Ltd, and Shanghai Weiyuan Fine Fluorine Technology Development Co., Ltd or other suppliers as indicated below and used without further purification. Reactions using microwave irradiation for heating were conducted using a Biotage Initiator+. The purification of multi-milligram to multi-gram scale was conducted by methods known to those skilled in the art such as elution of silica gel flash column chromatography; preparative flash column chromatography purifications were also affected in some cases by use of disposal pre-packed silica gel columns (Welch/Agela) eluted with a Biotage CombiFlash system.

For the purpose of judging compound identity and purity, typically, the analytical LC-MS (liquid chromatography/mass spectroscopy) system was used consisted of a Waters ZQ^{™} platform with electrospray ionization in positive ion detection mode with an Agilent 1100 series HPLC with autosampler. The column was usually a Water Xterra MS C18, 3.0 × 50 mm, 5 µm. The flow rate was 1 mL/min, and the injection volume was 10 µL. UV detection was in the range 210-400 nm. The mobile phase consisted of solvent A (water plus 0.06% TFA) and solvent B (acetonitrile plus 0.05% TFA) with a gradient of 100% solvent A for 0.7 min changing to 100% solvent B over 3.75 min, maintained for 1.1 min, then reverting to 100% solvent A over 0.2 min.

For some separations, the use of super critical fluid chromatography may also be useful. Super critical fluid chromatography separations were performed using a Mettler-Toledo Minigram system with the following typical conditions: 100 bar, 30 °C, 2.0 mL/min eluting a 12 mm AD column with 40% MeOH in super critical fluid CO₂. In the case of analytes with basic amino groups, 0.2% isopropyl amine was added to the methanol modifier.

Many compounds of Formula **(I)** were also purified by reverse phase HPLC, using methods well known to those skilled in the art. In some cases, preparative HPLC purification was conducted using PE Sciex 150 EX Mass Spec controlling a Gilson 215 collector attached to a Shimadzu preparative HPLC system and a Leap auto-injector. Compounds were collected from the elution stream using MS detection in the positive ion detection: The elution of compounds from C-18 columns (2.0 × 10 cm eluting at 20 mL/min) was affected using appropriate linear gradation mode over 10 minutes of Solvent (A) 0.05% TFA/H₂O and Solvent (B) 0.035% TFA/acetyl nitrile. For injection on to HPLC systems, the crude samples were dissolved in mixtures of methanol, acetyl nitrile and DMSO.

Compounds were characterized either by ¹H-NMR using a Bruker ADVANCE III HD 400MHz Spectrometer or Bruker AVANCE 300MHz Spectrometer.

### LIST OF ABBREVIATIONS

- DCE: 1,2-dichloroethane
- DCM: dichloromethane
- DIPEA: diisopropylethylamine
- DMF: *N*, *N*-dimethylformamide
- DMSO: dimethylsulfoxide
- EtOAc: ethyl acetate
- HOAc: Acetic acid
- HPLC: high pressure liquid chromatography
- Mel: methyl iodide
- MeOH: methyl alcohol
- MW: microwave
- NMP: 1-methyl-2-pyrrolidinone
- rt: ambient temperature
- TBDMS: *tert*-butyl-dimethylsilyl
- TEA: triethylamine
- TFA: tifluoroacetic acid
- TEMPO: 2,2,6,6-tetramethyl-1-piperidinyloxy
- THF: tetrahydrofuran

### PREPARATION OF COMPOUNDS

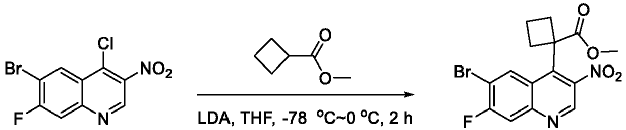

Methyl 1-(6-bromo-7-fluoro-3-nitroquinolin-4-yl)cyclobutane-1-carboxylate: A solution of methyl cyclobutanecarboxylate (0.73 g, 6.38 mmol) in tetrahydrofuran (5.00 mL) was treated with freshly prepared lithium diisopropylamide (6.38 mmol) in tetrahydrofuran (45.0 mL) for 1 hour at -78 °C under nitrogen atmosphere followed by the addition of 6-bromo-4-chloro-7-fluoro-3-nitroquinoline (1.50 g, 4.91 mmol) in portions over 2 min. After stirring for additional 1 hour at ambient temperature, the reaction was quenched by saturated aqueous ammonium chloride (60.0 mL) and diluted with water (120 mL). The resulting mixture was extracted with ethyl acetate (3 × 60.0 mL). The combined organic layers was washed with brine (2 × 50.0 mL) and dried over anhydrous sodium sulfate. After filtration, the filtrate was concentrated under reduced pressure. The residue was purified by silica gel column chromatography, eluted with 1%-2% ethyl acetate in petroleum ether to afford the title compound as a colorless solid (240 mg, 13%): ¹H NMR (400 MHz, CDCl₃) δ 9.14 (s, 1H), 8.20 (d, *J* = 7.2 Hz, 1H), 7.90 (d, *J* = 8.8 Hz, 1H), 3.84 (s, 3H), 3.12-2.99 (m, 1H), 2.58-2.48 (m, 3H), 1.91-1.83 (m, 1H), 1.45-1.27 (m, 1H); MS: [(M + 1)]⁺ = 383.17, 385.17.

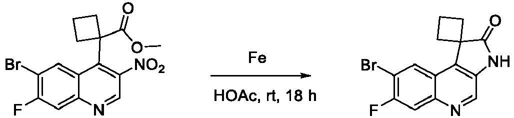

8'-Bromo-7'-fluorospiro[cyclobutane-1,1'-pyrrolo[2,3-*c*]quinolin]-2'(3'*H*)-one: A mixture of methyl 1-(6-bromo-7-fluoro-3-nitroquinolin-4-yl)cyclobutane-1-carboxylate (240 mg, 0.63 mmol) and iron powder (350 mg, 6.26 mmol) in acetic acid (10.0 mL) was stirred for 18 hours at ambient temperature. The resulting mixture was filtered and the filter cake was washed with ethyl acetate (5 × 100 mL). The filtrate was concentrated under reduced pressure. The residue was purified by silica gel column chromatography, eluted with 1%-2% methanol in dichloromethane to afford the title compound as a light yellow solid (100 mg, 50%): ¹H NMR (400 MHz, DMSO-*d*₆) δ 10.75 (s, 1H), 8.68 (s, 1H), 8.52 (d, *J* = 7.5 Hz, 1H), 7.98 (d, *J* = 10.1 Hz, 1H), 2.90-2.75 (m, 2H), 2.50-2.37 (m, 4H); MS: [(M + 1)]⁺ = 321.15, 323.15.

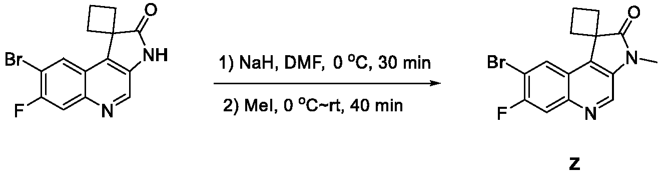

8'-Bromo-7'-fluoro-3'-methylspiro[cyclobutane-1,1'-pyrrolo[2,3-*c*]quinolin]-2'(3'*H*)-one: A solution of 8-bromo-7-fluoro-2,3-dihydrospiro[cyclobutane-1,1-pyrrolo[2,3-*c*]quinoline]-2-one (100 mg, 0.31 mmol) in *N*,*N*-dimethylformamide (10.0 mL) was treated with sodium hydride (19.9 mg, 0.50 mmol, 60% dispersed in mineral oil) at 0 °C for 30 min under nitrogen atmosphere followed by the addition of iodomethane (66.3 mg, 0.47 mmol). After stirring for additional 40 min at ambient temperature, the reaction was quenched by saturated aqueous ammonium chloride (10.0 mL). The resulting mixture was diluted with water (100 mL) and extracted with ethyl acetate (3 × 30.0 mL). The combined organic layers was washed with brine (2 × 20.0 mL) and dried over anhydrous sodium sulfate. After filtration, the filtrate was concentrated under reduced pressure. The residue was purified by Prep-TLC (DCM/MeOH = 20/1, v:v) to afford the title compound as a colorless solid (102 mg, 98%): ¹H NMR (400 MHz, CD₃OD) δ 8.78 (s, 1H), 8.61 (d, *J* = 7.4 Hz, 1H), 7.85 (d, *J* = 9.8 Hz, 1H), 3.36 (s, 3H), 2.94-2.85 (m, 2H), 2.72-2.61 (m, 3H), 2.56-2.48 (m, 1H); MS: [(M + 1)]⁺ = 335.00, 337.00.

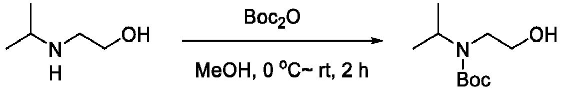

*tert*-Butyl *N*-(2-hydroxyethyl)-*N*-(propan-2-yl)carbamate: To a solution of 2-[(propan-2-yl)amino]ethan-1-ol (40.0 g, 388 mmol) in methanol (300 mL) was added di-*tert*-butyl dicarbonate (127 g, 586 mmol) dropwise at 0 °C. The resulting mixture was stirred for 2 hours at ambient temperature and concentrated under reduced pressure. The residue was purified by silica gel column chromatography, eluted with 0%-4% ethyl acetate in petroleum ether. The desired fractions were collected and concentrated under reduced pressure to afford the title compound as a colorless oil (65.0 g, 82%): ¹H NMR (400 MHz, CDCl₃) δ 4.17 (s, 1H), 3.71 (t, *J* = 5.4 Hz, 2H), 3.30 (t, *J* = 5.4 Hz, 2H), 1.47 (s, 9H), 1.12 (d, *J* = 6.8 Hz, 6H).

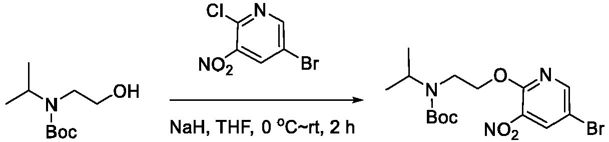

*tert*-Butyl *N*-[2-[(5-bromo-3-nitropyridin-2-yl)oxy]ethyl]-*N*-(propan-2-yl)carbamate: A solution of *tert*-butyl *N-*(2-hydroxyethyl)-*N-*(propan-2-yl)carbamate (15.4 g, 75.8 mmol) in anhydrous tetrahydrofuran (250 mL) was treated with sodium hydride (3.30 g, 82.1 mmol, 60% w/w dispersed in mineral oil) for 1 hour at 0 °C under nitrogen atmosphere followed by the addition of 5-bromo-2-chloro-3-nitropyridine (15.0 g, 63.2 mmol) over 2 min at 0 °C. After additional 2 hours at 25 °C, the reaction was quenched by saturated aqueous ammonium chloride (50.0 mL) and diluted with water (500 mL). The aqueous layer was extracted with ethyl acetate (3 × 150 mL). The combined organic layers was washed with brine (2 × 100 mL) and dried over anhydrous sodium sulfate. After filtration, the filtrate was concentrated under reduced pressure. The residue was purified by silica gel column chromatography, eluted with 1%~18% ethyl acetate in petroleum ether. The desired fractions were collected and concentrated under reduced pressure to afford the title compound as a light yellow oil (18.0 g, 71%): ¹H NMR (400 MHz, CDCl₃) δ 8.42 (d, *J* = 2.4 Hz, 1H), 8.37 (d, *J* = 2.4 Hz, 1H), 4.57 (t, *J* = 6.3 Hz, 2H), 4.32 (s, 1H), 3.51 (t, *J* = 6.3 Hz, 2H), 1.47 (s, 9H), 1.15 (d, *J* = 6.9 Hz, 6H); MS: [(M + 1)]⁺ = 404.00, 406.00.

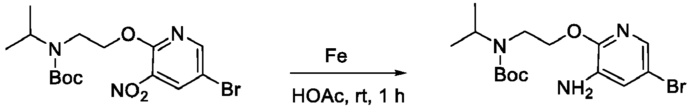

*tert*-Butyl *N*-[2-[(3-amino-5-bromopyridin-2-yl)oxy]ethyl]-*N*-(propan-2-yl)carbamate: To a solution of *tert*-butyl *N*-[2-[(5-bromo-3-nitropyridin-2-yl)oxy]ethyl]-*N*-(propan-2-yl)carbamate (15.0 g, 37.1 mmol) in acetic acid (150 mL) was added iron powder (20.7 g, 371 mmol) at ambient temperature. After stirring for additional 1 hour at ambient temperature, the resulting mixture was filtered and the filtered cake was washed with tetrahydrofuran (4 × 100 mL). The filtrate was concentrated under reduced pressure. The residue was purified by silica gel column chromatography, eluted with 20% ethyl acetate in petroleum ether. The desired fractions were collected and concentrated under reduced pressure to afford the title compound as a colorless solid (12.0 g, 86%): ¹H NMR (400 MHz, CDsOD) δ 7.40 (d, *J* = 2.2 Hz, 1H), 7.04 (d, *J* = 2.2 Hz, 1H), 4.40 (t, *J* = 6.3 Hz, 2H), 4.25-3.99 (m, 1H), 3.52 (t, *J* = 6.3 Hz, 2H), 1.46 (s, 9H), 1.17 (d, *J* = 6.8 Hz, 6H); MS: [(M + 1)]⁺ = 374.10, 376.10.

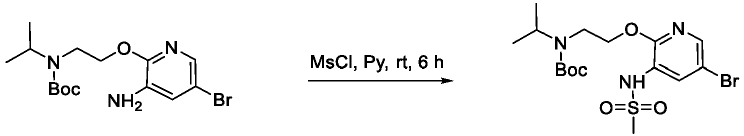

*tert*-Butyl (2-((5-bromo-3-(methylsulfonamido)pyridin-2-yl)oxy)ethyl)(isopropyl)carbamate: To a solution of *tert*-butyl *N*-[2-[(3-amino-5-bromopyridin-2-yl)oxy]ethyl]-*N*-(propan-2-yl)carbamate (18.8 g, 50.3 mmol) in pyridine (400 mL) was added dropwse methanesulfonyl chloride (8.63 g, 75.4 mmol) at ambient temperature. After stirirng for 6 hours at ambient temperature, the resulting mixture was concentrated under reduced pressure. The residue was purified by silica gel column chromatography, eluted with 3%~25% ethyl acetate in petroleum ether. The desired fractions were collected and concentrated under reduced pressure to afford the title compound as an off-colorless solid (16.3 g, 72%): ¹H NMR (400 MHz, DMSO-*d*₆) δ 9.41 (s, 1H), 8.05 (d, *J* = 2.2 Hz, 1H), 7.79 (d, *J* = 2.2 Hz, 1H), 4.35 (t, *J* = 6.3 Hz, 2H), 4.15 (s, 1H), 3.43 (t, *J* = 6.3 Hz, 2H), 3.11 (s, 3H), 1.39 (s, 9H), 1.09 (d, *J* = 6.8 Hz, 6H); MS: [(M + 1)]⁺ = 452.00, 454.00.

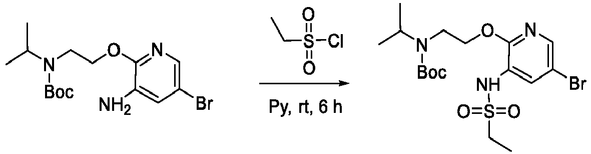

*tert*-Butyl (2-((5-bromo-3-(ethylsulfonamido)pyridin-2-yl)oxy)ethyl)(isopropyl)carbamate: To a stirred solution of *tert*-butyl (2-((3-amino-5-bromopyridin-2-yl)oxy)ethyl)(isopropyl)carbamate (5.00 g, 13.4 mmol) in pyridine (120 mL) was added ethanesulfonyl chloride (5.15 g, 40.1 mmol) dropwise at ambient temperature under nitrogen atmosphere. After stirring for 6 hours at ambient temperature under nitrogen atmosphere, the resulting mixture was concentrated under reduced pressure. The residue was purified by silica gel column chromatography, eluted with 3%~25% ethyl acetate in petroleum ether. The desired fractions were collected and concentrated under reduced pressure to afford the title compound as a light yellow solid (3.78 g, 61%): ¹H NMR (400 MHz, CD₃OD) δ 7.92 (s, 1H), 7.87 (s, 1H), 4.48 (t, *J* = 6.6 Hz, 2H), 4.25-4.20 (m, 1H), 3.54 (t, *J* = 6.6 Hz, 2H), 3.13 (t, *J* = 7.3 Hz, 2H), 1.49 (s, 9H), 1.35 (t, *J* = 7.4 Hz, 3H), 1.19 (d, *J* = 6.8 Hz, 6H); MS: [(M + 1)]⁺ = 466.10, 468.10.

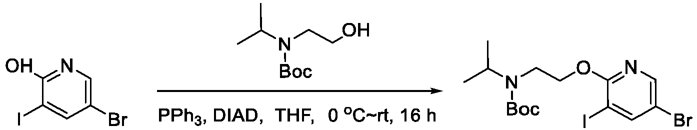

*tert*-Butyl *N*-[2-[(5-bromo-3-iodopyridin-2-yl)oxy]ethyl]-*N*-isopropylcarbamate: To a solution of 5-bromo-3-iodopyridin-2-ol (10.0 g, 33.3 mmol) in anhydrous tetrahydrofuran (300 mL) were added triphenylphosphine (11.4 g, 43.3 mmol), *tert*-butyl *N*-(2-hydroxyethyl)-*N*-isopropylcarbamate (8.80 g, 43.3 mmol) and diisopropyl azodiformate (8.80 g, 43.4 mmol) dropwise at 0 °C. The resulting mixture was stirred for additional 16 hours at ambient temperature under nitrogen atmosphere. The resulting mixture was concentrated under reduced pressure. The residue was purified by silica gel column chromatography, eluted with 2%~10% ethyl acetate in petroleum ether. The desired fractions were collected and concentrated under reduced pressure to afford the title compound as a colorless oil (14.0 g, 87%): ¹H NMR (400 MHz, CDCl₃) δ 8.14 (s, 2H), 4.43 (t, *J* = 6.4 Hz, 2H), 4.38-3.96 (m, 1H), 3.50 (t, *J* = 6.4 Hz, 2H), 1.49 (s, 9H), 1.19 (d, *J* = 6.8 Hz, 6H); MS: [(M + 1)]⁺ = 484.95, 486.95.

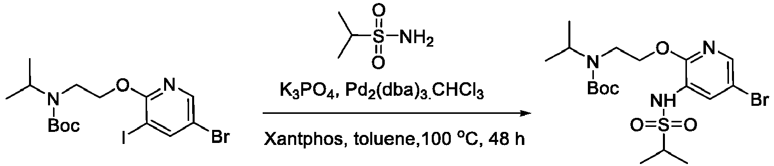

*tert*-Butyl (2-((5-bromo-3-((1-methylethyl)sulfonamido)pyridin-2-yl)oxy)ethyl)(isopropyl)carbamate: To a mixture of *tert*-butyl (2-((5-bromo-3-iodopyridin-2-yl)oxy)ethyl)(isopropyl)carbamate (5.00 g, 10.3 mmol), 4,5-bis(diphenylphosphino)-9,9-dimethylxanthene (1.80 g, 3.10 mmol) and propane-2-sulfonamide (1.50 g, 12.4 mmol) in toluene (125 mL) were added tripotassium phosphate (10.9 g, 51.5 mmol) and tris(dibenzylideneacetone)dipalladium-chloroform adduct (1.10 g, 1.10 mmol) at ambient temperature. The resulting mixture was stirred for 48 hours at 100 °C under argon atmosphere. After cooling down to ambient temperature, the resulting mixture was filtered. The filtered cake was washed with ethyl acetate (3 × 20 mL). The filtrate was concentrated under reduced pressure. The residue was purified by silica gel column chromatography, eluted with 1%~20% ethyl acetate in petroleum ether. The desired fractions were collected and concentrated under reduced pressure to afford the title compound as a colorless solid (1.90 g, 39%): ¹H NMR (400 MHz, CDCl₃) δ 7.94 (d, *J* = 2.1 Hz, 1H), 7.89 (d, *J* = 2.2 Hz, 1H), 4.44 (t, *J* = 6.3 Hz, 2H), 4.11 (s, 1H), 3.48 (t, *J* = 6.3 Hz, 2H), 3.24-3.30 (m, 1H), 1.48 (s, 9H), 1.41 (d, *J* = 6.8 Hz, 6H), 1.14 (d, *J* = 6.8 Hz, 6H); MS: [(M + 1)]⁺ = 480.20, 482.20.

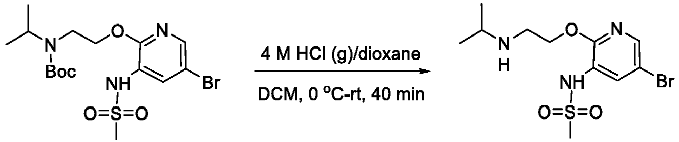

*N*-(5-Bromo-2-[2-[(propan-2-yl)amino]ethoxy]pyridin-3-yl)methanesulfonamide: To a solution of *tert*-butyl *N*-[2-[(5-bromo-3-methanesulfonamidopyridin-2-yl)oxy]ethyl]-*N*-(propan-2-yl)carbamate (3.00 g, 6.63 mmol) in dichloromethane (5.00 mL) was treated with hydrogen chloride (20.0 mL, 4 *M* in 1,4-dioxane) for 40 min at ambient temperature. The resulting mixture was concentrated under reduced pressure. The residue was basified to pH = 8 with saturated aqueous sodium bicarbonate (30.0 mL). The resulting mixture was extracted with ethyl acetate (6 × 200 mL). The combined organic layers was dried over anhydrous sodium sulfate and filtered. The filtrate was concentrated under reduced pressure. The residue was purified by silica gel column chromatography, eluted with 1%~10% methanol in dichloromethane. The desired fractions were collected and concentrated under reduced pressure to afford the title compound as a colorless solid (1.20 g, 50%): ¹H NMR (400 MHz, DMSO-*d*₆) δ 7.68 (d, *J* = 2.3 Hz, 1H), 7.61 (d, *J* = 2.3 Hz, 1H), 5.75 (s, 1H), 4.36 (t, *J* = 5.2 Hz, 2H), 3.19-3.12 (m, 1H), 3.07 (t, *J* = 5.1 Hz, 2H), 2.84 (s, 3H), 1.15 (d, *J* = 6.4 Hz, 6H); MS: [(M + 1)]⁺ = 352.10, 354.10.

The following intermediates were prepared according to the procedure described above:

| Intermediates | Structure | Name | MS: [(M + 1)]⁺ | ¹H NMR |
|---|---|---|---|---|
| CC108 | 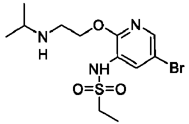 | *N*-(5-Bromos-2-(2-(isopropylamino)ethoxy)p yridin-3-yl)ethanesulfonamide | 366.10 | ¹H NMR (400 MHz, CDCl₃) δ 7.92 (s, 2H), 4.50 (t, *J* = 5.3 Hz, 2H), 3.15 (q, *J* = 7.4 Hz, 2H), 3.07 (t, *J* = 5.3 Hz, 2H), 3.01-2.93 (m, 1H), 1.40 (t, *J* = 7.4 Hz, 3H), 1.16 (d, *J* = 6.3 Hz, 6H). |
| | | | 368.10 | |
| CC110 | 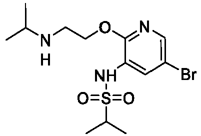 | *N*-(5-Bromo-2-(2-(isopropylamino)ethoxy)p yridin-3-yl)propane-2-sulfonamide | 380.15 | ¹H NMR (400 MHz, CDCl₃) δ 7.95 (d, *J* = 2.2 Hz, 1H), 7.91 (d, *J* = 2.3 Hz, 1H), 4.48 (t, *J* = 5.4 Hz, 2H), 3.31-3.23 (m, 1H), 3.02 (t, *J* = 5.4 Hz, 2H), 2.95-2.87 (m, 1H), 1.40 (d, *J* = 6.8 Hz, 6H), 1.11 (d, *J* = 6.2 Hz, 6H). |
| | | | 382.15 | |

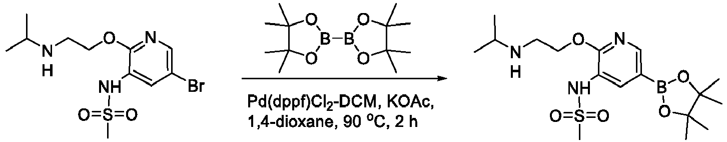

*N*-(2-(2-(Isopropylamino)ethoxy)-5-(4,4,5,5-tetramethyl-1,3,2-dioxaborolan-2-yl)pyridin-3-yl)methanesulfonamide: To a solution of *tert*-butyl *N*-[2-[(5-bromo-3-methanesulfonamidopyridin-2-yl)oxy]ethyl]-*N*-(propan-2-yl)carbamate (2.00 g, 5.68 mmol) and bis(pinacolato)diboron (2.88 g, 11.4 mmol) in 1,4-dioxane (50.0 mL) were added potassium acetate (2.23 g, 22.7 mmol) and bis(diphenylphosphino)ferrocene]dichloro palladium (II) dichloromethane adduct (0.46 g, 0.57 mmol) at ambient temperature. After stirring for 2 hours at 90 °C under nitrogen atmosphere, the resulting mixture was concentrated under reduced pressure. The resulting mixture was diluted with dichloromethane (100 mL). After filtration, the filtered cake was washed with dichloromethane (3 × 10.0 mL). The filtrate was concentrated under reduced pressure. The residue was purified by reversed phase flash chromatography with the following conditions: Column: Spherical C18, 20~40 µm, 330 g; Mobile Phase A: Water (plus 10 mM NH₄HCO₃), Mobile Phase B: acetonitrile; Flow rate: 65 mL/min; Gradient (B%): 5%-22%, 6 min; 22%~40%, 20 min; 40%-95%; 2 min; 95%, 5 min; Detector: UV 254 nm; Rt: 15 min. The fractions containing desired product were collected and concentrated under reduced pressure to afford the title compound as an off-white solid (1.57 g, 87%): ¹H NMR (400 MHz, CDCl₃) δ 8.27 (d, *J* = 1.7 Hz, 1H), 8.06 (d, *J* = 1.7 Hz, 1H), 4.51 (t, *J* = 5.2 Hz, 2H), 3.05-2.99 (m, 5H), 2.95-2.84 (m, 1H), 1.33 (s, 12H), 1.11 (d, *J* = 6.3 Hz, 6H); MS: [(M + 1)]⁺ = 400.30.

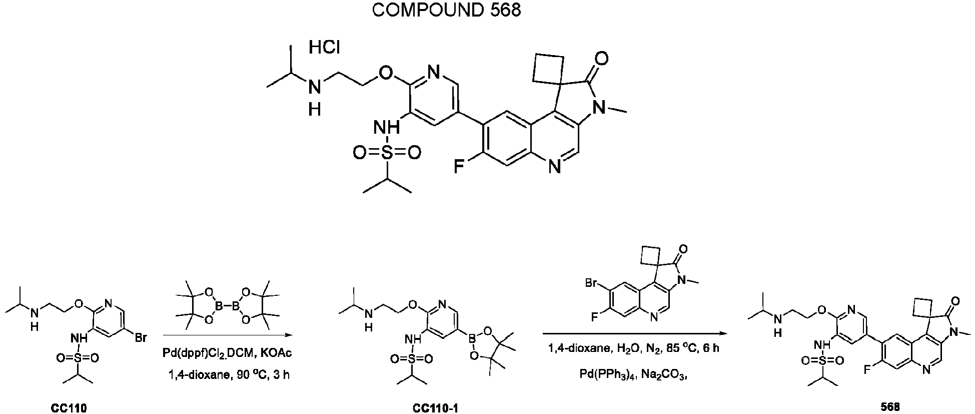

*N*-(5-(7'-Fluoro-3'-methyl-2'-oxo-2',3'-dihydrospiro[cyclobutane-1,1'-pyrrolo[2,3-*c*]quinolin]-8'-yl)-2-(2-(isopropylamino)ethoxy)pyridin-3-yl)propane-2-sulfonamide: To a solution of *N*-[5-bromo-2-[2-(isopropylamino)ethoxy]pyridin-3-yl]propane-2-sulfonamide (2.00 g, 5.26 mmol) in 1,4-dioxane (50.0 mL) were added bis(pinacolato)diboron (4.01 g, 15.8 mmol), potassium acetate (2.06 g, 21.1 mmol) and bis(diphenylphosphino)ferrocene]dichloro palladium (II) dichloromethane adduct (0.34 g, 0.42 mmol) at ambient temperature. The resulting mixture was stirred for 3 hours at 90 °C under nitrogen atmosphere.

The resulting mixture was cooled down to ambient temperature followed by the additions of 8-bromo-7-fluoro-3-methylspiro[cyclobutane-1,1-pyrrolo[2,3-*c*]quinolin]-2-one (1.26 g, 3.76 mmol), water (12.5 mL), sodium carbonate (0.80 g, 7.55 mmol) and *tetrakis*(triphenylphosphine)palladium (0) (0.43 g, 0.38 mmol). The resulting mixture was stirred for 6 hours at 85 °C under nitrogen atmosphere. After cooling down to ambient temperature, the resulting mixture was concentrated under reduced pressure. The residue was purified by reversed phase flash chromatography with the following conditions: Column: Spherical C18, 20~40 µm, 120 g; Mobile phase A: water (plus 10 mM NH₄HCO₃); Mobile phase B: acetonitrile; Flow rate: 45 mL/min; Gradient (B%): 5%, 2 min; 5%-25%, 8 min; 25%-39%, 9 min; 39%, 10 min; 39%~95%; 3 min; 95%, 2 min; Detector: UV 254 nm; Rt: 21 min. The fractions containing desired product were collected and concentrated under reduced pressure to afford the title compound as a colorless solid (1.66 g, 80%): ¹H NMR (400 MHz, DMSO-*d*₆) δ 8.88 (s, 1H), 8.35-8.31 (m, 2H), 8.07 (s, 1H), 7.99 (s, 1H), 7.96 (s, 1H), 4.55 (br, 1H), 4.44 (t, *J* = 5.2 Hz, 2H), 3.35-3.30 (m, 4H), 2.93-2.79 (m, 5H), 2.55-2.38 (m, 4H), 1.29 (d, *J* = 7.2 Hz, 6H), 1.02 (d, *J* = 6.0 Hz, 6H); MS: [(M + 1)]⁺ = 556.30.

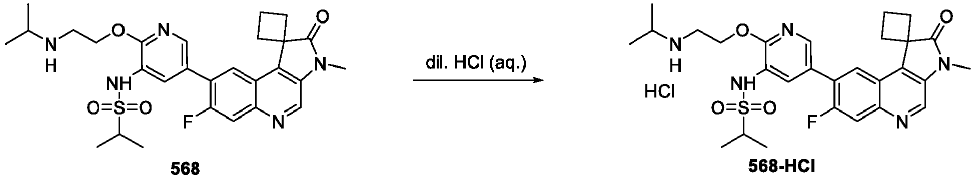

*N*-(5-(7'-Fluoro-3'-methyl-2'-oxo-2',3'-dihydrospiro[cyclobutane-1,1'-pyrrolo[2,3-*c*]quinolin]-8'-yl)-2-(2-(isopropylamino)ethoxy)pyridin-3-yl)propane-2-sulfonamide hydrogen chloride. A solution of *N*-(5-[7-Fluoro-3-methyl-2-oxospiro[cyclobutane-1,1-pyrrolo[2,3-*c*]quinolin]-8-yl]-2-[2-(isopropylamino)ethoxy]pyridin-3-yl)propane-2-sulfonamide (1.66 g, 2.99 mmol) in diluted aqueous hydrochloric acid solution (392 mL, 3.14 mmol, 0.008 M) and acetonitrile (79.0 mL) was lyophilized to afford the title compound as a yellow solid (1.76 g, 100%): ¹H NMR (400 MHz, DMSO-*d*₆) δ 9.45 (s, 1H), 9.02 (br, 2H), 8.90 (s, 1H), 8.40 (s, 1H), 8.32 (d, *J* = 8.4 Hz, 1H), 8.16 (s, 1H), 8.00 (d, *J* = 8.4 Hz, 1H), 4.65 (t, *J* = 4.4 Hz, 2H), 3.54-3.40 (m, 3H), 3.31 (s, 3H), 2.91 (t, *J* = 11.2 Hz, 2H), 2.55-2.45 (m, 5H), 1.34-1.30 (m, 12H); MS: [(M + 1)]⁺ = 556.30.

In a similar manner or palladium (II) couples were affected with Intermediates CC108 and CC110 to afford the following compounds example 569 and example 570.

| Comp. # | Structure | Name | MS: [(M+1)]⁺ | ¹H NMR |
|---|---|---|---|---|
| 569 | 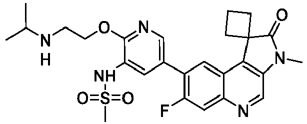 | *N*-(5-(7'-Fluoro-3'-methyl-2'-oxo-2',3'-dihydrospiro[cyclob utane-1,1'-pyrrolo[2,3-*c*]quinolin]-8'-yl)-2-(2-(isopropylamino)eth oxy)pyridin-3-yl)methanesulfonam ide | 528.30 | ¹H NMR (400 MHz, DMSO-*d₆*) δ 8.88 (s, 1H), 8.31 (d, *J* = 8.7 Hz, 2H), 8.01-7.94 (m, 2H), 4.44 (t, *J* = 5.4 Hz, 2H), 3.31 (s, 3H), 3.05 (s, 3H), 2.97 (t, *J* = 5.7 Hz, 2H), 2.95-2.85 (m, 3H), 2.60-2.51 (m, 2H), 2.49-2.41 (m, 2H), 1.07 (d, *J* = 6.3 Hz, 6H). |
| 569 (HCl salt) | 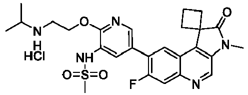 | *N*-(5-(7'-Fluoro-3'-methyl-2'-oxo-2',3'-dihydrospiro[cyclob utane-1,1'-pyrrolo[2,3-*c*]quinolin]-8'-yl)-2-(2-(isopropylamino)eth oxy)pyridin-3-yl)methanesulfonam ide hydrochloride | 528.30 | ¹H NMR (400 MHz, CDsOD) δ 8.77 (s, 1H), 8.42 (d, *J* = 8.1 Hz, 1H), 8.18 (t, *J* = 2.2 Hz, 1H), 8.13 (t, *J* = 1.8 Hz, 1H), 7.86 (d, *J* = 12.1 Hz, 1H), 4.63 (t, *J* = 5.2 Hz, 3.40-3.32 (m, 6H), 3.04 (s, 3H), 3.00-2.90 (m, 2H), 2.74-2.51 (m, 4H), 1.31 (d, *J* = 5.2 Hz, 6H). |
| 570 | 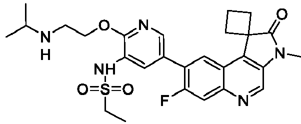 | *N*-(5-(7'-Fluoro-3'-methyl-2'-oxo-2',3'-dihydrospiro[cyclob utane-1,1'-pyrrolo[2,3-*c*]quinolin]-8'-yl)-2-(2-(isopropylamino)eth oxy)pyridin-3-yl)ethanesulfonamid e | 542.30 | ¹H NMR (400 MHz, DMSO-*d₆*) δ 8.88 (s, 1H), 8.35-8.28 (m, 2H), 8.02 (t, *J* = 2.0 Hz, 1H), 7.97 (d, *J* = 12.2 Hz, 1H), 4.43 (t, *J* = 5.4 Hz, 2H), 3.31 (s, 3H), 3.14 (q, *J* = 7.2 Hz, 2H), 2.96 (t, *J* = 5.5 Hz, 2H), 2.93-2.82 (m, 3H), 2.60-2.53 (m, 2H), 2.49-2.40 (m, 2H), 1.28 (t, *J* = 7.3 Hz, 3H), 1.05 (d, *J* = 6.2 Hz, 6H). |
| 570 (HCl salt) | 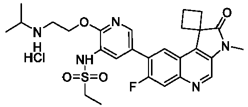 | *N*-(5-(7'-Fluoro-3'-methyl-2'-oxo-2',3'-dihydrospiro[cyclob utane-1,1'-pyrrolo[2,3-*c*]quinolin]-8'-yl)-2-(2-(isopropylamino)eth oxy)pyridin-3-yl)ethanesulfonamid e hydrochloride | 542.30 | ¹H NMR (400 MHz, CDsOD) δ 8.82 (s, 1H), 8.43 (d, *J* = 8.0 Hz, 1H), 8.35 (d, *J* = 2.1 Hz, 1H), 8.22 (t, *J* = 1.7 Hz, 1H), 7.88 (d, *J* = 11.8 Hz, 1H), 4.78 (t, *J* = 5.1 Hz, 2H), 3.60-3.50 (m, 3H), 3.39 (s, 3H), 3.27 (t, *J* = 7.3 Hz, 2H), 3.02-2.89 (m, 2H), 2.76-2.49 (m, 4H), 1.45-1.37 (m, 9H). |

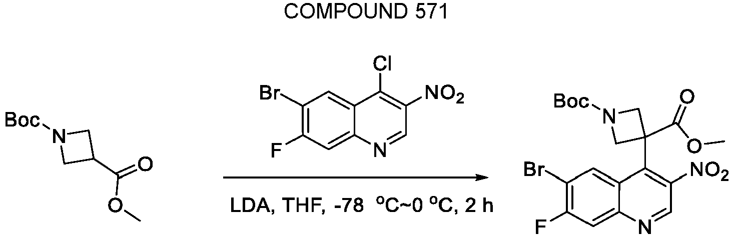

1-(*tert*-Butyl) 3-methyl 3-(6-bromo-7-fluoro-3-nitroquinolin-4-yl)azetidine-1,3-dicarboxylate: To a solution of freshly prepared lithium diisopropylamide (137 mmol) in anhydrous tetrahydrofuran (110 mL) was added a solution of 1-*tert*-butyl 3-methyl azetidine-1,3-dicarboxylate (29.3 g, 137 mmol) in tetrahydrofuran (100 mL) at -78 °C. After stirring for 1 hour, a solution of 6-bromo-4-chloro-7-fluoro-3-nitroquinoline (32.0 g, 105 mmol) in tetrahydrofuran (100 mL) was added to the reaction mixture over 20 min. The resulting mixture was slowly warmed to 0 °C for 2 hours. The reaction was quenched by saturated aqueous ammonium chloride (20.0 mL) and diluted with water (800 mL). The resulting mixture was extracted with ethyl acetate (3 × 150 mL). The combined organic layers was washed with brine (2 × 100 mL) and dried over anhydrous sodium sulfate. After filtration, the filtrate was concentrated under reduced pressure. The residue was purified by silica gel column chromatography, eluted with 5%~20% ethyl acetate in petroleum ether to afford the title compound as a light yellow solid (25.0 g, 49%): ¹H NMR (400 MHz, DMSO-*d*₆) δ 9.35 (s, 1H), 8.21 (d, *J* = 9.3 Hz, 1H), 8.14 (d, *J* = 7.1 Hz, 1H), 4.18-4.11 (m, 2H), 3.87-3.74 (m, 2H), 3.66 (s, 3H), 1.37 (s, 9H); MS: [(M + 1)]⁺ = 484.20, 486.20.

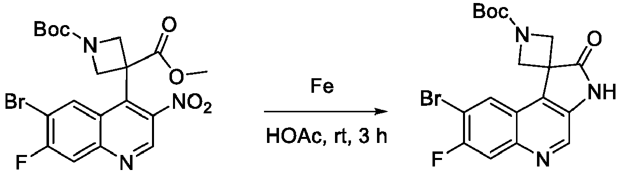

### tert-Butyl 8'-bromo-7'-fluoro-2'-oxo-2',3'-dihydrospiro[azetidine-3,1'-pyrrolo[2,3-c]quinoline]-1-carboxylate:

To a solution of 1-*tert*-butyl 3-methyl 3-(6-bromo-7-fluoro-3-nitroquinolin-4-yl)azetidine-1,3-dicarboxylate (12.0 g, 24.8 mmol) in acetic acid (300 mL) was added iron powder (9.69 g, 174 mmol) at ambient temperature. After stirring for 3 hours at ambient temperature, the resulting mixture was concentrated under reduced pressure. The residue was taken up with water (200 mL) and extracted with ethyl acetate (4 × 100 mL). The combined organic layers was washed with brine (2 × 100 mL) and dried over anhydrous sodium sulfate. After filtration, the filtrate was concentrated under reduced pressure. The residue was purified by silica gel column chromatography, eluted with 1%~5% methanol in dichloromethane to afford the title compound as an off-colorless solid (10.4 g, 99%): ¹H NMR (400 MHz, DMSO-*d*₆) δ 11.02 (br, 1H), 8.71 (s, 1H), 8.24 (d, *J* = 7.3 Hz, 1H), 8.03 (d, *J* = 10.1 Hz, 1H), 4.29 (d, *J* = 9.0 Hz, 2H), 4.21 (d, *J* = 9.0 Hz, 2H), 1.49 (s, 9H); MS: [(M + 1)]⁺ = 422.20, 424.20.

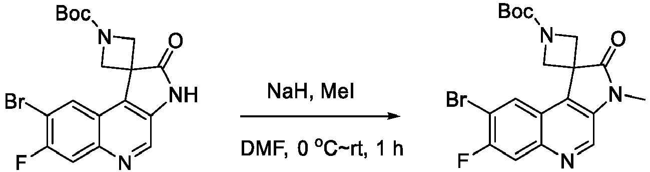

*tert*-Butyl 8'-bromo-7'-fluoro-3'-methyl-2'-oxo-2',3'-dihydrospiro[azetidine-3,1'-pyrrolo[2,3-*c*]quinoline]-1-carboxylate: To a stirred solution of *tert*-butyl 8-bromo-7-fluoro-2-oxo-2,3-dihydrospiro[azetidine-3,1-pyrrolo[2,3-*c*]quinoline]-1-carboxylate (4.22 g, 9.99 mmol) in *N*,*N*-dimethylformamide (100 mL) was added sodium hydride (0.52 g, 13.0 mmol, 60% dispersed in mineral oil) at 0 °C under nitrogen atmosphere. The resulting mixture was stirred for 1 hour at ambient temperature followed by the addition of iodomethane (1.70 g, 12.0 mmol). After stirring for additional 1 hour at ambient temperature, the reaction was quenched by saturated aqueous ammonium chloride (20.0 mL) and diluted with water (1.00 L). The precipitated solids was collected by filtration, washed with water (3 × 30.0 mL) and hexane (2 × 30.0 mL). The resulting solid was dried under infrared light to afford the title compound as a light yellow solid (3.93 g, 90%): ¹H NMR (400 MHz, CDCl₃) δ 8.71 (s, 1H), 8.47 (d, *J* = 7.0 Hz, 1H), 7.94 (d, *J* = 9.1 Hz, 1H), 4.54 (d, *J* = 9.1 Hz, 2H), 4.31 (d, *J* = 9.0 Hz, 2H), 3.40 (s, 3H), 1.56 (s, 9H); MS: [(M + 1)]⁺ = 436.15, 438.15.

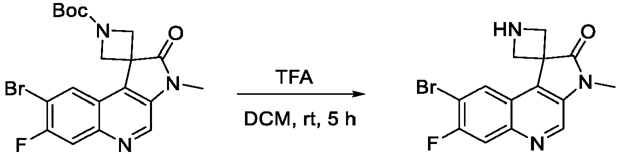

8-Bromo-7-fluoro-3-methyl-2,3-dihydrospiro[azetidine-3,1-pyrrolo[2,3-*c*]quinolin]-2-one: A solution of *tert*-butyl 8-bromo-7-fluoro-3-methyl-2-oxo-2,3-dihydrospiro[azetidine-3,1-pyrrolo[2,3-*c*]quinoline]-1-carboxylate (3.93 g, 9.01 mmol) and trifluoroacetic acid (20.0 mL) in dichloromethane (100 mL) was stirred for 5 hours at ambient temperature. The resulting mixture was concentrated under reduced pressure. The residue was basified to pH = 8 with saturated aqueous sodium bicarbonate solution. The resulting mixture was extracted with ethyl acetate (6 × 300 mL). The combined organic layers was washed with brine (2 × 300 mL) and dried over anhydrous sodium sulfate. After filtration, the filtrate was concentrated under reduced pressure to afford the title compound as a light yellow solid (3.00 g, 99%): ¹H NMR (400 MHz, DMSO-*d*₆) δ 9.56 (d, *J* = 7.7 Hz, 1H), 8.92 (s, 1H), 8.02 (d, *J* = 10.2 Hz, 1H), 4.18 (d, *J* = 7.5 Hz, 2H), 3.59 (d, *J* = 7.5 Hz, 2H), 3.29 (s, 3H); MS: [(M + 1)]⁺ = 335.95, 337.95.

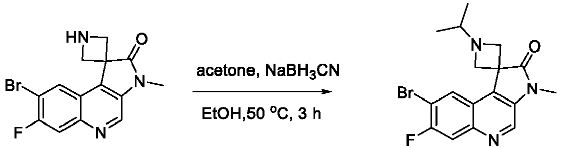

8'-Bromo-7'-fluoro-1-isopropyl-3'-methylspiro[azetidine-3,1'-pyrrolo[2,3-*c*]quinolin]-2'(3'*H*)-one: To a stirred solution of 8'-bromo-7'-fluoro-3'-methylspiro[azetidine-3,1'-pyrrolo[2,3-*c*]quinolin]-2'(3'*H*)-one (100 mg, 0.30 mmol) and acetone (4.00 mL) in ethanol (8.00 mL) was added sodium cyanoborohydride (94.0 mg, 1.50 mmol) at ambient temperature. The resulting mixture was stirred for 3 hours at 50 °C under nitrogen atmosphere. The resulting mixture was purified by reversed phase flash chromatography with the following conditions: Column: Spherical C18, 20~40 µm, 120 g; Mobile Phase A: water (plus 10 mM NH₄HCO₃); Mobile Phase B: acetonitrile; Flow rate: 50 mL/min; Gradient (B): 5%-20%, 6 min; 20%-50%, 30 min; 50%-95%, 5 min; 95%, 5min; Detector: UV 254 nm. The desired fractions were collected and concentrated under reduced pressure to afford the title compound as a colorless solid (85.0 mg, 77%): ¹H NMR: ¹H NMR (400 MHz, DMSO-*d*₆) δ 9.84 (d, *J* = 8.0 Hz, 1H), 8.93 (s, 1H), 8.00 (d, *J* = 10.2 Hz, 1H), 3.69 (d, *J* = 7.6 Hz, 2H), 3.46 (d, *J* = 7.3 Hz, 2H), 3.30 (s, 3H), 2.63-2.56 (m, 1H), 1.01 (d, *J* = 6.1 Hz, 6H); MS: [(M + 1)]⁺ = 378.10, 380.10.

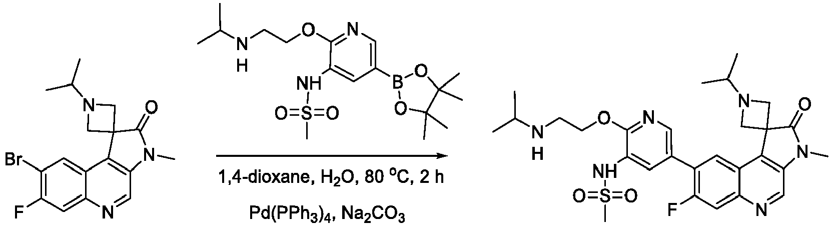

*N*-[5-[7-Fluoro-3-methyl-2-oxo-1-(propan-2-yl)-2,3-dihydrospiro[azetidine-3,1-pyrrolo[2,3-*c*]quinolin]-8-yl]-2-[2-[(propan-2-yl)amino]ethoxy]pyridin-3-yl]methanesulfonamide: To a solution of 8-bromo-7-fluoro-3-methyl-1-(propan-2-yl)-2,3-dihydrospiro[azetidine-3,1-pyrrolo[2,3-*c*]quinolin]-2-one (80.0 mg, 0.21 mmol) and *N*-(2-[2-[(propan-2-yl)amino]ethoxy]-5-(4,4,5,5-tetramethyl-1,3,2-dioxaborolan-2-yl)pyridin-3-yl)methanesulfonamide (169 mg, 0.42 mmol) in water (2.00 mL) and 1,4-dioxane (10.00 mL) were added sodium carbonate (33.6 mg, 0.32 mmol) and *tetrakis* (triphenylphosphine) palladium (0) (24.3 mg, 0.021 mmol). After stirring for 2 hours at 80 °C under nitrogen atmosphere, the resulting mixture was concentrated under reduced pressure. The residue was purified by Prep-TLC (DCM/MeOH = 10/1, v/v) to give a crude product which was further purified by reversed phase flash chromatography with the following conditions: Column: Spherical C18, 20~40 µm, 120 g; Mobile Phase A: Water (plus 10 mM NH₄HCO₃ ), Mobile Phase B: acetonitrile; Flow rate: 45 mL/min; Gradient (B%): 5%, 2 min; 5%-24%, 5 min; 24%-34%, 9 min; 34%, 8 min; 34%~95%; 3 min; 95%, 2 min; Detector: UV 254 nm; Rt: 21 min. The desired fractions were collected and concentrated under reduced pressure to afford the title compound as a colorless solid (65.0 mg, 54%): ¹H NMR (400 MHz, DMSO-*d*₆) δ 9.66 (d, *J* = 8.9 Hz, 1H), 8.89 (s, 1H), 8.25 (t, *J* = 1.9 Hz, 1H), 7.99-7.91 (m, 2H), 4.42 (t, *J* = 5.4 Hz, 2H), 3.76 (d, *J* = 7.2 Hz, 2H), 3.47 (d, *J* = 7.3 Hz, 2H), 3.31 (s, 3H), 3.02 (s, 3H), 2.96 (t, *J* = 5.4 Hz, 2H), 2.92-2.84 (m, 1H), 2.62-2.53 (m, 1H), 1.05 (d, *J* = 6.3 Hz, 6H), 0.97 (d, *J* = 6.1 Hz, 6H); MS: [(M + 1)]⁺ = 571.25.

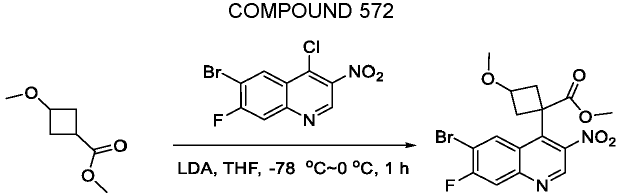

Methyl 1-(6-bromo-7-fluoro-3-nitroquinolin-4-yl)-3-methoxycyclobutane-1-carboxylate: To a solution of freshly prepared lithium diisopropylamide (10.6 mmol) in anhydrous tetrahydrofuran (100 mL) was added methyl 3-methoxycyclobutane-1-carboxylate (1.53 g, 10.6 mmol) at -78 °C. After stirring for additional 1 hour, a solution of 6-bromo-4-chloro-7-fluoro-3-nitroquinoline (2.50 g, 8.18 mmol) in tetrahydrofuran (5.00 mL) was added over 5 min. The resulting mixture was slowly warmed to 0 °C and then quenched by saturated aqueous ammonium chloride (100 mL). The resulting mixture was diluted with water (1.00 L) and the organic layer was separated. The aqueous layer was extracted with ethyl acetate (3 × 200 mL). The combined organic layers was washed with brine (2 × 200 mL) and dried over anhydrous sodium sulfate. After filtration, the filtrate was concentrated under reduced pressure. The residue was purified by silica gel column chromatography, eluted with 5%~50% ethyl acetate in petroleum ether to afford the title compound as a brown syrup (720 mg, 21%): ¹H NMR (400 MHz, DMSO-*d*₆) δ 9.25 (s, 1H), 8.31 (d, *J* = 7.3 Hz, 1H), 8.17 (d, *J* = 9.3 Hz, 1H), 4.16-4.13 (m, 1H), 3.71 (s, 3 H), 3.11 (s, 3H), 2.47-2.38 (m, 2H), 2.00-1.89 (m, 2H); MS: [(M + 1)]⁺ = 413.20, 415.20.

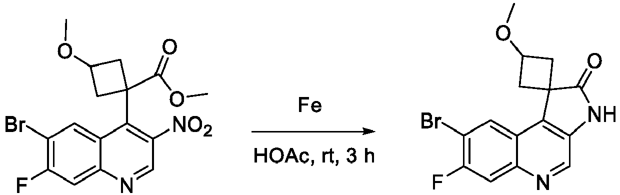

8'-Bromo-7'-fluoro-3-methoxyspiro[cyclobutane-1,1'-pyrrolo[2,3-*c*]quinolin]-2'(3'*H*)-one: To a solution of methyl 1-(6-bromo-7-fluoro-3-nitroquinolin-4-yl)-3-methoxycyclobutane-1-carboxylate (720 mg, 1.74 mmol) in acetic acid (10.0 mL) was added iron powder (681 mg, 12.2 mmol). The resulting mixture was stirred for 3 hours at ambient temperature. The resulting mixture was diluted with water (150 mL) and extracted with ethyl acetate (4 × 50.0 mL). The combined organic layers was washed with brine (2 × 50.0 mL) and dried over anhydrous sodium sulfate. After filtration, the filtrate was concentrated under reduced pressure. The residue was purified by silica gel column chromatography, eluted with 5%~30% ethyl acetate in petroleum ether to afford the title compound as a light yellow solid (550 mg, 89%): ¹H NMR (400 MHz, CDCl₃) δ 8.93 (s, 0.45 H), 8.88 (d, *J* = 7.2 Hz, 0.55H), 8.84 (s, 1H), 8.78 (s, 0.55H), 8.27 (d, *J* = 7.0 Hz, 0.45H) 7.92 (d, *J* = 9.3 Hz, 1H), 4.71-4.63 (m, 0.45H), 4.57-4.49 (m, 0.55H), 3.48 (d, *J* = 6.6 Hz, 3H), 3.07-2.83 (m, 4H); MS: [(M + 1)]⁺ = 351.00, 353.00.

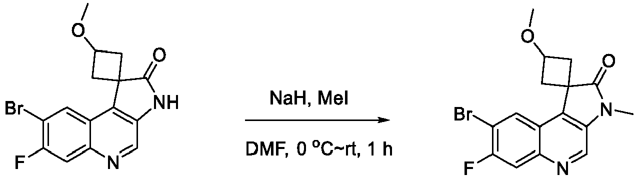

8'-Bromo-7'-fluoro-3-methoxy-3'-methylspiro[cyclobutane-1,1'-pyrrolo[2,3-*c*]quinolin]-2'(3'*H*)-one: A solution of 8-bromo-7-fluoro-3-methoxy-2,3-dihydrospiro[cyclobutane-1,1-pyrrolo[2,3-*c*]quinolin]-2-one (550 mg, 1.56 mmol) in *N,N*-dimethylformamide (10.0 mL) was treated with sodium hydride (81.4 mg, 2.04 mmol, 60% dispersed in mineral oil) for 0.5 hours at 0 °C followed by the addition of iodomethane (265 mg, 1.87 mmol) dropwise over 2 min at 0 °C. After stirring for additional 1 hour at ambient temperature, the reaction was quenched by saturated aqueous ammonium chloride (20.0 mL) and diluted with water (150 mL). The resulting mixture was extracted with ethyl acetate (3 × 50.0 mL). The combined organic layers was washed with brine (2 × 30.0 mL) and dried over anhydrous sodium sulfate. After filtration, the filtrate was concentrated under reduced pressure. The residue was purified by silica gel column chromatography, eluted with 5%-10% ethyl acetate in petroleum ether to afford the title compound as a yellow solid (500 mg, 87%): MS: [(M + 1)]⁺ = 365.10, 367.10.

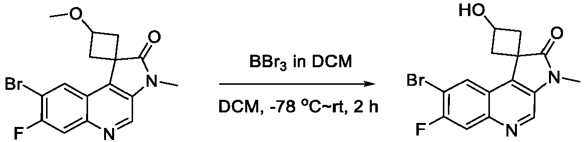

8'-Bromo-7'-fluoro-3-hydroxy-3'-methylspiro[cyclobutane-1,1'-pyrrolo[2,3-*c*]quinolin]-2'(3'*H*)-one: To a stirred solution of 8'-bromo-7'-fluoro-3-methoxy-3'-methylspiro[cyclobutane-1,1'-pyrrolo[2,3-c]quinolin]-2'(3'*H*)-one (900 mg, 2.46 mmol) in dichloromethane (20.0 mL) was added boron tribromide (24.6 mL, 24.6 mmol, 1*M* in dichloromethane) dropwise at -78 °C under nitrogen atmosphere. The resulting mixture was warmed to ambient temperature slowly. The resulting mixture was stirred for 2 hours at ambient temperature under nitrogen atmosphere. The mixture was neutralized with saturated aqueous sodium bicarbonate solution. The aqueous layer was extracted with ethyl acetate (3 × 100 mL). The combined organic layers was washed with brine (3x 100 mL) and dried over anhydrous sodium sulfate. After filtration, the filtrate was concentrated under reduced pressure. The residue was purified by silica gel column chromatography, eluted with 1%~5% methanol in dichloromethane. The desired fractions were collected and concentrated under reduced pressure to afford the title compound as a colorless solid (530 mg, 62%): ¹H NMR (400 MHz, DMSO-*d*₆) δ 8.96-8.89 (m, 1.6H), 8.32 (d, *J* = 7.4 Hz, 0.4H), 8.05-7.98 (m, 1H), 5.99 (d, *J* = 6.5 Hz, 0.6H), 5.73 (d, *J* = 5.7 Hz, 0.4H), 4.97-4.87 (m, 0.4H), 4.75-4.65 (m, 0.6H), 3.31 (s, 1.2 H), 3.29 (s, 1.8H), 2.98-2.87 (m, 0.8H), 2.81-2.67 (m, 2.4H), 2.65-2.55 (m, 0.8H); MS: [(M + 1)]⁺ = 351.00, 353.00.

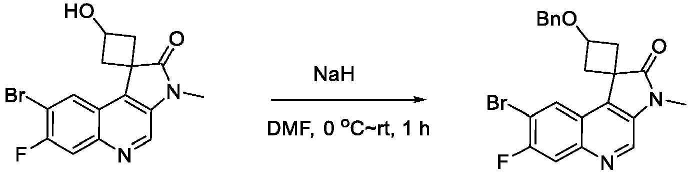

### 3-(Benzyloxy)-8'-bromo-7'-fluoro-3'-methylspiro[cyclobutane-1,1'-pyrrolo[2,3-c]quinolin]-2'(3'H)-one:

To a solution of 8'-bromo-7'-fluoro-3-hydroxy-3'-methylspiro[cyclobutane-1,1'-pyrrolo[2,3-*c*]quinolin]-2'(3'*H*)-one (100 mg, 0.29 mmol) in *N*,*N*-dimethylformamide (5.00 mL) was added sodium hydride (13.7 mg, 0.35 mmol, 60% dispersed in mineral oil) at 0 °C under nitrogen atmosphere. The resulting mixture was stirred for 1 hour at 25 °C followed by the addition of benzyl bromide (97.4 mg, 0.57 mmol) at 0 °C. After stirring for additional 1 hour at 25 °C, the reaction was quenched by saturated aqueous ammonium chloride solution (10.0 mL). The resulting mixture was diluted with water (100 mL) and extracted with ethyl acetate (3 × 50.0 mL). The combined organic layers were washed with brine (2 × 50.0 mL) and dried over anhydrous sodium sulfate. After filtration, the filtrate was concentrated under reduced pressure. The residue was purified by Prep-TLC (DCM/MeOH = 25/1, v/v) to afford the title compound as a yellow solid (64.0 mg, 51%): ¹H NMR (400 MHz, DMSO-*d*₆) δ 8.92 (d, *J* = 3.6 Hz, 1H), 8.79 (d, *J* = 7.6 Hz, 0.5H), 8.25 (d, *J* = 7.2 Hz, 0.5H), 8.03 (d, *J* = 10.1 Hz, 1H), 7.50 (d, *J* = 7.0 Hz, 1H), 7.46-7.37 (m, 3H), 7.37-7.30 (m, 1H), 4.87-4.79 (m, 0.5H), 4.62-4.50 (m, 2.5H), 3.30 (d, *J* = 5.7 Hz, 3H), 2.98-2.90 (m, 1H), 2.80 (d, *J* = 6.7 Hz, 2H), 2.72-2.64 (m, 1H); MS: [(M + 1)]⁺ = 441.00, 443.00.

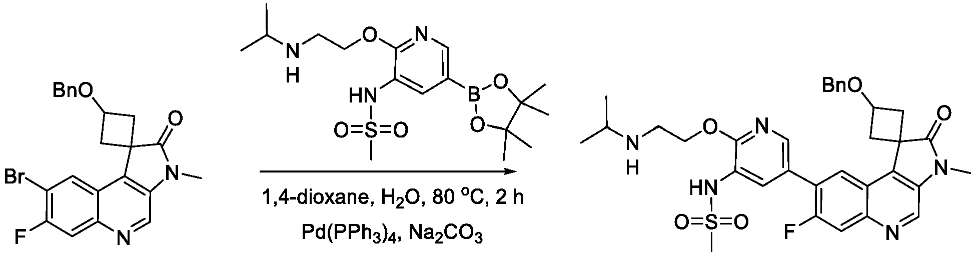

*N*-(5-(3-(Benzyloxy)-7'-fluoro-3'-methyl-2'-oxo-2',3'-dihydrospiro[cyclobutane-1,1'-pyrrolo[2,3-*c*]quinolin]-8'-yl)-2-(2-(isopropylamino)ethoxy)pyridin-3-yl)methanesulfonamide: To a solution of 3-(benzyloxy)-8'-bromo-7'-fluoro-3'-methylspiro[cyclobutane-1,1'-pyrrolo[2,3-*c*]quinolin]-2'(3'*H*)-one (64.0 mg, 0.15 mmol) and *N*-[2-[2-(isopropylamino)ethoxy]-5-(4,4,5,5-tetramethyl-1,3,2-dioxaborolan-2-yl)pyridin-3-yl]methanesulfonamide (69.5 mg, 0.17 mmol) in water (1.00 mL) and 1,4-dioxane (4.00 mL) were added sodium carbonate (15.4 mg, 0.15 mmol) and *tetrakis* (triphenylphosphine) palladium (0) (33.5 mg, 0.029 mmol). After stirring for 2 hours at 80 °C under nitrogen atmosphere, the resulting mixture was concentrated under reduced pressure. The residue was purified by Prep-TLC (DCM/MeOH = 10/1, v/v) to afford the title compound as a light yellow solid (55.0 mg, 60%): MS: [(M + 1)]⁺ = 634.55.

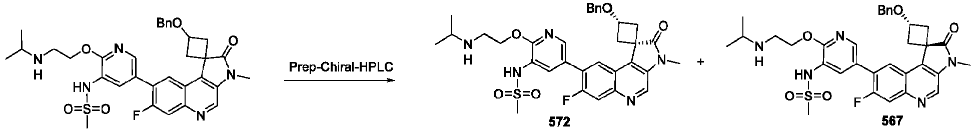

*cis-N*-(5-(3-(Benzyloxy)-7'-fluoro-3'-methyl-2'-oxo-2',3'-dihydrospiro[cyclobutane-1,1'-pyrrolo[2,3-*c*]quinolin]-8'-yl)-2-(2-(isopropylamino)ethoxy)pyridin-3-yl)methanesulfonamide (EXAMPLE 572) and *trans-N*-(5-(3-(Benzyloxy)-7'-fluoro-3'-methyl-2'-oxo-2',3'-dihydrospiro[cyclobutane-1,1'-pyrrolo[2,3-*c*]quinolin]-8'-yl)-2-(2-(isopropylamino)ethoxy)pyridin-3-yl)methanesulfonamide (EXAMPLE 567). The above *N*-(5-(3-(benzyloxy)-7'-fluoro-3'-methyl-2'-oxo-2',3'-dihydrospiro[cyclobutane-1,1'-pyrrolo[2,3-*c*]quinolin]-8'-yl)-2-(2-(isopropylamino)ethoxy)pyridin-3-yl)methanesulfonamide (55.0 mg, 0.087 mmol) was seperated by Prep-Chiral-HPLC with the following conditions: (Column: CHIRALPAK ID, 2 × 25 cm (5 µm); Mobile Phase A: methyl *tert*-butyl ether (plus 0.2% isopropylamine), Mobile Phase B: EtOH; Flow rate: 17 mL/min; Gradient: 10% B in 13 min; Detector: UV 220/254 nm) to afford *cis-N*-(5-(3-(benzyloxy)-7'-fluoro-3'-methyl-2'-oxo-2',3'-dihydrospiro[cyclobutane-1,1'-pyrrolo[2,3-*c*]quinolin]-8'-yl)-2-(2-(isopropylamino)ethoxy)pyridin-3-yl)methanesulfonamide (EXAMPLE 572, RT1: 8.70 min) as a light yellow solid (15.3 mg, 28%): ¹H NMR (400 MHz, DMSO-*d*₆) δ 8.90 (s, 1H), 8.29 (s, 1H), 8.09 (d, *J* = 8.3 Hz, 1H), 8.02 (d, *J* = 1.9 Hz, 1H), 7.98 (d, *J* = 12.1 Hz, 1H), 7.42-7.34 (m, 4H), 7.32-7.27 (m, 1H), 4.90-4.82 (m, 1H), 4.53 (s, 2H), 4.47 (t, *J* = 5.4 Hz, 2H), 3.32 (s, 3H), 3.06 (s, 3H), 3.05-2.88 (m, 5H), 2.68 (dd, *J* = 13.9, 6.2 Hz, 2H), 1.08 (d, *J* = 6.2 Hz, 6H); MS: [(M + 1)]⁺ = 634.50 and *trans*-*N*-(5-(3-(benzyloxy)-7'-fluoro-3'-methyl-2'-oxo-2',3'-dihydrospiro[cyclobutane-1,1'-pyrrolo[2,3-*c*]quinolin]-8'-yl)-2-(2-(isopropylamino)ethoxy)pyridin-3-yl)methanesulfonamide (EXAMPLE 567, RT2:11.2 min) as a colorless solid (12.6 mg, 23%): ¹H NMR (400 MHz, DMSO-*d*₆) δ 8.88 (s, 1H), 8.59 (d, *J* = 8.3 Hz, 1H), 8.33 (s, 1H), 8.02-7.94 (m, 2H), 7.36-7.21 (m, 5H), 4.58 (q, *J* = 6.3 Hz, 1H), 4.44 (t, *J* = 5.4 Hz, 2H), 4.41-4.33 (m, 1H), 3.28 (s, 3H), 3.05 (s, 3H), 2.00-3.94 (m, 3H), 2.92-2.84 (m, 1H), 2.83-2.74 (m, 1H), 2.69 (dd, *J* = 13.1, 5.8 Hz, 2H), 2.59-2.54 (m, 1H), 1.35 (d, *J* = 6.4 Hz, 3H), 1.05 (d, *J* = 6.3 Hz, 6H); MS: [(M + 1)]⁺ = 634.55.

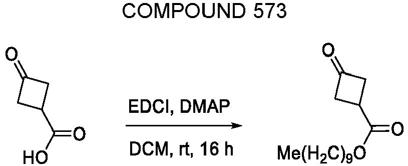

Decyl 3-oxocyclobutane-1-carboxylate: To a stirred solution of 3-methylcyclobutane-1-carboxylic acid (3.00 g, 26.3 mmol) and 1-decanol (4.16 g, 26.3 mmol) in dichloromethane (90.0 mL) were added 1-ethyl-3-(3-dimethylaminopropyl)carbodiimide hydrochloride (7.56 g, 39.4 mmol) and 4-dimethylaminopyridine (0.32 g, 2.62 mmol) at ambient temperature. The resulting mixture was stirred for 16 hours at 25 °C and quenched by water (30.0 mL). The resulting mixture was extracted with dichloromethane (3 × 100 mL). The combined organic layers was washed with brine (2 × 50.0 mL) and dried over anhydrous sodium sulfate. After filtration, the filtrate was concentrated under reduced pressure. The residue was purified by silica gel column chromatography, eluted with 1%~8% ethyl acetate in petroleum ether. The desired fractions were collected and concentrated under reduced pressure to afford the title compound as a light yellow oil (3.10 g, 47%): ¹H NMR (400 MHz, CDCl₃) δ 4.15 (t, *J* = 6.7 Hz, 2H), 3.47-3.35 (m, 2H), 3.35-3.15 (m, 3H), 1.69-1.61 (m, 2H), 1.39-1.22 (m, 14H), 0.88 (t, *J* = 6.7 Hz, 3H); MS: [(M + 1)]⁺ = 252.20.

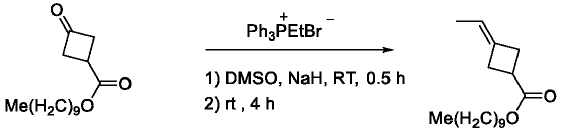

Decyl 3-ethylidenecyclobutane-1-carboxylate: To a solution of ethyltriphenylphosphonium bromide (17.3 g, 46.5 mmol) in dimethyl sulfoxide (300 mL) was added potassium *t*-butoxide (4.94 g, 44.1 mmol) in portions at 14 °C. The resulting mixture was stirred for 0.5 hours at 25 °C under nitrogen atmosphere followed by the addition of decyl 3-oxocyclobutane-1-carboxylate (8.00 g, 31.5 mmol) dropwise over 2 min at 14 °C. After stirring for 4 hours at 25 °C, the reaction was quenched by saturated aqueous ammonium chloride solution (20.0 mL) at 0°C. The resulting mixture was diluted with water (1.00 L) and extracted with ethyl acetate (3 × 200 mL). The combined organic layers was washed with brine (2 × 100 mL) and dried over anhydrous sodium sulfate. After filtration, the filtrate was concentrated under reduced pressure. The residue was purified by silica gel column chromatography, eluted with 1%~5% ethyl acetate in petroleum ether. The desired fractions were collected and concentrated under reduced pressure to afford the title compound as a colorless oil (1.70 g, 21%): ¹H NMR (400 MHz, CDCl₃) δ 5.22-5.14 (m, 1H), 4.09 (t, *J* = 6.7 Hz, 2H), 3.10 (tt, *J* = 9.2, 7.2 Hz, 1H), 2.96-2.79 (m, 4H), 1.67-1.58 (m, 2H), 1.49 (dq, *J* = 6.0, 2.0 Hz, 3H), 1.39-1.20 (m, 14H), 0.88 (t, *J* = 6.7 Hz, 3H).

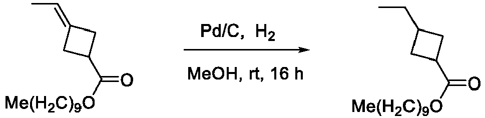

Decyl 3-ethylcyclobutane-1-carboxylate: To a stirred solution of decyl 3-ethylidenecyclobutane-1-carboxylate (700 mg, 4.94 mmol) in methanol (10.0 mL) was added anhydrous Pd/C (70.0 mg, 10% palladium on charcoal) at ambient temperature. After stirring for 16 hours at ambient temperature under hydrogen atmosphere (2 atm.), the resulting mixture was filtered. The filter cake was washed with methanol (3 × 20.0 mL). The filtrate was concentrated under reduced pressure to afford the title compound as a light yellow oil (666 mg, 95%): ¹H NMR (400 MHz, CDCl₃) δ 4.10-4.02 (m, 2H), 3.10-2.87 (m, 1H), 2.40-2.22 (m, 2H), 2.16-2.03 (m, 1H), 1.91-1.76 (m, 2H), 1.67-1.56 (m, 2H), 1.48-1.21 (m, 16H), 0.92-0.75 (m, 6H).

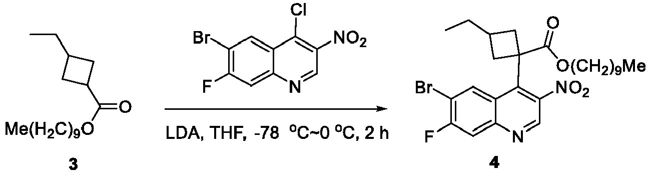

Decyl 1-(6-bromo-7-fluoro-3-nitroquinolin-4-yl)-3-ethylcyclobutane-1-carboxylate: To a solution of freshly prepared lithium diisopropylamide (2.45 mmol) in anhydrous tetrahydrofuran (20.0 mL) was added decyl 3-ethylcyclobutane-1-carboxylate (657 mg, 2.45 mmol) at -78 °C under nitrogen atmosphere. The resulting mixture was stirred for 1 hour followed by the addition of 6-bromo-4-chloro-7-fluoro-3-nitroquinoline (575 mg, 1.88 mmol) at -78 °C. After stirring for additional 1 hour at 25 °C, the reaction was quenched by saturated aqueous ammonium chloride solution (10.0 mL). The resulting mixture was diluted with water (20.0 mL) and separated. The aqueous layer was extracted with ethyl acetate (5 × 30 mL). The combined organic layers was washed with brine (4 × 30.0 mL) and dried over anhydrous sodium sulfate. After filtration, the filtrate was concentrated under reduced pressure. The residue was purified by silica gel column chromatography, eluted with 3%~9% ethyl acetate in petroleum ether. The desired fractions were collected and concentrated under reduced pressure to afford the title compound as a yellow oil (450 mg, crude): MS: [(M + 1)]⁺ = 537.25, 539.25.

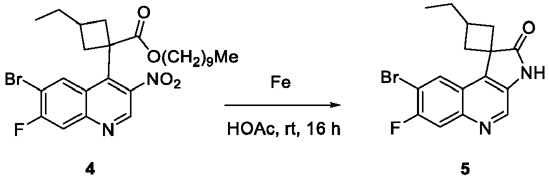

8'-Bromo-3-ethyl-7'-fluorospiro[cyclobutane-1,1'-pyrrolo[2,3-*c*]quinolin]-2'(3'*H*)-one: A mixture of crude decyl 1-(6-bromo-7-fluoro-3-nitroquinolin-4-yl)-3-ethylcyclobutane-1-carboxylate (450 mg, 0.84 mmol) and iron power (327 mg, 5.86 mmol) in acetic acid (8.00 mL) was stirred for 16 h at ambient temperature . The resulting mixture was filtered, the filtered cake was washed with tetrahydrofuran (5 × 300 mL). The filtrate was concentrated under reduced pressure. The residue was basified to pH = 8 with saturated aqueous sodium bicarbonate solution. The resulting mixture was extracted with ethyl acetate (5 × 100 mL). The combined organic layers was washed with brine (50.0 mL) and dried over anhydrous sodium sulfate. After filtration, the filtrate was concentrated under reduced pressure. The residue was purified by Prep-TLC (DCM/MeOH = 15/1, v/v) to afford the title compound as a yellow solid (40.0 mg, 14%): ¹H NMR (400 MHz, DMSO-*d*₆) δ 10.76 (d, *J* = 5.3 Hz, 1H), 8.67 (d, *J* = 1.7 Hz, 1H), 8.45 (d, *J* = 7.2 Hz, 0.4H), 8.39 (d, *J* = 7.4 Hz, 0.6H), 7.98 (dd, *J* = 10.1, 3.4 Hz, 1H), 2.88-2.73 (m, 2H), 2.55-2.51 (m, 2H), 2.29-2.19 (m, 1H), 1.78-1.64 (m, 2H), 0.99-0.88 (m, 3H); MS: [(M + 1)]⁺ = 349.05, 351.05.

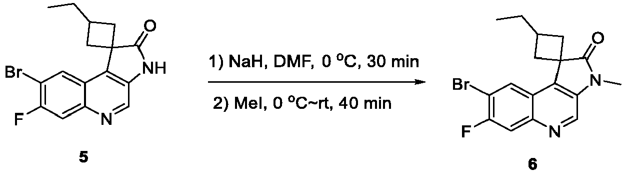

8'-Bromo-3-ethyl-7'-fluoro-3'-methylspiro[cyclobutane-1,1'-pyrrolo[2,3-*c*]quinolin]-2'(3'*H*)-one : To a solution of 8'-bromo-3-ethyl-7'-fluorospiro[cyclobutane-1,1'-pyrrolo[2,3-*c*]quinolin]-2'(3'*H*)-one (35.0 mg, 0.10 mmol) in *N*,*N*-dimethylformamide (3.00 mL) was added sodium hydride (5.21 mg, 0.13 mmol, 60% dispersed in mineral oil) at 0 °C under nitrogen atmosphere. The resulting mixture was stirred for 30 minutes at 25 °C followed by the addition of iodomethane (21.4 mg, 0.15 mmol) at 0 °C. After stirring for additional 40 minutes at 25 °C, the reaction was quenched by saturated aqueous ammonium chloride solution (20.0 mL). The resulting mixture was diluted by water (100 mL) and extracted with ethyl acetate (4 × 100 mL). The combined organic layers was washed with brine (4 × 100 mL) and dried over anhydrous sodium sulfate. After filtration, the filtrate was concentrated under reduced pressure. The residue was purified by Prep-TLC (DCM/MeOH = 20/1, v/v) to afford the title compound as a yellow solid (30.0 mg, 83%): ¹H NMR (400 MHz, DMSO-*d*₆) δ 8.90 (d, *J* = 2.9 Hz, 1H), 8.49 (d, *J* = 7.4 Hz, 0.4H), 8.42 (d, *J* = 7.4 Hz, 0.6H), 8.02 (dd, *J* = 10.1, 3.1 Hz, 1H), 3.29 (d, *J* = 4.5 Hz, 3H), 2.93-2.75 (m, 2H), 2.61-2.52 (m, 2H), 2.29-2.21 (m, 1H), 1.82-1.67 (m, 2H), 0.98-0.89 (m, 3H); MS: [(M + 1)]⁺ = 363.05, 365.05.

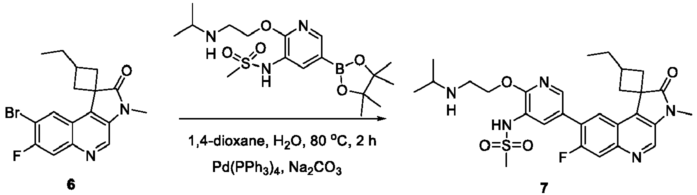

*N*-(5-(3-Ethyl-7'-fluoro-3'-methyl-2'-oxo-2',3'-dihydrospiro[cyclobutane-1,1'-pyrrolo[2,3-*c*]quinolin]-8'-yl)-2-(2-(isopropylamino)ethoxy)pyridin-3-yl)methanesulfonamide: To a solution of 8'-bromo-3-ethyl-7'-fluoro-3'-methylspiro[cyclobutane-1,1'-pyrrolo[2,3-*c*]quinolin]-2'(3'*H*)-one (50.0 mg, 0.14 mmol) and *N*-[2-[2-(isopropylamino)ethoxy]-5-(4,4,5,5-tetramethyl-1,3,2-dioxaborolan-2-yl)pyridin-3-yl]methanesulfonamide (165 mg, 0.41 mmol) in water (0.75 mL) and 1,4-dioxane (3.00 mL) were added sodium carbonate (17.5 mg, 0.17 mmol) and *tetrakis* (triphenylphosphine) palladium (0) (31.8 mg, 0.028 mmol). After stirring for 2 hours at 80 °C under nitrogen atmosphere, the resulting mixture was concentrated under reduced pressure. The residue was purified by Prep-TLC (DCM/MeOH = 10/1, v/v) to give a crude product which was further purified by reversed phase flash chromatography with the following conditions: Column: Spherical C18, 20~40 µm, 120 g; Mobile Phase A: Water (plus 10 mM NH₄HCO₃), Mobile Phase B: acetonitrile; Flow rate: 45 mL/min; Gradient (B%): 5%-25%, 7 min; 25%-45%, 25 min; 45%-65%, 8 min; 95%, 5 min; Detector: UV 254 nm. The fractions containing desired product was collected at 17 min and concentrated under reduced pressure to afford the title compound as a yellow solid (30.0 mg, 40%): MS: [(M + 1)]⁺ = 556.20.

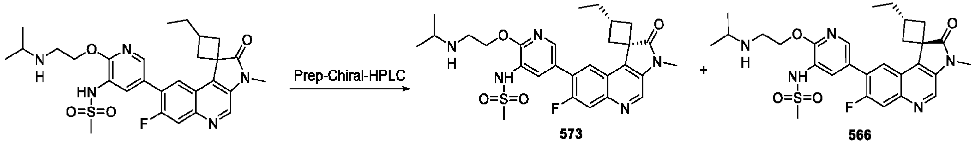

*cis-N*-(5-(3-Ethyl-7'-fluoro-3'-methyl-2'-oxo-2',3'-dihydrospiro[cyclobutane-1,1'-pyrrolo[2,3-*c*]quinolin]-8'-yl)-2-(2-(isopropylamino)ethoxy)pyridin-3-yl)methanesulfonamide (EXAMPLE 573) and *trans-N*-(5-(3-Ethyl-7'-fluoro-3'-methyl-2'-oxo-2',3'-dihydrospiro[cyclobutane-1,1'-pyrrolo[2,3-*c*]quinolin]-8'-yl)-2-(2-(isopropylamino)ethoxy)pyridin-3-yl)methanesulfonamide (EXAMPLE 566): The above *N*-(5-(3-ethyl-7'-fluoro-3'-methyl-2'-oxo-2',3'-dihydrospiro[cyclobutane-1,1'-pyrrolo[2,3-*c*]quinolin]-8'-yl)-2-(2-(isopropylamino)ethoxy)pyridin-3-yl)methanesulfonamide (30.0 mg) was separated by Prep-Chiral-HPLC with the following conditions: Column: Chiralpak IC, 2 × 25 cm, 5 µm; Mobile Phase A:Hexane/DCM=3/1 (plus 0.2% isopropylamine), Mobile Phase B:EtOH; Flow rate: 20 mL/min; Gradient: 30% B in 15 min; Detector: UV 220/254 nm. The desired fractions were collected and concentrated under reduced pressure to afford *cis-N*-(5-(3-ethyl-7'-fluoro-3'-methyl-2'-oxo-2',3'-dihydrospiro[cyclobutane-1,1'-pyrrolo[2,3-c]quinolin]-8'-yl)-2-(2-(isopropylamino)ethoxy)pyridin-3-yl)methanesulfonamide (EXAMPLE 573, RT1: 10.94 min) as a colorless solid (12.8 mg, 43%): ¹H NMR (400 MHz, DMSO-*d*₆) δ 8.87 (s, 1H), 8.32-8.22 (m, 2H), 7.98 (d, *J* = 12.2 Hz, 2H), 4.44 (t, *J* = 5.3 Hz, 2H), 3.30 (s, 3H), 3.04 (s, 3H), 2.99 (t, *J* = 5.3 Hz, 2H), 2.94-2.78 (m, 4H), 2.26 (dd, *J* = 12.2, 5.7 Hz, 2H), 1.75 (t, *J* = 7.2 Hz, 2H), 1.07 (d, *J* = 6.3 Hz, 6H), 0.89 (t, *J* = 7.3 Hz, 3H); MS: [(M + 1)]⁺ = 556.25
and *trans-N*-(5-(3-ethyl-7'-fluoro-3'-methyl-2'-oxo-2',3'-dihydrospiro[cyclobutane-1,1'-pyrrolo[2,3-*c*]quinolin]-8'-yl)-2-(2-(isopropylamino)ethoxy)pyridin-3-yl)methanesulfonamide (EXAMPLE 566, RT2:13.63 min) as a colorless solid (8.6 mg, 29%): ¹H NMR (400 MHz, DMSO-*d*₆) δ 8.88 (s, 1H), 8.34-8.26 (m, 2H), 8.01-7.93 (m, 2H), 4.44 (t, *J* = 5.4 Hz, 2H), 3.31 (s, 3H), 3.05 (s, 3H), 3.00 (d, *J* = 5.4 Hz, 2H), 2.96-2.83 (m, 2H), 2.70- 2.60 (m, 2H), 1.66 (q, *J* = 7.2, 6.7 Hz, 2H), 2.56-2.52 (m, 2H), 1.07 (d, *J* = 6.2 Hz, 6H), 0.89 (t, *J* = 7.3 Hz, 3H); MS: [(M + 1)]⁺ = 556.20.

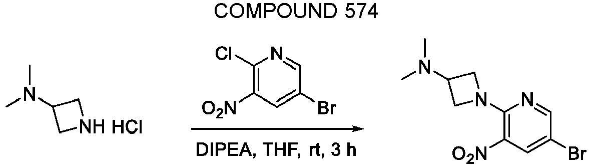

1-(5-Bromo-3-nitropyridin-2-yl)-*N*,*N*-dimethylazetidin-3-amine: To a stirred solution of *N,N-*dimethylazetidin-3-amine hydrochloride (0.27 g, 2.08 mmol) and 5-bromo-2-chloro-3-nitropyridine (0.49 g, 2.08 mmol) in tetrahydrofuran (40.0 mL) was added diisopropylethylamine (0.67 g, 5.19 mmol) at ambient temperature. The resulting mixture was stirred for 3 hours and concentrated under reduced pressure. The residue was purified by silica gel column chromatography, eluted with 3%~9% ethyl acetate in petroleum ether. The desired fractions were collected and concentrated under reduced pressure to afford the title compound as a yellow solid (0.50 g, 59%): ¹H NMR (400 MHz, CDCl₃) δ 8.37 (d, *J* = 2.2 Hz, 1H), 8.29 (d, *J* = 2.2 Hz, 1H), 4.20 (ddd, *J* = 10.0, 7.0, 1.2 Hz, 2H), 3.93 (ddd, *J* = 9.9, 5.1, 1.2 Hz, 2H), 3.16 (tt, *J* = 7.0, 5.1 Hz, 1H), 2.20 (s, 6H); MS: [(M + 1)]⁺ = 301.00, 303.00.

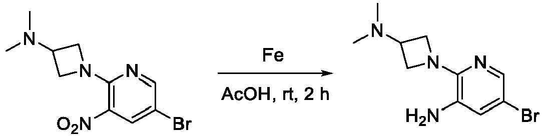

1-(5-Bromo-3-nitropyridin-2-yl)-*N*,*N*-dimethylazetidin-3-amine. To a solution of 1-(5-bromo-3-nitropyridin-2-yl)-*N*,*N*-dimethylpiperidin-4-amine (6.20 g, 20.7 mmol) in acetic acid (90.0 mL) was added iron powder (11.5 g, 206 mmol) at ambient temperature. The resulting mixture was stirred for 2 hours at ambient temperature. The resulting mixture was filtered and the filtered cake was washed with tetrahydrofurane (3 × 100 mL). The filtrate was concentrated under reduced pressure. The residue was taken up with saturated aqueous sodium carbonate (100 mL) and extracted with ethyl acetate (3 × 100 mL). The combined organic layers was washed with brine (3 × 100 mL) and dried over anhydrous sodium sulfate. After filtration, the filtrate was concentrated under reduced pressure. The residue was purified by silica gel column chromatography, eluted with 2%~4% methanol in dichloromethane to afford the title compound as a grey solid (5.20 g, 92%): ¹H NMR (400 MHz, CDCl₃) δ 7.74 (d, *J* = 2.0 Hz, 1H), 6.93 (d, *J* = 2.0 Hz, 1H), 4.19-4.04 (m, 2H), 3.93-3.88 (m, 2H), 3.21-3.14 (m, 1H), 2.24 (s, 6H); MS: [(M + 1)]⁺ = 271.00, 273.00.

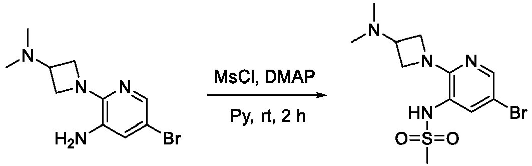

*N*-(5-Bromo-2-(3-(dimethylamino)azetidin-1-yl)pyridin-3-yl)methanesulfonamide: To a stirred solution of 5-bromo-2-[3-(dimethylamino)azetidin-1-yl]pyridin-3-amine (2.10 g, 7.77 mmol) and *N,N*-4-dimethylaminopyridine (77.0 mg, 0.63 mmol) in pyridine (70.0 mL) was added methanesulfonyl chloride (1.77 g, 15.5 mmol) dropwise at ambient temperature. After stirring for 3 hours at ambient temperature under nitrogen atmosphere, the resulting mixture was concentrated under reduced pressure. The residue was purified by reversed phase flash chromatography with the following conditions: Column: Spherical C18, 20~40 µm, 330 g; Mobile Phase A: Water (plus 10 mM NH₄HCO₃), Mobile Phase B: acetonitrile; Flow rate: 65 mL/min; Gradient (B%): 5%-20%, 8 min; 20%-40%, 20 min; 40%-95%, 2 min; 95%, 5 min; Detector: UV 254 nm. The desired fractions were collected at 19 min and concentrated under reduced pressure to afford the title compound as a colorless solid (1.40 g, 52%): ¹H NMR (400 MHz, CDsOD) δ 7.92 (d, *J* = 2.2 Hz, 1H), 7.61 (d, *J* = 2.2 Hz, 1H), 4.27 (dd, *J* = 8.8, 7.2 Hz, 2H), 3.96 (dd, *J* = 9.0, 5.6 Hz, 2H), 3.24-3.16 (m, 1H), 3.00 (s, 3H), 2.20 (s, 6H); MS: [(M + 1)]⁺ = 349.00, 351.00.

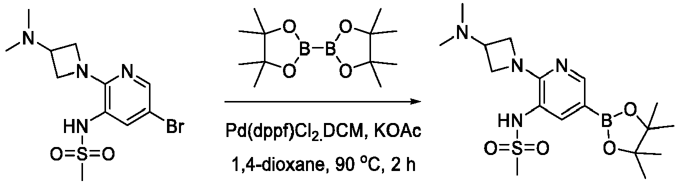

*N*-(2-(3-(Dimethylamino)azetidin-1-yl)-5-(4,4,5,5-tetramethyl-1,3,2-dioxaborolan-2-yl)pyridin-3-yl)methanesulfonamide: To a solution of *N*-[5-bromo-2-[3-(dimethylamino)azetidin-1-yl]pyridin-3-yl]methanesulfonamide (1.00 g, 2.86 mmol) and 4 bis(pinacolato)diboron (2.18 g, 8.59 mmol) in 1,4-dioxane (30.0 mL) were added potassium acetate (1.12 g, 11.5 mmol) and bis(diphenylphosphino)ferrocene]dichloro palladium (II) dichloromethane adduct (351 mg, 0.43 mmol) at ambient temperature. The resulting mixture was stirred for 2 hours at 85 °C under nitrogen atmosphere. The resulting mixture was concentrated under reduced pressure. The residue was purified by reversed flash chromatography with the following conditions: Column: Spherical C18, 20~40 µm, 330 g; Mobile Phase A: Water (plus 10 mM NH₄HCO₃), Mobile Phase B: acetonitrile; Flow rate: 65 mL/min; Gradient (B%): 5%-20%, 7 min; 20%-40%, 12 min; 40%-95%; 2 min; 95%, 5 min; Detector: UV 254 nm. The desired fractions were collected at 20 min and concentrated under reduced pressure to afford the title compound as a yellow solid (800 mg, 71%): ¹H NMR (400 MHz, DMSO-*d*₆) δ 8.78 (s, 1H), 8.18 (d, *J* = 1.5 Hz, 1H), 7.50 (d, *J* = 1.6 Hz, 1H), 4.19 (t, *J* = 8.0 Hz, 2H), 3.93 (dd, *J* = 8.9, 5.0 Hz, 2H), 3.12-3.04 (m, 1H), 2.99 (s, 3H), 2.09 (s, 6H), 1.27 (s, 12H); MS: [(M + 1)]⁺ = 397.20.

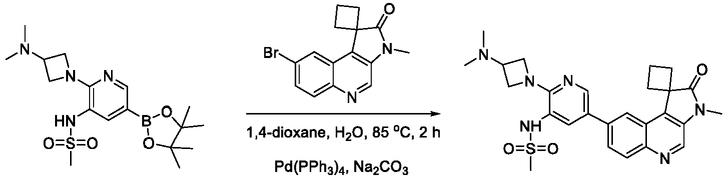

*N*-(2-(3-(Dimethylamino)azetidin-1-yl)-5-(3'-methyl-2'-oxo-2',3'-dihydrospiro[cyclobutane-1,1'-pyrrolo[2,3-*c*]quinolin]-8'-yl)pyridin-3-yl)methanesulfonamide: To a solution of 8-bromo-3-methyl-2,3-dihydrospiro[cyclobutane-1,1-pyrrolo[2,3-*c*]quinolin]-2-one (320 mg, 1.01 mmol) and *N*-[2-[3-(dimethylamino)azetidin-1-yl]-5-(4,4,5,5-tetramethyl-1,3,2-dioxaborolan-2-yl)pyridin-3-yl]methanesulfonamide (600 mg, 1.51 mmol) in 1,4-dioxane (13.0 mL) and water (2.00 mL) were added sodium carbonate (160 mg, 1.51 mmol) and *tetrakis* (triphenylphosphine) palladium (0) (175 mg, 0.15 mmol). After stirring for 2 hours at 85 °C under a nitrogen atmosphere, the resulting mixture was concentrated under reduced pressure. The residue was purified by Prep-TLC (DCM/MeOH = 8/1, v/v) to afford the title compound as a light yellow solid (350 mg, 69%): ¹H NMR (400 MHz, DMSO-*d*₆) δ 9.07 (s, 1H), 8.80 (s, 1H), 8.58 (s, 1H), 8.33 (s, 1H), 8.13 (d, *J* = 8.9 Hz, 1H), 7.95 (d, *J* = 9.2 Hz, 1H), 7.91 (d, *J* = 2.1 Hz, 1H), 4.28-4.21 (m, 2H), 4.02-3.95 (m, 2H), 3.30 (s, 3H), 3.14 (s, 4H), 2.98-2.90 (m, 2H), 2.60-2.52 (m, 4H), 2.13 (s, 6H); MS: [(M + 1)]⁺ = 507.20.

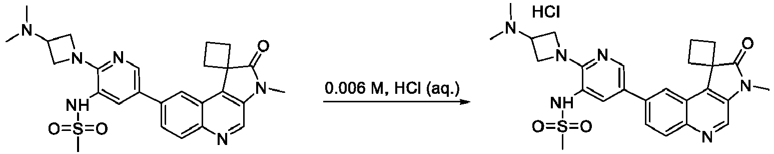

*N*-(2-(3-(Dimethylamino)azetidin-1-yl)-5-(3'-methyl-2'-oxo-2',3'-dihydrospiro[cyclobutane-1,1'-pyrrolo[2,3-c]quinolin]-8'-yl)pyridin-3-yl)methanesulfonamide hydrochloride: A solution of *N*-(2-(3-(dimethylamino)azetidin-1-yl)-5-(3'-methyl-2'-oxo-2',3'-dihydrospiro[cyclobutane-1,1'-pyrrolo[2,3-*c*]quinolin]-8'-yl)pyridin-3-yl)methanesulfonamide (350 mg, 0.69 mmol) in diluted aqueous hydrochloric acid solution (115 mL, 0.69 mmol, 0.006 M) and acetonitrile (20.0 mL) was lyophilized directly to afford the title compound as an orange solid (375 mg, 100%): ¹H NMR (400 MHz, DMSO-*d*₆) δ 10.77 (s, 1H), 9.24 (s, 1H), 8.83 (s, 1H), 8.64 (d, *J* = 2.1 Hz, 1H), 8.35 (s, 1H), 8.17 (d, *J* = 8.9 Hz, 1H), 8.02-7.94 (m, 2H), 4.50-4.41 (m, 2H), 4.39-4.32 (m, 2H), 4.27-4.16 (m, 1H), 3.31 (s, 3H), 3.18 (s, 3H), 2.99-2.88 (m, 2H), 2.82 (d, *J* = 4.1 Hz, 6H), 2.63-2.52 (m, 4H); MS: [(M + 1)]⁺ = 507.20.

### TARGET AND OFF-TARGET INHIBITORY ACTIVITY

### ATM In Cell Western Assay:

In the morning, MCF-7 breast cancer cells were placed in a 384 well plate at the density of 10,000 cells/well (Corning, #356663), 25 µL cells per well. The following day, compounds were added to the plate using a pin tool (Echo 550) to a final concentration of 1 µM through a 3-fold serial dilution (a total of 10 doses). Then, etoposide (Sigma, #E1383) was added to a final concentration of 100 µM. The plate was incubated at 37 °C for 1 h, and the cells were fixed by the addition of 25 µL of fixing solution (8% paraformaldehyde) for 20 minutes at ambient temperature. Cells were permeabilized by 5 washes with 1X PBS (phosphate buffered saline) containing 0.1% Triton X-100; each wash was 5 minutes long. Cells were blocked by adding 50 µL of Odyssey Blocking Buffer (LI-COR, #927-40000) in 384 well plates for 1.5 hours with shaking at ambient temperature. Blocking buffer was then removed, and 20 µL of anti-pKAP1 antibody (Bethyl Laboratories, #A300-767A) (1/2000) solution were added to each well of 384-well plate. The plate was incubated overnight at 4 °C and then washed 5 times with 1X PBST (1X PBS containing 0.1% Tween-20). A secondary antibody (IRDye 800CW Goat anti-Rabbit IgG, LI-COR, #926-32211) (1/5,000) solution containing DNA stain DRAQ5 (CST, #4084L) (1/5,000) (20 µL) was added to each well in the plate, and the plate was incubated 1 hour with gentle shaking in the dark. Cells were washed 5 times with 1X PBST (1X PBS containing 0.1% Tween-20) at ambient temperature with gentle shaking in the dark. After the last wash, the wash solution was removed, the plate was inverted upside down onto a thin paper towel and centrifuged at 1000 rpm for 1 min to absorb all wash buffer. The bottom of the plate was cleaned with a moist, lint-free paper. The plate was immediately scanned using ODYSSEY CLx (LI-COR).

### DNA-PK Enzyme-linked immunosorbent Assay:

On day one, a 96-well plate (ThermoFisher, Cat#: 442404) was coated with GST-p53 (1-101) peptide (purified by Pharmaron, BCS department) by diluting 3 µg of GST-p53 in each well with 0.1 M Na₂CO₃/NaHCO₃ (pH 9.6). The plate was incubated overnight at 4 °C. On the second day, the coating buffer was removed, and the plate was washed twice with PBST (1X PBS containing 0.1 % Tween-20). The DNA-PK enzyme solution (Invitrogen, #PR9107A; the final DNA-PK concentration: 0.1 µg/mL) was then added. The compounds were serially diluted to the final maximal concentration of 100 nM (3 fold series dilution, a total of 10 doses), and an ATP solution (the final ATP concentration: 20 µM) was added to the plate. Incubate the plate at 25 °C for 1 hour. The plate was washed three times with PBST (1X PBS containing 0.1% Tween-20) and blocked with a solution of PBST and 1% BSA at 4 °C overnight. The third day, the plate was washed four times with PBST (1X PBS containing 0.1% Tween-20). Anti-phospho-p53 primary antibody (cell signaling Technology, #9286, Phospho-p53 (Ser15) (16G8) Mouse mAb) (1/1000) was added to each well. The plate was sealed, incubated 1 h at 37 °C, and washed four times with PBST (1X PBS containing 0.1% Tween-20). An HRP-linked secondary antibody (Cell signaling Technology, #7076, Anti-mouse IgG, HRP-linked Antibody) (1/1000) (100 µL) was added to each well. The plate was sealed with tape, incubated 30 min at 37 °C, and washed four times with PBST (1X PBS containing 0.1 % Tween-20). At this time, 100 µL of TMB (Cell signaling Technology, #7004) substrate were added to each well. The plate was sealed with tape and incubated the plate 10 min at 37 °C. Stop solution (Cell signaling Technology, #7002) (100 µL) was added to each well, and the the plate was subjected to the absorption detection at 450 nm.

### mTOR Biochemical Assay:

mTOR Kinase reactions were performed in a 10 µL volume in low-volume 384-well plates. Typically, PerkinElmer model 6008260 plates were used. The composition of the 1x kinase reaction buffer was: 50 mM HEPES pH 7.5, 0.01% Tween 20, 1 mM EGTA, 10 mM MnCl₂, and 2 mM DTT. The solution of mTOR enzyme (ThermoFisher, # PR8683B; the final mTOR concentration: 0.5 µg/mL) was added, and the compounds were serially diluted to the final maximal concentration of 100 nM (3 fold series dilution, a total of 10 doses). GFP-4E-BP1 (the final concentration: 0.4 µM) and the ATP solution (the final ATP concentration: 3 µM) were added to the 384-well plate. The plate was incubated at 25 °C for 1 hour, and 10 µL of the EDTA solution (20 mM) and Tb-labeled anti-p4E-BP1 antibody (4 nM) in TR-FRET dilution buffer were added to each well. The plate was sealed, incubated 30 min at 25 °C, and read on a plate reader configured for LanthaScreen^{™} TRFRET.

### PI3Ka and PI3Kδ Biochemical Assay:

PI3K□ and PI3K□ Kinase reactions were performed in a 5 µL volume in low-volume 384-well plates. Typically, PerkinElmer model 6008280 plates were used. The 1x kinase reaction buffer consisted of 50 mM HEPES pH 7.5, 3mM MgCl₂, 0.03% CHAPS, 1 mM EGTA, 100 mM NaCl, and 2 mM DTT. PI3K□ (ThermoFisher, # PV4788; the final PI3K□ concentration: 120 ng/mL) or PI3K□ enzyme solution (ThermoFisher, # PV6451; the final PI3K□ concentration: 250 ng/mL) was added to the plate, compounds were serially diluted to the final maximal concentration of 100 nM (3 fold series dilution, a total of 10 doses), and the PIP2:3PS (the final concentration: 10 □g/mL) and ATP solution (the final ATP concentration: 10 µM) was added to the 384-well plate. The plate was incubated at 25 °C for 1 hour. ADP-Glo reagent buffer (5 µL) was added to each well. The plate was sealed and incubated for 40 min at 25 °C. ADP-Glo detection buffer (10 µL) was added to each well, and the plate was incubated for 40 min at 25 °C and read on a plate reader configured for Luminescence.

### In-vitro hERG Inhibition Assay:

The hERG-T-REx ^{™} HEK 293 cells (Invitrogen, K1236) were generated by transfecting the hERG coding sequence in the Tet-regulated expression vector pT-Rex-DEST30 into cells expressing the Tet-repressor (T-Rex ^{™} HEK293), thereby producing cells that can be induced to express high level of hERG channels. The cells were cultured in a medium containing of 85% DMEM, 10% dialyzed FBS, 0.1 mM NEAA, 25 mM HEPES, 100 U/mL Penicillin-Streptomycin, 5 µg/mL Blasticidin, and 400 µg/mL Geneticin. The cells were split using TrypLE^{™} Express (Gibco, 12604) about three times a week and maintained between ~40% to ~80% confluence. Before the assay, the cells were induced with doxycycline (Sigma, D9891) at 1 µg/mL for 48 hours. On the experiment day, the induced cells were resuspended and plated onto the coverslips at 5 × 10⁵ cells /per 6 cm cell culture dish prior to use. The hERG channel-mediated current was acquired by manual patch clamp recording systems equipped with amplifiers (HEKA, EPC10 and Molecular Devices, multiclamp 700B) and the inverted phase contrast microscope (Olympus, IX51/71/73). Glass pipettes were prepared by micropipette puller (Sutter, P97 and Narishige, PC-10) and qualified by the pipette resistance in the range of 2-4 M□. The internal pipette solution was 140 mM KCI, 2 mM MgCl₂, 10 mM EGTA, 5 mM MgATP, and 10 mM HEPES (pH adjusted to 7.35 with KOH), and the external buffer was 132 mM NaCl, 4 mM KCI, 3 mM CaCl₂, 0.5 mM MgCl₂, 11.1 mM glucose, and 10 mM HEPES (pH adjusted to 7.35 with NaOH). The whole-cell configuration was maintained with access resistance continuously monitored (< 15 M□). The hERG current was elicited by depolarizing membrane to +30 mV for 4.8 sec, and the voltage was set back to -50 mV for 5.2 sec to remove the inactivation and measure the deactivating tail current. The maximum amount of tail current size was used to determine hERG current amplitude. To evaluate the hERG inhibition, the blank vehicle and test articles were perfused to cells under whole-cell recording configuration through the liquid perfusion system (ALA, VM8 gravity-flow delivery system). For dose response assay, test article was applied to the cells accumulatively from low to high concentrations. A positive control (Dofetilide) was used in the experiments to ensure the performance of the cells and operations as major part of method validation. The percentage hERG current inhibition was fitted against dose concentrations to build the dose-response curve and determine IC₅₀.

In some embodiments, the compound of the invention is selected from the group consisting of compounds listed in the table below.

**TABLE Assay results**

| Compound | nomenclature | ATM IC₅₀ (nM) (cell) | DNA-PK IC₅₀ (nM) | mTOR IC₅₀ (nM) | PI3K-α/δ IC₅₀ (nM) | hERG IC₅₀ (µM) |
|---|---|---|---|---|---|---|
| **566** | *trans-N*-(5-(3-Ethyl-1'-fluoro-3'-methyl-2'-oxo-2',3'-dihydrospiro[cyclobutane-1,1'-pyrrolo[2,3-*c*]quinolin]-8'-yl)-2-(2-(isopropylamino)ethoxy)pyridin-3-yl)methanesulfonamide | 6.72 | 1.03 | >100 | >100/14 | 2.10 |
| **567** | *trans-N*-(5-(3-(Benzyloxy)-1'-fluoro-3'-methyl-2'-oxo-2',3'-dihydrospiro[cyclobutane-1,1'-pyrrolo[2,3-*c*]quinolin]-8'-yl)-2-(2-(isopropylamino)ethoxy)pyridin-3-yl)methanesulfonamide | 11 | 2.70 | >100 | >100/>100 | 5.50 |
| **568** | *N*-(5-(7'-Fluoro-3'-methyl-2'-oxo-2',3'-dihydrospiro[cyclobutane-1,1'-pyrrolo[2,3-*c*]quinolin]-8'-yl)-2-(2-(isopropylamino)ethoxy)pyridin-3-yl)propane-2-sulfonamide | 2.88 | 0.79 | >100 | 18/54 | 14 |
| **569** | *N*-(5-(7'-Fluoro-3'-methyl-2'-oxo-2',3'-dihydrospiro[cyclobutane-1,1'-pyrrolo[2,3-*c*]quinolin]-8'-yl)-2-(2-(isopropylamino)ethoxy)pyridin-3-yl)methanesulfonamide hydrochloride | 2.43 | 0.63 | >100 | 16/73 | >30 |
| **570** | *N*-(5-(7'-Fluoro-3'-methyl-2'-oxo-2',3'-dihydrospiro[cyclobutane-1,1'-pyrrolo[2,3-*c*]quinolin]-8'-yl)-2-(2-(isopropylamino)ethoxy)pyridin-3-yl)ethanesulfonamide hydrochloride | 3.60 | 0.63 | >100 | 21/55 | 10.5 |
| **571** | *N*-(5-(7'-Fluoro-1 -isopropyl-3'-methyl-2'-oxo-2',3'-dihydrospiro[azetidine-3,1'-pyrrolo[2,3-*c*]quinolin]-8'-yl)-2-(2-(isopropylamino)ethoxy)pyridin-3-yl)methanesulfonamide | 5.76/5.51 | 0.67/0.94 | >100 | 62/100 | 5.60 |
| **572** | *cis-N*-(5-(3-(Benzyloxy)-7'-fluoro-3'-methyl-2'-oxo-2',3'-dihydrospiro[cyclobutane-1,1'-pyrrolo[2,3-*c*]quinolin]-8'-yl)-2-(2-(isopropylamino)ethoxy)pyridin-3-yl)methanesulfonamide | 1.05 | 0.32 | >100 | 23/15 | 7.5 |
| **573** | *cis-N*-(5-(3-Ethyl-7'-fluoro-3'-methyl-2'-oxo-2',3'-dihydrospiro[cyclobutane-1,1'-pyrrolo[2,3-*c*]quinolin]-8'-yl)-2-(2-(isopropylamino)ethoxy)pyridin-3-yl)methanesulfonamide | 0.92 | 0.30 | >100 | 18/12 | 3 |
| **574** | *N*-(2-(3-(Dimethylamino)azetidin-1-yl)-5-(3'-methyl-2'-oxo-2',3'-dihydrospiro[cyclobutane-1,1'-pyrrolo[2,3-*c*]quinolin]-8'-yl)pyridin-3-yl)methanesulfonamide hydrochloride | 1.52 | 1.03 | 19.9 | 0.12/3 | > 30 |

### CLONOGENIC AND IN VIVO EFFICACY ASSAYS

### Methods

**Cell Culture.** The MCF-7 human breast carcinoma cell line and HCT116 p53 wild-type and HCT116 p53 -/- cell lines were obtained from ATCC. MCF7 and HCT116 cells were cultured in Dulbecco's modified Eagle's medium (DMEM (Gibco #11995-065). MDA-MB-231 human triple negative breast cancer cell line and FADU, human head and neck squamous cell carcinoma line, were obtained from Charles River Laboratories (Morrisville, NC). MDA-MB-231 cells were cultured in RPMI 1640 medium (Gibco, 11875-093). FADU cells were cultured in Minimum Essential Medium α (MEM α) (Gibco, 12571-063) supplemented with 1mM Sodium Pyruvate (Gibco, 11360-070), 1X MEM Non-Essential Amino Acids (Gibco, 11140-050). All cell lines were supplemented with 10% fetal bovine serum (FBS) (Corning, 35-010-CV) and 1X Antibiotic-Antimycotic (Gibco # 15240-062) and were grown at 37°C in 5% CO2. All cell lines were authenticated by short tandem repeat profiling and tested negative for mycoplasma.

**Antibodies.** Antibodies recognizing phosphorylated/activated ATM (S1981, #ab81292) and DNA-PKcs (S2056, #ab18192) were purchased from Abcam Biotechnology (Cambridge, MA). Antibodies to ATM (#A1106) and DNA-PKcs (#ab1832) were from Sigma and Abcam, respectively. Phospho-KAP1 (S824, #A300-767A) and KAP1 (A700-014) antibodies were from Bethyl Labs (Montgomery, TX). Phospho-TBK1 (#5483), cGAS (#15102), and pSTING (S366, #19781S) antibodies were from Cell Signaling Technology (CST) (Danvers, MA). STING (PA5-23381) antibodies were from ThermoFisher Scientific (Waltham, MA), and actin (#A5316) antibodies were from Sigma. All antibodies were diluted 1:1000 with the exception of actin which was diluted 1:3000 in 1X TBST (1X TBS -10X TBS, (Corning, 46-012-CM), 0.05% Tween-20 (P7949) Sigma and 1% BSA. Secondary antibodies used were (a) Li-Cor method- Goat anti-Rabbit (926-3221) and Goat anti-Mouse (926-68020) Li-Cor (b) ECL method: Anti-rabbit IgG, HRP-linked (#7074S), CST.

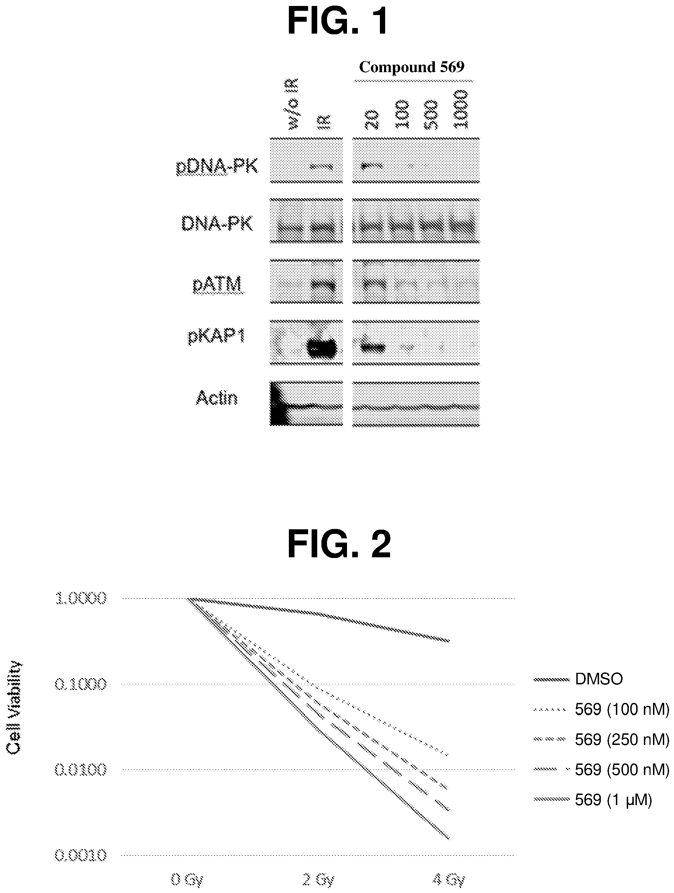

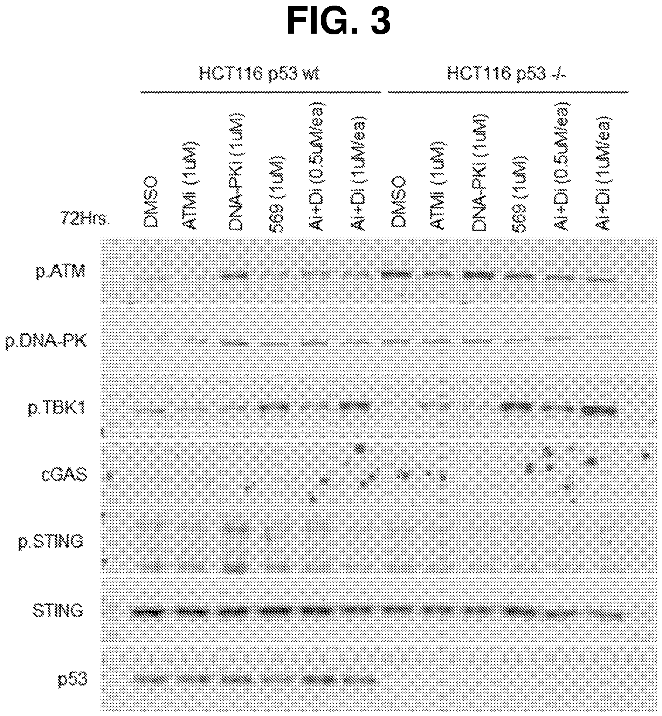

**Immunoblotting.** Cells were pretreated with Compound 569 at 20, 100, 500, or 1000 nM or DMSO for 30 min and then irradiated with 0 or 10 Gy (XRAD 160, Precision X Ray) as shown in FIG. 1. In FIG. 3, cells were pretreated with Compound 569 at 1 µM, ATMi (AZD0156) at 1 µM, DNA-PKi (peposertib) at 1 µM as single agents or DMSO or with ATMi and DNA-PKi in combination at 0.5 micromolar each or at 1 micromolar each for 30 min and then irradiated with 0 or 10 Gy (XRAD 160, Precision X Ray) as shown in FIG. 1. One hour after IR, cells were lysed in RIPA buffer (50nM Tris, pH 8.0, 150mM NaCl, 0.1% SDS, 0.5% sodium deoxycholate, 1% IGEPAL^{®} CA-630 (Sigma, I8896) for 20 min at 4°C in the presence of protease and phosphatase inhibitor cocktails (Roche, #04693159001 & #04906837001). Cell lysates were centrifuged at 13,200g for 10 min at 4°C and supernatants were analyzed for protein content using BCA assay (Pierce^{™} BCA Protein Assay Kit, cat# 23227, ThermoFisher Waltham, MA 02451) and equal amounts of protein/lane were used for subsequent immunoblot analyses. Proteins were denatured by adding NuPAGE LSD Sample Buffer [4X] (Invitrogen, NP0007) containing β-mercaptoethanol at a final concentration of 2.5% and the samples were boiled for 5 minutes. Samples were loaded into NuPAGE 3-8% Tris-Acetate Protein Gels (Invitrogen, EA03785BOX) and electrophoresed with 1X Tris-Acetate SDS Running Buffer (Invitrogen, LA0041), or were loaded into NuPAGE 4-12% Bis-Tris Protein Gels (Invitrogen, NP0336BOX) and electrophoresed with 1X MOPS SDS Running Buffer (Invitrogen, NP0001).

Proteins were transferred overnight to nitrocellulose membranes (Amersham, 10600003). Membranes were blocked with 5% non-fat dry milk in 1X TBST for 1 hour and incubated with primary antibodies overnight at 4°C. Membranes were washed 4 × 10 min with 1X TBST at RT, and then incubated with secondary antibodies for 1 hr at RT, followed by washing 3 × 10 min with 1X TBST at RT. Membranes were visualized with ODYSSEY CLX system, or with ECL (ECL Blotting substrate, Pierce, 32209, 34075, 34095).

**Clonogenic survival assay.** This assay was performed with compound 569. Cells were plated in 6-well plates at different densities: 250 cells for no IR control, 5000 cells for 2 Gy of IR and 10000 cells for 4 Gy of IR and cultured overnight. The next day, cells were preincubated with compound 569 at 100, 250, 500, or 1000 nM or DMSO for 30 min before being exposed to increasing doses of IR (0, 2, or 4 Gy). After IR, cells were continuously incubated with DMSO or compound 569 for 5 h before the culture medium was removed and cells were washed with PBS. Cells were cultured in complete medium in the absence of inhibitor for 9 days. Then cells were stained (PBS, 0.0037% v/v formaldehyde, 0,1 % crystal violet) rinsed with water and dried.

**Tumor Studies.** Female NCr nu/nu mice (CrI:NU(NCr)-Foxn1nu, Strain 490) were obtained from Charles River at 5-6 weeks of age. All animal procedures used in these studies were approved by the Institutional Animal Care and Use Committee (IACUC) at Duke University.

FADU or MDA-MB-231 cells were harvested during log phase growth for in vivo implantation. The resuspended cells were washed three times in phosphate buffered saline (PBS) before preparing the working dilution in PBS. FADU cells were diluted to 1 × 10⁷ cells/mL and MDA-MB-231 cells were diluted to 5 × 10⁷ cells/mL. Each mouse was injected subcutaneously in the right flank with 100µL of the cellular suspension. Tumor growth was monitored until the tumors reached a target volume of at least 100 mm³, at which point mice were randomly stratified into four treatment groups.

Following implantation of the tumor cell lines, mice were monitored once weekly for tumor development. Upon detection, tumors were measured in two dimensions using digital calipers. Tumors were treated after reaching at least 100 mm³ and were measured two to three times weekly following enrollment in one of the treatment groups. The study endpoint was defined as tumor quintupling from the volume at time of treatment.

The vehicle used in this study was 0.5% (Hydroxypropyl)methyl cellulose (HPMC) and 0.2% Tween80 dissolved in deionized water. The pH of the vehicle was adjusted to 7.0-8.0 and the vehicle was stored at 4°C. On each day of in vivo treatment an appropriate mass of test compound was added to the vehicle at room temperature. The mixture was vortexed as necessary to generate a final concentration of 1.6 mg/mL.

**Pharmacodynamic (PD) assays.** Tumor studies were performed as follows. Mice bearing FADU or MDA-231 tumors were pre-dosed with vehicle alone or compound 569 alone at 3, 6, and 10 mg/kg. Tumors were then irradiated with 10 Gy 45 min after vehicle or compound 569 administration or left unirradiated (vehicle and compound 569 at 10 mg/kg). Tumors were harvested 1 h post radiation.

Tissue homogenates for pharmacodynamic analyses were collected and processed as follows. Tumor tissues from FADU or MDA-MB-231 tumors were flash frozen in liquid nitrogen and pulverized on dry ice using a tissue pulverizer. A portion of the tumor powder was transferred into a microtube, followed by addition of 500 µL of RIPA buffer [50 nM Tris, pH 8.0, 150 mM NaCl, 0.1% SDS, 0.5% sodium deoxycholate, 1% IGEPAL^{®} CA-630 (Sigma, I8896)]. To 10 ml of RIPA buffer, 2 tablets each of Protease inhibitor and Phosphatase inhibitor (Roche, #04693159001 & #04906837001), 200 µL of 0.1 M PMSF and 160 µL of Aprotinin, (Sigma, A6279) were added. The suspension was homogenized on ice with a pellet pestle motor (KONTES) and incubated on ice for 30 minutes. Cell lysates were centrifuged at 13,200 rpm for 15 min at 4°C and supernatants were transferred to a new tube and analyzed for protein concentration with a BCA kit (Pierce, 23227), followed by immunoblotting.

In vivo tumor growth delay study was performed as follows. Following implantation of the tumor cell lines, mice were monitored once weekly for tumor development. Upon development of a tumor measuring at least 100 mm³, tumor-bearing mice were stratified to one of 4 groups, utilizing 5 mice per group. Tumor volumes were between 100 and 500 mm³. Vehicle or compound 569 (10 mg/kg) were administered by oral gavage once daily for three consecutive days (qd × 3), alone or in combination with focal radiation. The delivered volume of vehicle or compound 569 was adjusted based on body weight of the individual animals. Mice stratified to the radiation therapy groups received 3 Gy per day for three consecutive days (qd × 3) 45 minutes following administration of the vehicle or compound 569. The mice were anesthetized with isoflurane during the radiation treatment, which was performed with the X-RAD 225Cx small animal image-guided irradiator (Precision X-Ray). The 40mm × 40mm irradiation field was centered on the tumor-bearing leg via fluoroscopy. Mice were irradiated with parallel-opposed anterior and posterior fields of 225 kVp, 13mA X-rays using a 0.3mm Cu filter at an average dose rate of 300 cGy/min.

Group 1 mice received vehicle qd × 3.

Group 2 mice received 10 mg/kg compound 569 qd × 3.

Group 3 mice received vehicle 45 minutes prior to 3 Gy focal irradiation qd × 3.

Group 4 mice received 10 mg/kg compound 569 45 minutes prior to 3 Gy focal irradiation qd × 3.

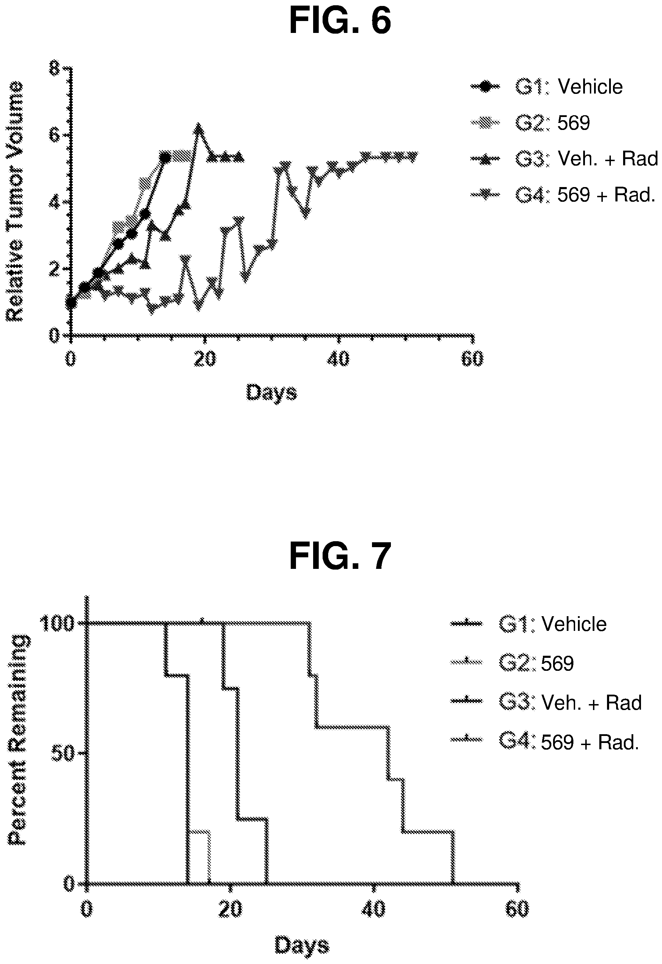

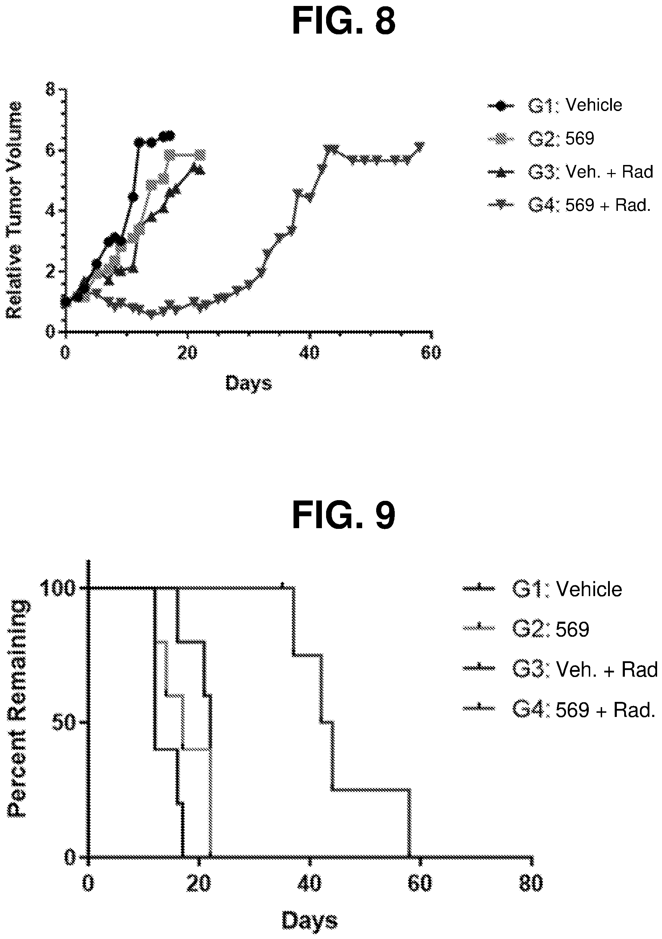

**Statistical and Graphical Analysis of the *in vivo* data.** Prism (GraphPad) was used for graphical presentations. SAS 9.4 and R 3.6.1 were used for statistical analyses. Tumor growth rates among four treatment groups were compared. In order to adjust for the differing tumor sizes at the start of treatment, the relative tumor volume was calculated by dividing the tumor volume at each time point by the volume at baseline. Line plots were used to display median relative tumor volume over time (FIGS. 6 and 8). When an animal exited the study due to tumor quintupling, the final tumor volume recorded for the animal was included with the data used to calculate the median volume at subsequent time points. Kaplan-Meier plots were used to show the percentage of mice in each treatment group remaining in the study over time (FIGS. 7 and 9). Mice that were found dead prior to reaching the quintupling endpoint were subjected to right-censoring. Differences in quintupling-free survival between the four treatment groups were evaluated with a log-rank test. A P-value less than 0.05 was considered statistically significant.

### Results

FIG. 1 shows an image of an immunoblot from an *in vitro* experiment assessing the effect of compound 569 on MCF7 cells with or without radiation. The immunoblot shows the inhibition of radiation-induced autophosphorylation of ATM and DNA-PK kinases and radiation-induced phosphorylation of KAP1, an ATM substrate, by compound 569 in tumor cells. MCF7, a human breast carcinoma cell line, was pre-treated for 30 min with compound 569 at concentrations of 20, 100, 500, and 1000 nM. DMSO was used as a negative control. The cells were then exposed to 0 or 10 Gy of ionizing radiation (IR) and one hour later, cells were harvested for immunoblot analyses. Compound 569 showed potent dose-dependent inhibition of radiation-induced phosphorylation of DNA-PKcs at Ser2056 and ATM at Ser1981 (both autophosphorylation events) and the ATM substrate KAP1 (ser824) demonstrating compound 569 inhibited both DNA-PK and ATM kinases in a cell.

FIG. 2 demonstrates the radiosensitizing properties of compound 569 in a clonogenic survival assay *in vitro.* MCF7 cells were pre-treated with vehicle or compound 569 at 100, 250, 500, and 1000 nM for 30 min before irradiation with increasing doses of IR (0, 2, and 4 Gy). After 5h, the medium was removed and fresh medium without inhibitors was added to cells, and they were cultured for 9 days before being fixed, stained and colonies counted. The 5-h exposure of cells to compound 569 induced a significant degree of radiosensitization without evidence of cellular toxicity in the absence of IR in MCF7 cells (FIG. 2, Table 1).

FIG. 3 is an immunoblot showing the induction of phosphorylation of TBK1 by compound 569, AZD0156 (a selective ATM inhibitor; ATMi), peposertib (a selective DNA-PK inhibitor; DNA-PKi), and a combination of AZD0156 and peposertib (Ai+Di) in HCT116 cells expressing wild-type p53 or HCT116 cells that were negative for p53 expression. Phosphorylation of TBK1 is a marker of activation of the type I interferon response and has been linked to enhanced tumor response to immune checkpoint blockade therapy. Dual inhibition of ATM and DNA-PKcs by combining the two selective chemical inhibitors (AZD0156 and peposertib) together resulted in more profound activation of TBK1 than either of the selective inhibitors alone. Similarly, exposure of cells to compound 569, a dual inhibitor of ATM and DNA-PKcs, was a more potent activator of TBK1 phosphorylation than either of the single target kinase inhibitors and was independent of p53 status. The TBK1 activation by 569 and dual inhibition of ATM and DNA-PKcs appeared to be independent of the cGAS and phospho-STING induction.

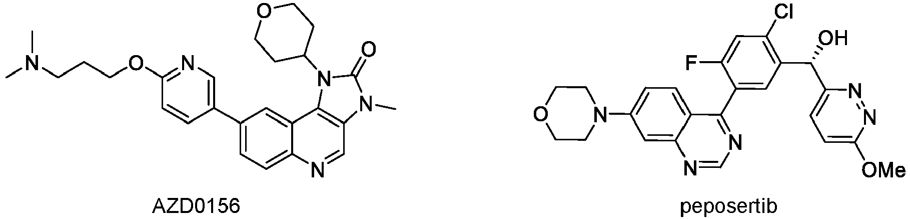

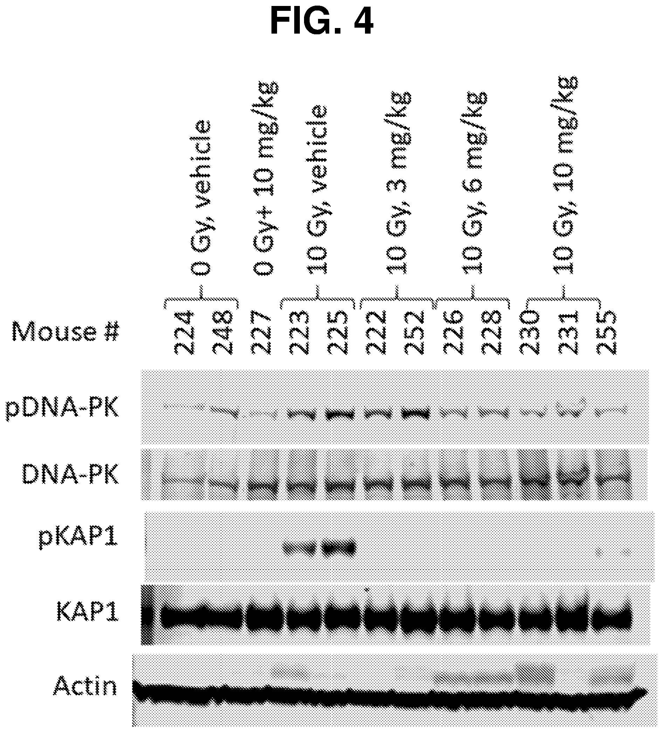

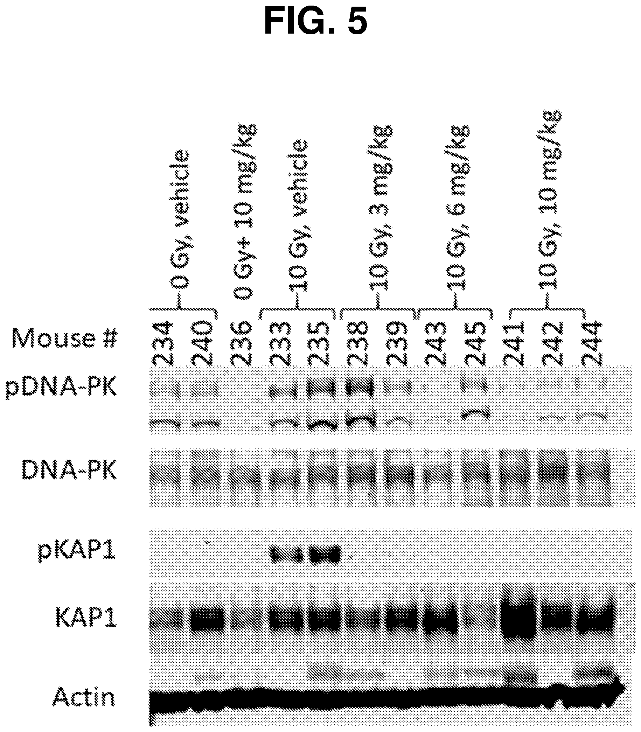

To assess the pharmacodynamic activity of compound 569 in vivo, FADU and MDA-MB-231 cells were grown as tumor xenografts in nude mice. Vehicle or compound 569 were administered by oral gavage alone or in combination with focal radiation. Mice were dosed with vehicle or compound 569 at 3, 6, and 10 mg/kg. Tumors then received 10 Gy of focal irradiation 45 min later or were left unirradiated (0 Gy, vehicle and 0 Gy + compound 569 at 10mg/kg). Tumors were harvested 1 h post radiation. Immunoblotting of lysates from these tumors shows radiation induced autophosphorylation of pDNA-PK and radiation-induced phosphorylation of KAP1, a downstream target of ATM and dose-dependent inhibition of those phosphorylation events in FADU and MDA231 tumors treated with radiation plus compound 569. Tumor levels of radiation-induced autophosphorylation of DNA-PK and radiation-induced phosphorylation of KAP1 were significantly inhibited by ~55% and >80%, respectively at 10mg/kg (FIGS. 4 and 5, Tables 2-5). Basal phosphorylation of DNA-PK was also inhibited by compound 569 in tumors.

**TABLE 1 Compound 569 Clonogenic Assay in MCF7 cells (see FIG. 2)**

| Compound (concentration) | Clonogenic survival ratio | Clonogenic survival ratio | Fold | Clonogenic survival ratio | Fold | % survival @ 0 Gy w/out IR |
|---|---|---|---|---|---|---|
| | 0 Gy | 2 Gy | 2 Gy | 4 Gy | 4 Gy | 100% |
| DMSO | 1.000 | 0.662 | 1.0 | 0.317 | 1.0 | 97% |
| 569 (100 nM) | 1.000 | 0.0882 | 7.5 | 0.0146 | 21.8 | 92% |
| 569 (250 nM) | 1.000 | 0.0610 | 10.8 | 0.00588 | 54.0 | 97% |
| 569 (500 nM) | 1.000 | 0.0451 | 14.7 | 0.00347 | 91.5 | 105% |
| 569 (1000 nM) | 1.000 | 0.0301 | 22.0 | 0.00160 | 198 | 100% |

**TABLE 2 Quantification of pDNA-PK/ DNA-PK in FADU tumors**

| Treatment | pDNA-PK/DNA-PK | Standard deviation |
|---|---|---|
| No radiation, vehicle | 1.14 | 0.263 |
| No radiation, 10 mg/kg compound 569 | 0.343 | NA |
| 10 Gy, vehicle | 1 | 0.0565 |
| 10 Gy, 3 mg/kg compound 569 | 0.938 | 0.181 |
| 10 Gy, 6 mg/kg compound 569 | 0.658 | 0.126 |
| 10 Gy, 10 mg/kg compound 569 | 0.476 | 0.188 |

**TABLE 3 Quantification of pKAP1/KAP1 in FADU tumors**

| Treatment | pKAP1/KAP1 | Standard deviation |
|---|---|---|
| No radiation, vehicle | 0.00505 | 0.000265 |
| No radiation, 10 mg/kg compound 569 | 0.00595 | NA |
| 10 Gy, vehicle | 1 | 0.0616 |
| 10 Gy, 3 mg/kg compound 569 | 0.0903 | 0.005 |
| 10 Gy, 6 mg/kg compound 569 | 0.0982 | 0.0741 |
| 10 Gy, 10 mg/kg compound 569 | 0.156 | 0.171 |

**TABLE 4 Quantification of pDNA-PK/DNA-PK in MDA-231 tumors**

| Treatment | pDNA-PK/DNA-PK | Standard deviation |
|---|---|---|
| No radiation, vehicle | 0.648 | 0.0948 |
| No radiation, 10mg/kg compound 569 | 0.223 | NA |
| 10 Gy, vehicle | 1 | 0.231 |
| 10 Gy, 3 mg/kg compound 569 | 0.889 | 0.414 |
| 10 Gy, 6 mg/kg compound 569 | 0.670 | 0.278 |
| 10 Gy, 10 mg/kg compound 569 | 0.454 | 0.116 |

**TABLE 5 Quantification of pKAP1/KAP1 in MDA-231 tumors**

| Treatment | pKAP1/KAP1 | Standard deviation |
|---|---|---|
| No radiation, vehicle | 0.00357 | 0.000826 |
| No radiation, 10mg/kg compound 569 | -0.00319 | NA |
| 10 Gy, vehicle | 1 | 0.114 |
| 10 Gy, 3 mg/kg compound 569 | 0.327 | 0.158 |
| 10 Gy, 6 mg/kg compound 569 | 0.0130 | 0.00879 |
| 10 Gy, 10 mg/kg compound 569 | 0.00901 | 0.00242 |

**FADU Tumors.** Mice in Group 1 received vehicle, qd × 3. Aggregate tumor growth in this group was progressive. The median time to endpoint was 14 days with a range of 11-14 days (FIGS. 6 and 7). Mice in Group 2 received compound 569 at a dose of 10 mg/kg, qd × 3. The median time to endpoint was 14 days with a range of 11 to 17 days. The tumor growth curves and quintupling-free survival for Group 2 animals are nearly identical to those for Group 1 (FIGS. 6 and 7). Mice in Group 3 received vehicle and a focal radiation dose of 3 Gy, which was delivered 45 minutes after administration of the vehicle. Both the vehicle and radiation were qd × 3. One animal was found dead on Day 16 due to unknown causes. The median time to endpoint was 21 days with a range of 19 to 25 days. Overall survival was significantly improved compared to Group 1 and Group 2 (P < 0.01, logrank). The delay in aggregate tumor growth and increase in quintupling-free survival for Group 3 relative to Groups 1 and 2 is shown in FIGS. 6 and 7. Mice in Group 4 received compound 569 and a focal radiation dose of 3 Gy, which was delivered 45 minutes after administration of the compound. Both compound 569 and radiation were administered qd × 3. The median time to endpoint was 42 days with a range of 31 to 51 days. Overall survival was significantly improved compared to Group 1, Group 2, and Group 3 (P < 0.01, logrank). The delay in aggregate tumor growth and increase in quintupling-free survival for Group 4 relative to all other groups is shown in FIGS. 6 and 7. All treatments were well-tolerated.

**MDA-MB-231 Tumors.** Mice in Group 1 received vehicle, qd × 3. The median time to endpoint was 12 days with a range of 12 to 17 days. Mice in Group 2 received compound 569 at a dose of 10 mg/kg, qd × 3. The median time to endpoint was 17 days with a range of 12 to 22 days. The tumor growth curves and quintupling-free survival for Group 2 animals are nearly identical to those for Group 1 (FIGS. 8 and 9). Mice in Group 3 received vehicle and a focal radiation dose of 3 Gy, which was delivered 45 minutes after administration of the vehicle. Both the vehicle and radiation were qd × 3. The median time to endpoint was 22 days with a range of 16 to 22 days. Overall survival was significantly improved compared to Group 1 animals treated with vehicle alone (*P* < 0.01, logrank). The increase in quintupling-free survival for Group 3 relative to Groups 1 and 2 is shown in FIG. 9. Mice in Group 4 received 10 mg/kg compound 569 and a focal radiation dose of 3 Gy, which was delivered 45 minutes after administration of compound. Both compound 569 and radiation were qd × 3. One animal was found dead on Day 35 due to unknown causes. The median time to endpoint was 43 days with a range of 37 to 58 days. Overall survival was significantly improved compared to Group 1, Group 2, and Group 3 (P < 0.01, logrank). The delay in aggregate tumor growth and increase in quintupling-free survival for Group 4 relative to all other groups is shown in FIGS. 8 and 9. All treatments were well-tolerated.

### OTHER EMBODIMENTS

Various modifications and variations of the described invention will be apparent to those skilled in the art without departing from the scope of the invention. Although the invention has been described in connection with specific embodiments, it should be understood that the invention as claimed should not be unduly limited to such specific embodiments. Indeed, various modifications of the described modes for carrying out the invention that are obvious to those skilled in the art are intended to be within the scope of the invention.

## Claims

1. A compound of formula (IA): 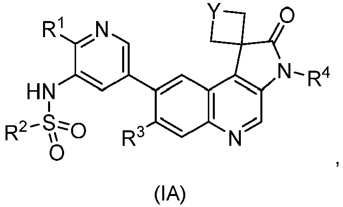 or a pharmaceutically acceptable salt thereof; wherein
Y is CHR⁵;
R¹ is -O-L-N(R⁷)₂;
R² is C₁-₃ alkyl;
each R³ is independently halogen or C₁-₃ alkyl;
R⁴ is alkyl;
R⁵ is hydrogen or benzyloxy;
each R⁷ is independently H or C₁-₃ alkyl; and
L is ethylene.

2. The compound of claim 1, or a pharmaceutically acceptable salt thereof, wherein R³ is halogen; preferably wherein the halogen is fluorine.

3. The compound of claims 1 or 2, or a pharmaceutically acceptable salt thereof, wherein one R⁷ is H, and the remaining R⁷ is C₁-₃ alkyl.

4. The compound of any one of claims 1 to 3, or a pharmaceutically acceptable salt thereof, wherein at least one R⁷ is isopropyl.

5. The compound of any one of claims 1 to 4, or a pharmaceutically acceptable salt thereof, wherein R² is methyl, ethyl, or isopropyl.

6. The compound of any one of claims 1 to 5, or a pharmaceutically acceptable salt thereof, wherein R² is methyl.

7. The compound of any one of claims 1 to 6, or a pharmaceutically acceptable salt thereof, wherein R⁴ is methyl.

8. The compound of any one of claims 1 to 7, or a pharmaceutically acceptable salt thereof, wherein R⁵ is hydrogen.

9. The compound of any one of claims 1 to 7, or a pharmaceutically acceptable salt thereof, wherein R⁵ is benzyloxy.

10. A compound selected from the group consisting of: 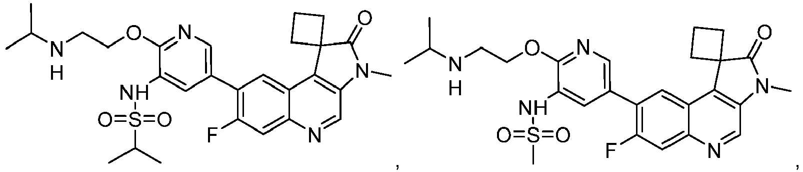 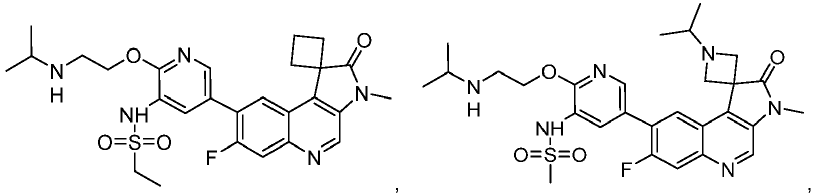 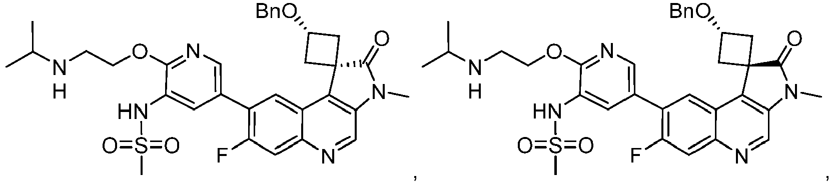 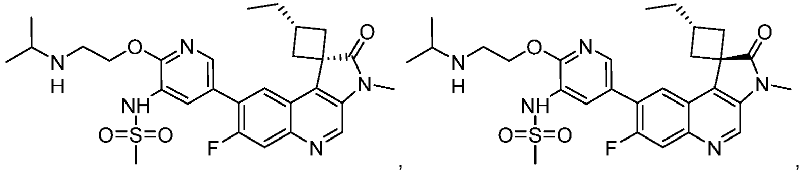 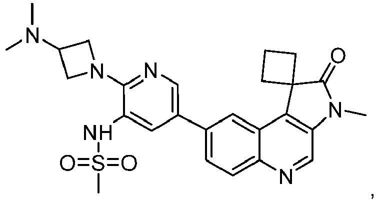 and pharmaceutically acceptable salts thereof.

11. The compound of claim 10, or a pharmaceutically acceptable salt thereof, wherein the compound is: 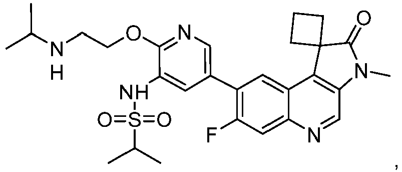 or a pharmaceutically acceptable salt thereof.

12. The compound of claim 10, or a pharmaceutically acceptable salt thereof, wherein the compound is: 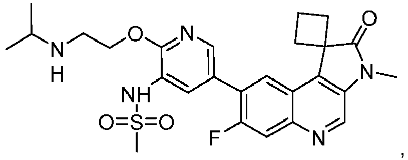 or a pharmaceutically acceptable salt thereof.

13. The compound of claim 10, or a pharmaceutically acceptable salt thereof, wherein the compound is: 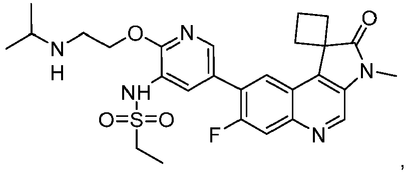 or a pharmaceutically acceptable salt thereof.

14. A pharmaceutical composition comprising the compound of any one of claims 1 to 13 or a pharmaceutically acceptable salt thereof and a pharmaceutically acceptable excipient.

15. A compound, or a pharmaceutically acceptable salt thereof, of any one of claims 1 to 13, or a pharmaceutical composition of claim 14, for use in a method of treating an oncological disease, said method comprising administering a therapeutically effective amount of the compound of any one of claims 1 to 13, or a pharmaceutically acceptable salt thereof, or the pharmaceutical composition of claim 14 to a patient in need thereof.

16. The compound, or the pharmaceutically acceptable salt thereof, or the pharmaceutical composition for use according to claim 15, wherein:
the patient is receiving radiotherapy; optionally wherein the compound or the pharmaceutical composition is administered to the patient concomitantly with the radiotherapy; or the compound or the pharmaceutical composition is administered to the patient before radiotherapy; or the compound or the pharmaceutical composition is administered to the patient after radiotherapy; optionally wherein:
i) the radiotherapy comprises external, internal, brachytherapy, or systemic exposure; and/or
ii) the radiotherapy comprises administering an antibody radionucleotide conjugate.

17. The compound, or the pharmaceutically acceptable salt thereof, or the pharmaceutical composition for use according to claims 15 or 16, wherein:
the patient is receiving an anti-tumor agent; optionally wherein:
i) the anti-tumor agent is cisplatin, oxaliplatin, carboplatin, valrubicin, idarubicin, calicheamicin, or a PARP inhibitor; and/or
ii) the anti-tumor agent is an anti-tumor biological agent or an anti-tumor immunotherapeutic agent; and/or
iii) the compound or the pharmaceutical composition is administered to the patient concomitantly with the anti-tumor agent; or
iv) the compound or the pharmaceutical composition is administered to the patient after the anti-tumor agent.

18. The compound, or the pharmaceutically acceptable salt thereof, or the pharmaceutical composition for use according to any one of claims 15 to 17, wherein:
the oncological disease is a brain cancer, bladder cancer, breast cancer, central nervous system cancer, cervical cancer, colon cancer, endometrial cancer, esophageal cancer, gastrointestinal stromal tumor, gastric cancer, head and neck cancer, buccal cancer, cancer of the mouth, hepatocellular cancer, lung cancer, melanoma, Merkel cell carcinoma, mesothelioma, nasopharyngeal cancer, neuroblastoma, osteosarcoma, ovarian cancer, pancreatic cancer, prostate cancer, renal cancer, salivary gland cancer, sarcomas, testicular cancer, urothelial cancer, vulvar cancer, or Wilm's tumor;
and/or
the oncological disease is a breast cancer, lung cancer, head and neck cancer, pancreatic cancer, rectal cancer, glioblastoma, hepatocellular carcinoma, cholangiocarcinoma, metastic liver lesions, melanoma, bone sarcoma, soft tissue sarcoma, endometrial cancer, cervical cancer, prostate cancer, or Merkel cell carcinoma.

## Patentansprüche

1. Verbindung der Formel (IA): 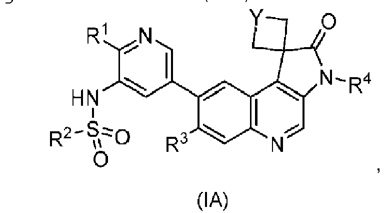 oder pharmazeutisch unbedenkliches Salz davon, wobei
Y für CHR⁵ steht,
R¹ für -O-L-N(R⁷)₂ steht,
R² für C₁₋₃-Alkyl steht,
R³ jeweils yunabhängig für Halogen oder C₁₋₃-Alkyl steht,
R⁴ für Alkyl steht,
R⁵ für Wasserstoff oder Benzyloxy steht,
R⁷ jeweils unabhängig für H oder C₁-₃ Alkyl steht und
L für Ethylen steht.

2. Verbindung nach Anspruch 1 oder ein pharmazeutisch unbedenkliches Salz davon, wobei R³ für Halogen steht, wobei sich bei dem Halogen vorzugsweise um Fluor handelt.

3. Verbindung nach Ansprüchen 1 oder 2 oder ein pharmazeutisch unbedenkliches Salz davon, wobei ein R⁷ für H steht und die restlichen R⁷ für C₁₋₃-Alkyl stehen.

4. Verbindung nach einem der Ansprüche 1 bis 3 oder ein pharmazeutisch unbedenkliches Salz davon, wobei mindestens ein R⁷ für Isopropyl steht.

5. Verbindung nach einem der Ansprüche 1 bis 4 oder ein pharmazeutisch unbedenkliches Salz davon, wobei R² für Methyl, Ethyl oder Isopropyl steht.

6. Verbindung nach einem der Ansprüche 1 bis 5 oder ein pharmazeutisch unbedenkliches Salz davon, wobei R² für Methyl steht.

7. Verbindung nach einem der Ansprüche 1 bis 6 oder ein pharmazeutisch unbedenkliches Salz davon, wobei R⁴ für Methyl steht.

8. Verbindung nach einem der Ansprüche 1 bis 7 oder ein pharmazeutisch unbedenkliches Salz davon, wobei R⁵ für Wasserstoff steht.

9. Verbindung nach einem der Ansprüche 1 bis 7 oder ein pharmazeutisch unbedenkliches Salz davon, wobei R⁵ für Benzyloxy steht.

10. Verbindung, ausgewählt aus der Gruppe bestehend aus: 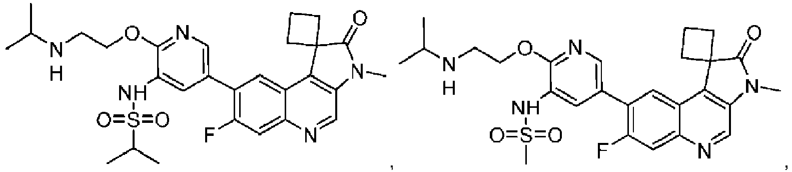 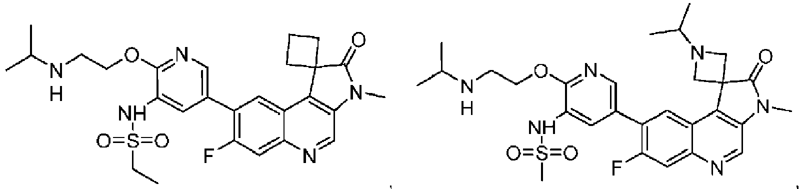 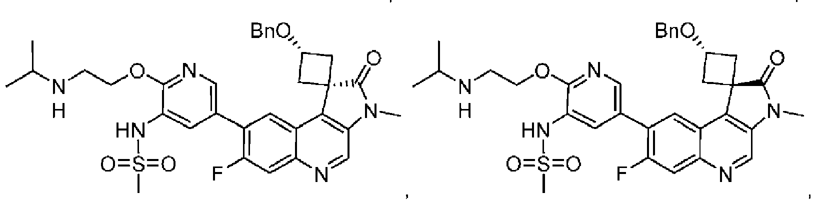 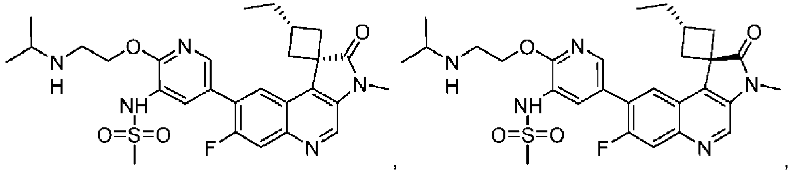 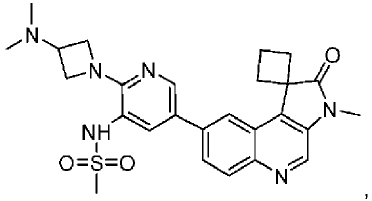 und pharmazeutisch unbedenkliche Salze davon.

11. Verbindung nach Anspruch 10 oder pharmazeutisch unbedenkliches Salz davon, wobei es sich bei der Verbindung um die folgende handelt: 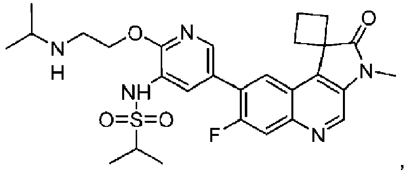 oder pharmazeutisch unbedenkliches Salz davon.

12. Verbindung nach Anspruch 10 oder pharmazeutisch unbedenkliches Salz davon, wobei es sich bei der Verbindung um die folgende handelt: 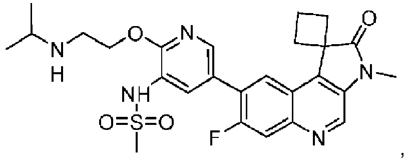 oder pharmazeutisch unbedenkliches Salz davon.

13. Verbindung nach Anspruch 10 oder pharmazeutisch unbedenkliches Salz davon, wobei es sich bei der Verbindung um die folgende handelt: 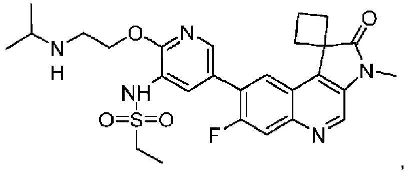 oder pharmazeutisch unbedenkliches Salz davon.

14. Pharmazeutische Zusammensetzung, umfassend die Verbindung nach einem der Ansprüche 1 bis 13 oder ein pharmazeutisch unbedenkliches Salz davon und einen pharmazeutisch unbedenklichen Exzipienten.

15. Verbindung oder ein pharmazeutisch unbedenkliches Salz davon nach einem der Ansprüche 1 bis 13 oder pharmazeutische Zusammensetzung nach Anspruch 14 zur Verwendung in einem Verfahren zur Behandlung einer onkologischen Erkrankung, wobei das Verfahren die Verabreichung einer therapeutisch wirksamen Menge der Verbindung nach einem der Ansprüche 1 bis 13 oder eines pharmazeutisch unbedenklichen Salzes davon oder der pharmazeutischen Zusammensetzung nach Anspruch 14 an einen dessen bedürftigen Patienten umfasst.

16. Verbindung oder pharmazeutisch unbedenkliches Salz davon oder pharmazeutische Zusammensetzung zur Verwendung nach Anspruch 15, wobei:
der Patient eine Strahlentherapie erhält, wobei die Verbindung oder die pharmazeutische Zusammensetzung dem Patienten gegebenenfalls einhergehend mit der Strahlentherapie verabreicht wird oder die Verbindung oder die pharmazeutische Zusammensetzung dem Patienten vor der Strahlentherapie verabreicht wird oder die Verbindung oder die pharmazeutische Zusammensetzung dem Patienten nach der Strahlentherapie verabreicht wird, wobei gegebenenfalls:
i) die Strahlentherapie externe, interne, Brachytherapie oder systemische Exposition umfasst und/oder
ii) die Strahlentherapie die Verabreichung eines Antikörper-Radionukleotid-Konjugats umfasst.

17. Verbindung oder pharmazeutisch unbedenkliches Salz davon oder pharmazeutische Zusammensetzung zur Verwendung nach Anspruch 15 oder 16, wobei:
der Patient ein Anti-Tumor-Mittel erhält, wobei gegebenenfalls:
i) das Antitumormittel Cisplatin, Oxaliplatin, Carboplatin, Valrubicin, Idarubicin, Calicheamicin oder einen PARP-Inhibitor ist und/oder
ii) das Antitumormittel ein biologisches Antitumormittel oder ein Antitumor-Immuntherapeutikum ist und/oder
iii) die Verbindung oder die pharmazeutische Zusammensetzung dem Patienten einhergehend mit dem Antitumormittel verabreicht wird oder
iv) die Verbindung oder die pharmazeutische Zusammensetzung dem Patienten nach dem Antitumormittel verabreicht wird.

18. Verbindung oder pharmazeutisch unbedenkliches Salz davon oder pharmazeutische Zusammensetzung zur Verwendung nach einem der Ansprüche 15 bis 17, wobei:
die onkologische Krankheit ein Hirnkrebs, Blasenkrebs, Brustkrebs, Krebs des Zentralnervensystems, Gebärmutterhalskrebs, Darmkrebs, Endometriumkrebs, Speiseröhrenkrebs, gastrointestinaler Stromatumor, Magenkrebs, Kopf-Hals-Krebs, Wangenschleimhautkrebs, Mundkrebs, Leberzellkarzinom, Lungenkrebs, Melanom, Merkelzellkarzinom, Mesotheliom, Nasopharynxkarzinom, Neuroblastom, Osteosarkom, Ovarialkarzinom, Bauchspeicheldrüsenkrebs, Prostatakrebs, Nierenkrebs, Speicheldrüsenkrebs, Sarkom, Hodenkrebs, Urothelkarzinom, Vulvakrebs oder Wilm-Tumor ist
und/oder
die onkologische Erkrankung Brustkrebs, Lungenkrebs, Kopf-Hals-Krebs, Bauchspeicheldrüsenkrebs, Rektumkrebs, Glioblastom, hepatozelluläres Karzinom, Cholangiokarzinom, metastische Leberläsionen, Melanom, Knochensarkom, Weichteilsarkom, Endometriumkarzinom, Gebärmutterhalskrebs, Prostatakrebs oder Merkelzellkarzinom ist.

## Revendications

1. Composé de formule (IA) : 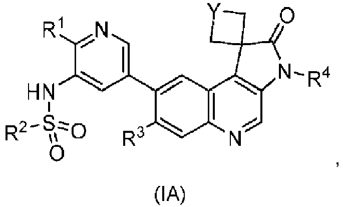 ou sel pharmaceutiquement acceptable correspondant ;
Y étant CHR⁵ ;
R¹ étant -O-L-N(R⁷)₂ ;
R² étant C₁-₃ alkyle ;
chaque R³ étant indépendamment halogène ou C₁-₃ alkyle ;
R⁴ étant alkyle ;
R⁵ étant hydrogène ou benzyloxy ;
chaque R⁷ étant indépendamment H ou C₁-₃ alkyle ; et
L étant éthylène.

2. Composé selon la revendication 1, ou sel pharmaceutiquement acceptable correspondant, R³ étant halogène ; préférablement l'halogène étant le fluor.

3. Composé selon les revendications 1 ou 2, ou sel pharmaceutiquement acceptable correspondant, un R⁷ étant H, et les autres R⁷ étant C₁-₃ alkyle.

4. Composé selon l'une quelconque des revendications 1 à 3, ou sel pharmaceutiquement acceptable correspondant, au moins un R⁷ étant isopropyle.

5. Composé selon l'une quelconque des revendications 1 à 4, ou sel pharmaceutiquement acceptable correspondant, R² étant méthyle, éthyle, ou isopropyle.

6. Composé selon l'une quelconque des revendications 1 à 5, ou sel pharmaceutiquement acceptable correspondant, R² étant méthyle.

7. Composé selon l'une quelconque des revendications 1 à 6, ou sel pharmaceutiquement acceptable correspondant, R⁴ étant méthyle.

8. Composé selon l'une quelconque des revendications 1 à 7, ou sel pharmaceutiquement acceptable correspondant, R⁵ étant hydrogène.

9. Composé selon l'une quelconque des revendications 1 à 7, ou sel pharmaceutiquement acceptable correspondant, R⁵ étant benzyloxy.

10. Composé choisi dans le groupe constitué par : 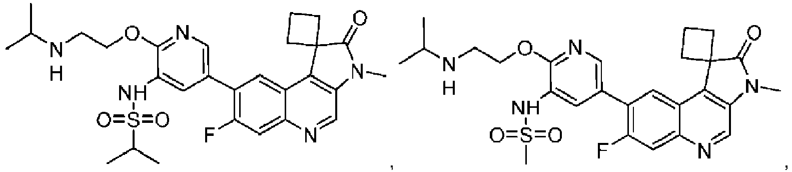 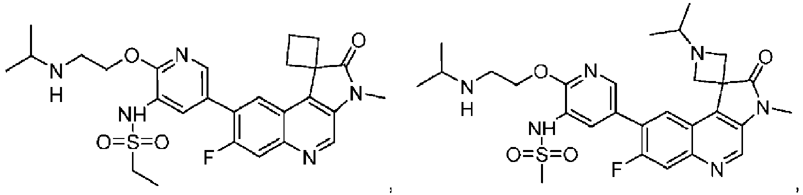 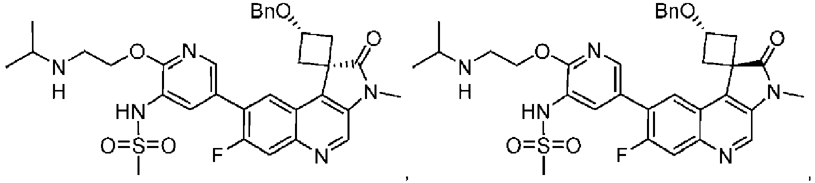 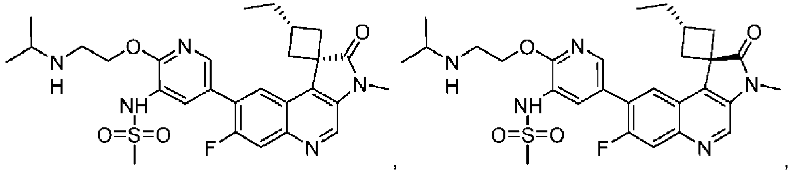 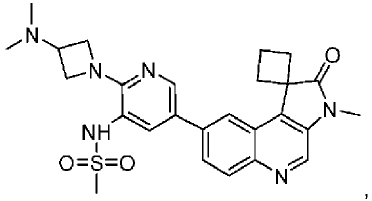 et des sels pharmaceutiquement acceptables correspondants.

11. Composé selon la revendication 10, ou sel pharmaceutiquement acceptable correspondant, le composé étant : 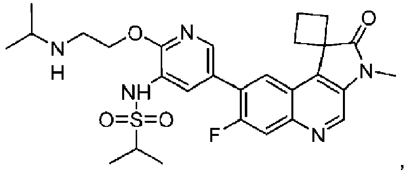 ou un sel pharmaceutiquement acceptable correspondant.

12. Composé selon la revendication 10, ou sel pharmaceutiquement acceptable correspondant, le composé étant : 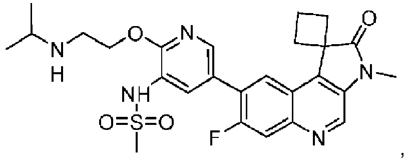 ou un sel pharmaceutiquement acceptable correspondant.

13. Composé selon la revendication 10, ou sel pharmaceutiquement acceptable correspondant, le composé étant : 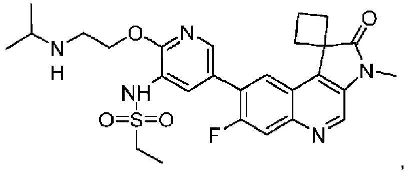 ou un sel pharmaceutiquement acceptable correspondant.

14. Composition pharmaceutique comprenant un composé selon l'une quelconque des revendications 1 à 13 ou un sel pharmaceutiquement acceptable correspondant et un excipient pharmaceutiquement acceptable.

15. Composé, ou sel pharmaceutiquement acceptable correspondant, selon l'une quelconque des revendications 1 à 13, ou composition pharmaceutique selon la revendication 14, pour une utilisation dans un procédé de traitement d'une maladie oncologique, ledit procédé comprenant une administration d'une quantité thérapeutiquement efficace du composé selon l'une quelconque des revendications 1 à 13, ou d'un sel pharmaceutiquement acceptable correspondant, ou de la composition pharmaceutique selon la revendication 14 à un patient qui en a besoin.

16. Composé, ou sel pharmaceutiquement acceptable correspondant, ou composition pharmaceutique pour une utilisation selon la revendication 15 :
le patient recevant une radiothérapie ; éventuellement le composé ou la composition pharmaceutique étant administré(e) au patient en même temps que la radiothérapie ; ou le composé ou la composition pharmaceutique étant administré(e) au patient avant la radiothérapie ; ou le composé ou la composition pharmaceutique étant administré(e) au patient après la radiothérapie ; éventuellement :
i) la radiothérapie comprenant une exposition externe, interne, par curiethérapie ou systémique ; et/ou
ii) la radiothérapie comprenant une administration d'un conjugué anticorps radionucléotide.

17. Composé, ou sel pharmaceutiquement acceptable correspondant, ou composition pharmaceutique pour une utilisation selon les revendications 15 ou 16 :
le patient recevant un agent antitumoral ; éventuellement :
i) l'agent antitumoral étant le cisplatine, l'oxaliplatine, le carboplatine, la valrubicine, l'idarubicine, la calicheamicine ou un inhibiteur de PARP ; et/ou
ii) l'agent antitumoral étant un agent biologique antitumoral ou un agent immunothérapeutique antitumoral ; et/ou
iii) le composé ou la composition pharmaceutique étant administré(e) au patient en même temps que l'agent antitumoral ; ou
iv) le composé ou la composition pharmaceutique étant administré(e) au patient après l'agent antitumoral.

18. Composé, ou sel pharmaceutiquement acceptable correspondant, ou composition pharmaceutique pour une utilisation selon l'une quelconque des revendications 15 à 17 :
la maladie oncologique étant un cancer du cerveau, un cancer de la vessie, un cancer du sein, un cancer du système nerveux central, un cancer du col de l'utérus, un cancer du côlon, un cancer de l'endomètre, un cancer de l'œsophage, une tumeur stromale gastro-intestinale, un cancer gastrique, un cancer de la tête et du cou, un cancer buccal, un cancer de la bouche, un cancer hépatocellulaire, un cancer du poumon, un mélanome, un carcinome à cellules de Merkel, un mésothéliome, un cancer du nasopharynx, un neuroblastome, un ostéosarcome, un cancer de l'ovaire, un cancer du pancréas, un cancer de la prostate, un cancer du rein, un cancer des glandes salivaires, des sarcomes, un cancer des testicules, un cancer urothélial, un cancer de la vulve ou une tumeur de Wilms ;
et/ou
la maladie oncologique étant un cancer du sein, un cancer du poumon, un cancer de la tête et du cou, un cancer du pancréas, un cancer du rectum, un glioblastome, un carcinome hépatocellulaire, un cholangiocarcinome, des lésions métastatiques du foie, un mélanome, un sarcome osseux, un sarcome des tissus mous, un cancer de l'endomètre, un cancer du col de l'utérus, un cancer de la prostate ou un carcinome à cellules de Merkel.
